# EUROPEAN PATENT APPLICATION

(11) **EP 3 831 823 A1**
(43) Date of publication of application: **09.06.2021**
(21) Application number: 19843979.6
(22) Date of filing: 01.08.2019
(51) Int. Cl.: C07D 403/00, C07D 405/00, C07D 407/00, C07D 409/00, C07D 471/04, C07D 487/04, C07D 491/04, A61K 31/33, A61P 37/02

(54) **PREPARATION AND APPLICATION OF AROMATIC COMPOUND HAVING IMMUNOREGULATORY FUNCTION**

(30) Priority: 01.08.2018 CN 201810865177; 11.06.2019 CN 201910503368
(71) Applicant: Shanghai Ennovabio Pharmaceuticals Co., Ltd., Shanghai 201203 (CN)
(72) Inventor: ZHANG, Yi, Shanghai 201203 (CN); DENG, Jianwen, Shanghai 201203 (CN); FENG, Zhiyong, Shanghai 201203 (CN); HUANG, Lei, Shanghai 201203 (CN); JIANG, Lei, Shanghai 201203 (CN); LU, Xiaoli, Shanghai 201203 (CN); SHANG, Ke, Shanghai 201203 (CN); SHOU, Jianyong, Shanghai 201203 (CN); WANG, Bing, Shanghai 201203 (CN); XU, Xueli, Shanghai 201203 (CN); XU, Yuan, Shanghai 201203 (CN)
(74) Representative: dompatent von Kreisler Selting Werner - Partnerschaft von Patent- und Rechtsanwälten mbB
(86) International application number: PCT/CN2019/098925
(87) International publication number: WO 2020/025030

(57) **Abstract**

Provided are an aromatic compound having immunoregulatory effect, a preparation method thereof, and a use of the same in regulating immune responses or inhibiting PD-1/PD-L1.

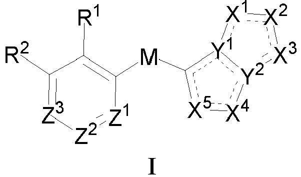

## Description

### FIELD OF THE INVENTION

The present invention relates to the field of small molecule protein inhibitors. Specifically, provided herein are the preparation and application of compound having immunomodulatory function.

### BACKGROUND OF THE INVENTION

The immune system has functions such as surveillance, defense, and regulation. Cellular immunity is mainly involved in the immune response to intracellular parasitic pathogenic microorganisms and tumor cells, the formation of delayed type hypersensitivity and autoimmune diseases, transplantation rejection and the regulation of humoral immunity. The activation of T lymphocytes by antigen presenting cells is usually regulated by two different signals. Primary signal is transduced by presenting foreign antigen peptidesthrough the major histocompatibility complex (MHC) on APC cells to the T cell receptor (TCR). Secondary signals, also known as costimulatory signals, are transduced through the interaction of costimulatory molecules on APC cells with T cell surface receptors, to regulate the proliferation of T cell, secretion of cytokine and effector functions. Secondary signals include positive regulation and negative regulation. Positive signals promote T cell activation and negative signals induce T cell tolerance, which is essential for the human body to adapt and adjust the response of self-immune cells to different external antigens.

Programmed death-ligand 1 (PD-L1), is also known as cluster of differentiation 274 (CD274) or B7 homolog1 (B7-H1), belongs to tumor necrosis factor superfamily and is a type I transmembrane glycoprotein consisting of 290 amino acid residues. It contains an IgV-like domain, an IgC-like domain, a hydrophobic transmembrane domain, and an intracellular tail containing 30 amino acids. The molecular weight of PD-L1 is 40 kDa. PD-L1 mRNA is present in almost all tissues, while PD-L1 protein is only constitutively expressed in a few tissues, including liver, lungs, tonsils, and immune amnesty tissues, such as eyes, placenta, etc. PD-L1 is also expressed on activated T cells, B cells, monocytes, dendritic cells, macrophages, etc.

The receptor of PD-L1 is PD-1, which is mainly expressed on the surface of activated immune cells, such as CD4⁺ T cells, CD8⁺ T cells, NK cells, B cells, monocytes, etc. The binding of PD-L1 to PD-1 can initiate the phosphorylation of tyrosine residues in ITIM (immunoreceptor tyrosine inhibitory motif) in PD-1 cytoplasmic region, promote the binding of tyrosine phospholipase to SHP2, activate SHP2, and dephosphorylate downstream Syk and PI3K, thereby transmitting a termination signal and inhibiting the interaction between antigen-presenting cells or dendritic cells with T cells. Such binding can further inhibit the metabolism of T cells, inhibit the secretion of anti-apoptotic protein Bc1-2, reduce the secretion of effector cytokines IL-2, IFN-γ, and induce T cell depletion and apoptosis, thereby reducing immune responses in which immune T cells are involved, and exerting negative regulation.

After T cells recognize the antigen and are activated, IFN-γ will be secreted. T cell-derived IFN-γ will expand T cells and maintain functions of T cells, such as up-regulating MHC molecules, enhancing antigen processing and presentation of target cells, and promoting T cell differentiation. IFN-γ will also induce the expression of PD-L1 at the site of immune inflammation in a tissue, thereby preventing the tissue from being damaged by excessive immunity. IFN-γ can induce the expression of PD-L1 on the surface of normal epithelial cells, vascular endothelial cells, myeloid cells, naive T cells, and the like. IFN-γ-regulatory factor 1 (IRF-1) induced by interferon can also bind to interferon regulatory factor binding sites at 200 bp and 320 bp upstream to the transcription start site of PD-L1, thereby regulating PD-L1 at the transcription level. PD-L1 can bind PD-1 on the surface of T cells to exert negative regulation, thereby protecting inflammatory sites.

The negative regulation of PD-L1 plays an important role in tumor immunology. In 2004, Konishi et al. first found that PD-L1 was expressed in tissue samples from patients with non-small cell lung cancer, and then PD-L1 was found to be expressed in the tissues of patients with various tumor, including gastric cancer, lung cancer, liver cancer, and intrahepatic cholangiocarcinoma, colon cancer, pancreatic cancer, ovarian cancer, breast cancer, cervical cancer, head and neck squamous cell carcinoma, nasopharyngeal carcinoma, esophageal cancer, bladder cancer, renal cell carcinoma, skin cancer, oral squamous cell carcinoma, etc. During the malignant transformation of cells, new protein molecules will be generated due to gene mutations, exogenous gene (viral) expression or static gene activation, and the like. After these new proteins are degraded in a cell, certain degraded peptide fragments can be expressed on the cell surface and become tumor antigens. The immune system can recognize tumor antigens and eliminate tumor cells through immune monitoring, while tumor cells can escape immune attacks by means of PD-L1.

The expression of PD-L1 at the tumor site can protect tumor cells through various ways. Tumor infiltrating lymphocytes (TIL) secretes IFN-γ, which can induce tumor cells and surrounding stromal cells to express PD-L1. PD-L1 of tumor cells can bind to PD-1 on TIL, inhibit the activation of TIL cells, and further cause apoptosis thereof. *In vitro* experiments have shown that tumor cell-associated PD-L1 can increase the apoptosis of tumor-specific T cells, while PD-L1 monoclonal antibodies can reduce such effect. Tumor-associated PD-L1 can promote the expression of IL-10 by T cells, and further inhibit the immune response. PD-L1 is not only a ligand of PD-1, but also can act as a receptor to transmit reverse signals to protect tumor cells from apoptosis induced by other anti-tumor pathways, such as FAS-FASL.

Several marketed monoclonal antibody drugs targeting PD-1 or PD-L1 have proven that blockers for PD-1 / PD-L1 can be clinically useful in the treatment of various tumors. However, antibody drugs exhibit their own characteristics, such as high production cost, poor stability, necessity to be administered by injection, and proneness to inducing immunogenicity, etc. Small molecule drugs have advantages, such as good tissue permeability, convenient storage and transportation, low production cost, non-immunogenicity, and availability of oral administration, etc. Therefore, it is of use and social significance to research and develop small molecule blockers for PD-1 / PD-L1.

In summary, there is an urgent need in developing small molecule PD-1/PD-L1 blockers in the field.

### BRIEF SUMMARY OF THE INVENTION

The purpose of the present invention is to provide a class of small molecule PD-1/PD-L1 blockers.

The first aspect of the present invention provides a compound according to the Formula I, the stereoisomers or tautomers thereof, or pharmaceutically acceptable salts, hydrates or solvates thereof: 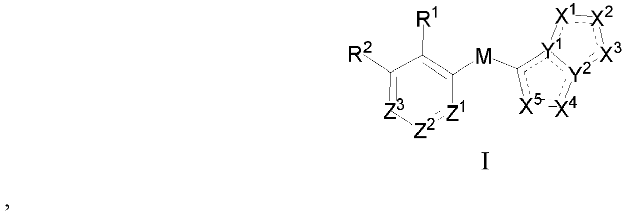 wherein
M is selected from O and NH;
X² is selected from N, NR³, CR³, O, S, N=CR³, CR³=N and CR³=CR³;
X⁴ is selected from N, CH, O, S, N=CH, CH=N and CH=CH; and only one of X² and X⁴ is group selected from the group consisting of CR³=CR³, N=CR³ or CR³=N (R³ is H in X⁴);
X¹ and X⁵ are independently selected from N, CH, O and S;
X³ is selected from N, CR³, O and S; and
at least one of X³ and X⁴ is O, S or N;
Y¹ and Y² are independently N or C;
Z¹, Z² and Z³ are independently N or CR⁴;
R¹ is selected from the group consisting of H, halogen, CN, substituted or unsubstituted C₁-C₆ alkyl, substituted or unsubstituted C₁-C₆ alkoxyl;
R² is selected from the group consisting of substituted or unsubstituted C₆-C₁₀ aryl, substituted or unsubstituted 5-10 membered heteroaryl with 1-3 heteroatoms selected from N, S and O, substituted or unsubstituted 5-10 membered heterocyclyl with 1-3 heteroatoms selected from N, S and O; and one or more H of R² are substituted by R⁵;
R³ is selected from H, substituted or unsubstituted C₁-C₆ alkyl, or 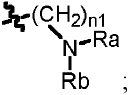 and at least one of R³ is 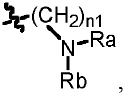 n1 is 1, 2, 3 or 4;
Ra and Rb are independently selected from H, -(C=O)-substituted or unsubstituted C₁-C₈ alkyl, substituted or unsubstituted C₁-C₈ alkyl, substituted or unsubstituted C₂-C₆ alkenyl, substituted or unsubstituted C₂-C₆ alkynyl, substituted or unsubstituted C₁-C₈ alkylamino, substituted or unsubstituted C₁-C₈ alkoxyl, substituted or unsubstituted C₃-C₁₀ cycloalkyl, substituted or unsubstituted 3-10 membered heterocyclyl with 1-3 heteroatoms selected from the group consisting of N, S and O, substituted or unsubstituted C₆-C₁₀ aryl, substituted or unsubstituted 5-10 membered heteroaryl with 1-3 heteroatoms selected from the group consisting of N, S and O, or substituted or unsubstituted 5-10 membered heterocyclyl with 1-3 heteroatoms selected from the group consisting of N, S and O; or
Ra and Rb and adjacent N atoms together form a substituted or unsubstituted 5-10 membered heterocyclyl with 1-3 heteroatoms selected from the group consisting of N, S and O;
R⁴ is selected from the group consisting of H, halogen, CN, substituted or unsubstituted C₁-C₆ alkyl, substituted or unsubstituted C₁-C₆ alkoxyl;
R⁵ is selected from the group consisting of H, CN, halogen, substituted or unsubstituted (-L₁-L₂-(CH₂)_{q} -(substituted or unsubstituted 5-7 membered heteroaryl)), substituted or unsubstituted (-L₁-L₂-(CH₂)_{q}-N(Ra)(Rb)), -O-substituted or unsubstituted (-(CH₂)_{q}O-(CH₂)_{q}-N(Ra)(Rb)), or substituted or unsubstituted group selected from the group consisting of C₆-C₁₀ aryl group, 5-10 membered heteroaryl with 1-3 heteroatoms selected from the group consisting of N, S and O, -L₁-L₂-6-10 membered aryl-(CH₂)_{q}-N(Ra)(Rb), -L₁-L₂-5-10 membered heteroaryl-(CH₂)_{q}-N(Ra)(Rb); wherein L₁ and L₂ are each independent selected from the group consisting of none, substituted or unsubstituted C₁-C₈ alkylene, -NH-C(=O)-NH-, -C(=O)-NH-, -O-, -S- or -NH-; or two R⁵ and the connected carbon atoms together form a group selected from substituted or unsubstituted 5-7 membered heteroaryl, substituted or unsubstituted 5-7 membered heterocyclyl;
q is selected from 0, 1, 2, 3, 4, 5, 6, 7 or 8;
unless otherwise specified, "substituted" refers to being substituted by one or more (for example, 2, 3, 4, etc.) substituents selected from halogen, C₁-C₆ alkoxyl, halogenated C₁-C₆ alkoxyl, C₃-C₈ cycloalkyl, halogenated C₃-C₈ cycloalkyl, methyl sulfone group, -S(=O)₂NHz, - S(=O)₂NHCH₃, oxo(= O), -CN, hydroxyl, -NH₂, C₁-C₆ amine, carboxyl, C₁-C₆ amide (-C(=O)-N(Rc)₂ or -NH-C(=O)(Rc), Rc is H or C₁-C₅ alkyl), 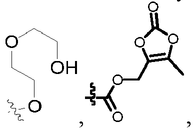 or substituted or unsubstituted groups selected from the group consisting of C1-C6 alkyl, C6-C10 aryl, 5-10 membered heteroaryl with 1-3 heteroatoms selected from N, S, O, and 5-10 membered heterocyclyl (including fused or spiro ring) with 1-3 heteroatoms selected from N, S and O, -(CH₂) -C6-C10 aryl, -(CH₂)- (5-10 membered heteroaryl with 1-3 heteroatoms selected from N, S and O), and the substitution means replaced by substituents selected from the group consisting of halogen, C1-C6 alkyl, C1-C6 alkoxyl, oxo, -CN, -OH, C6-C10 aryl, 5-10 membered heteroaryl with 1-3 heteroatoms selected from N, S and O, -O-(CH_{z})₄-CN; 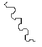 is the attachment site of the group;
each 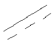 is a single bond or a double bond independently;
with the proviso that the compound of formula I has a chemically stable structure.

In another preferred embodiment, q is selected from 1, 2, 3 or 4.

In another preferred embodiment, R² is selected from the group consisting of substituted or unsubstituted phenyl.

In another preferred embodiment R² is selected from the group consisting of

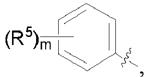

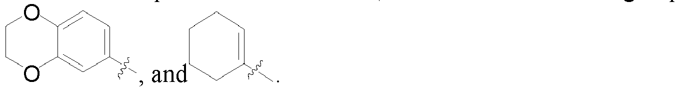

In another preferred embodiment the compound has a structure according to Formula II:

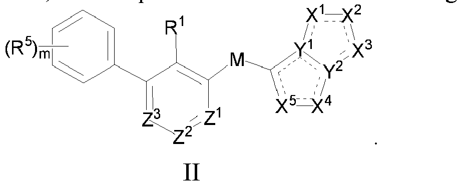

In another preferred embodiment the compound has a structure according to Formula III: 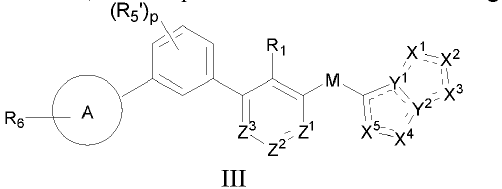 wherein R₅' is selected from H, CN, halogen, substituted or unsubstituted C₁-C₆ alkyl, substituted or unsubstituted C₁-C₆ alkoxyl;
R₆ is (C₁-C₈ alkylene)-N(Ra)(Rb);
ring A is selected from the group consisting of substituted or unsubstituted C₆-C₁₀ aryl, substituted or unsubstituted 5-10 membered heteroaryl with 1-3 heteroatoms selected from the group consisting of N, S and O, substituted or unsubstituted 5-10 membered heterocyclyl with 1-3 heteroatoms selected from the group consisting of N, S and O;
p is 1, 2, 3, or 4.

In another preferred embodiment, the compound of Formula I has the structure according to Formula I: 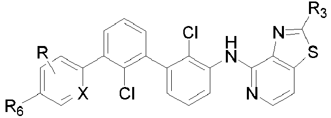 X is CH or N;
R is a substituent selected from the group consisting of halogen, CN, C₁-C₆ alkyl, halogenated C₁-C₆ alkyl, and C₁-C₆ alkoxyl.

In another preferred embodiment, 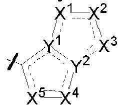 is a structure formed by a heterocyclic ring selected from the group consisting of

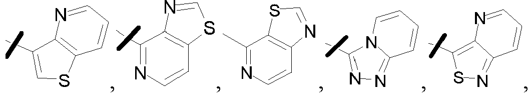

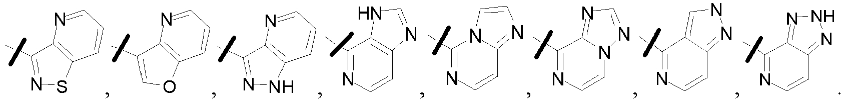

In another preferred embodiment, Z¹, Z² and Z³ are each independently CR⁴.

In another preferred embodiment, Z¹, Z² and Z³ are each independently CH.

In another preferred embodiment, the compound has a structure according to Formula IV: 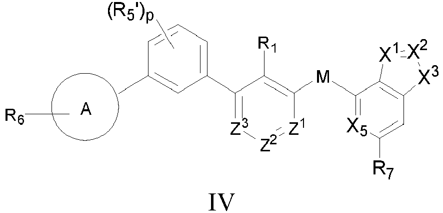 wherin,
X¹ is selected from the group consisting of N or CH;
X² is selected from the group consisting of N or CR₃;
X³ is selected from the group consisting of NR³, O or S;
R₇ is selected from the group consisting of H and halogen.

In another preferred embodiment, -N(Ra)Rb is selected from the following group:

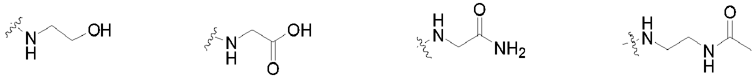

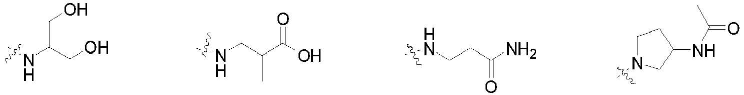

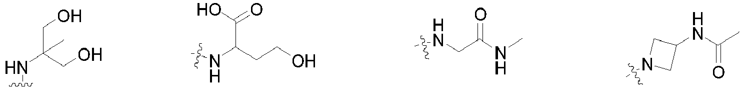

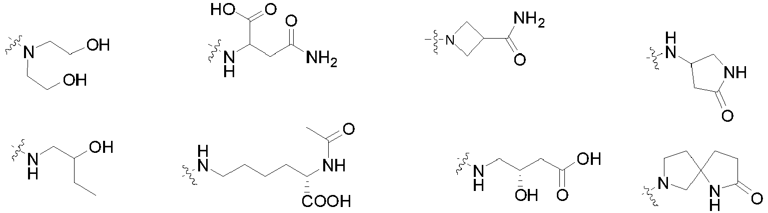

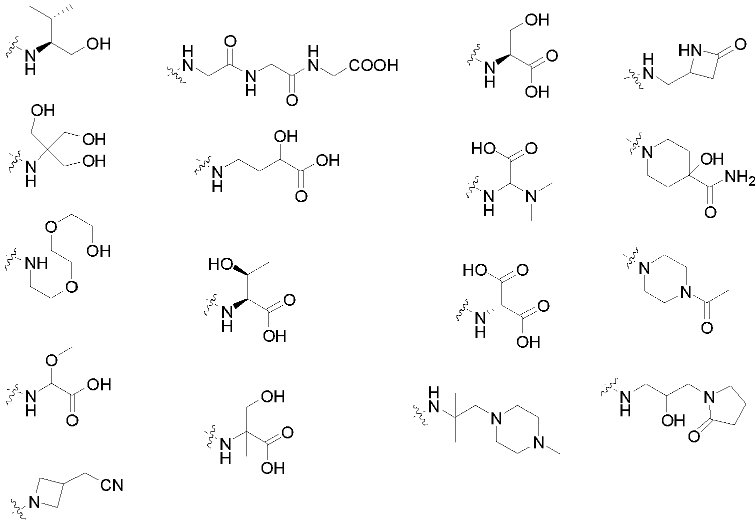

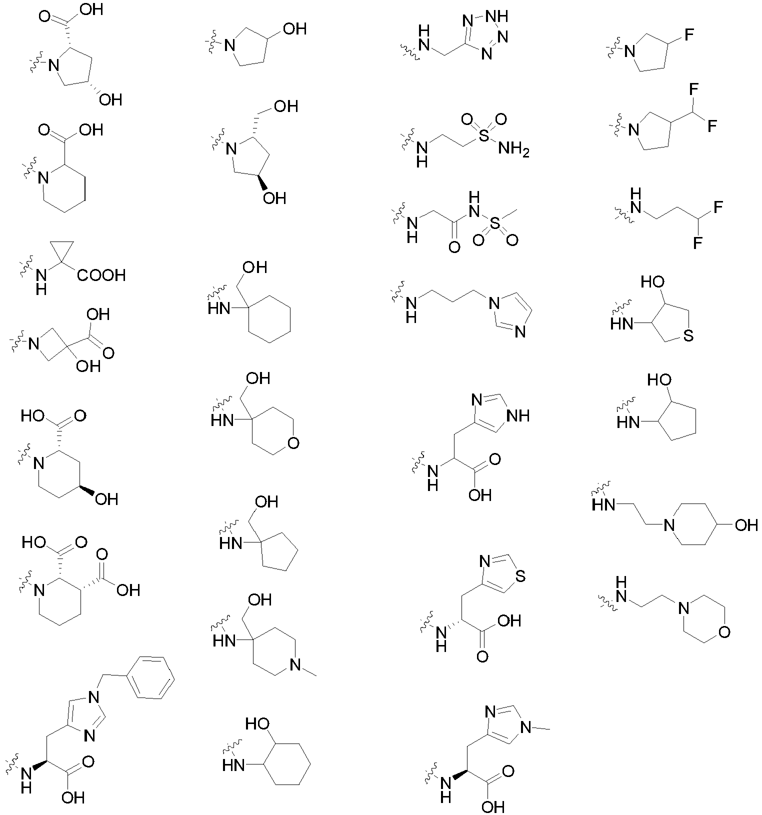

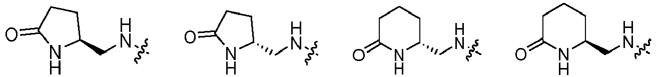

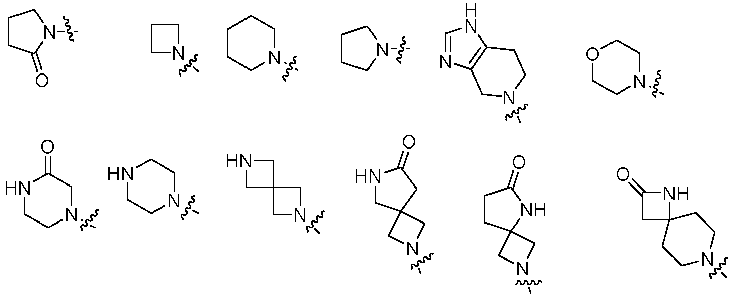

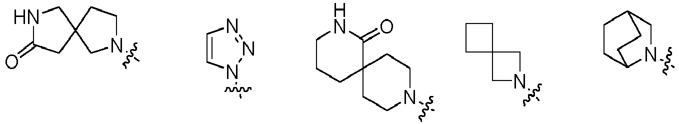

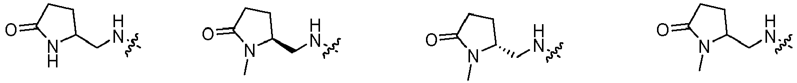

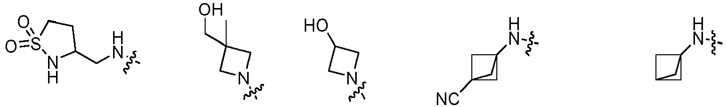

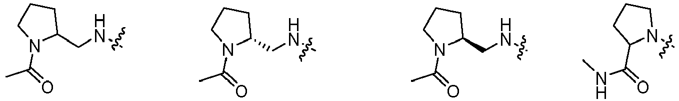

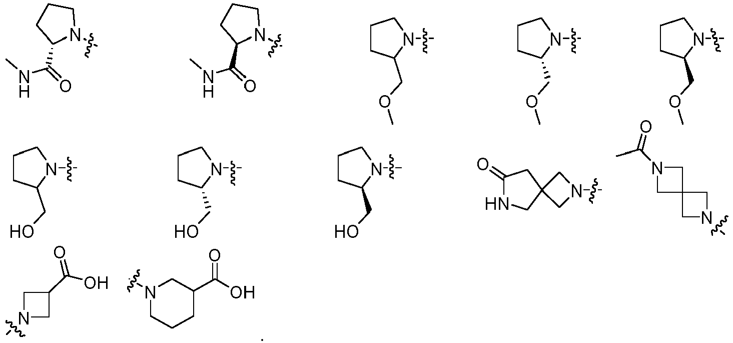

In another preferred embodiment, R⁵ is selected from the group consisting of H, Me, Cl, CN, -O(CH₂)ₙ- (substituted or unsubstituted 5-7 membered heteroaryl), -O(CH₂)ₙ-N(Ra)(Rb).

In another preferred embodiment, R⁵ is selected from H, Me, Cl, CN, or a group selected from the following: 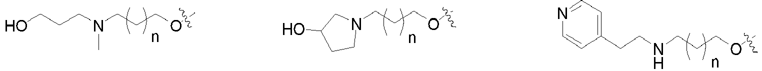 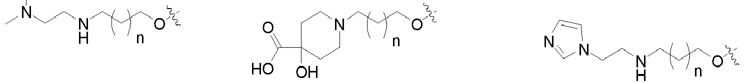 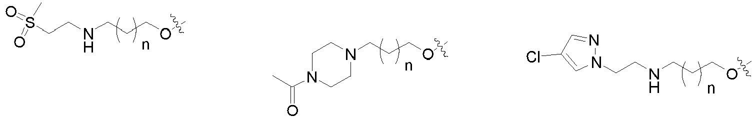 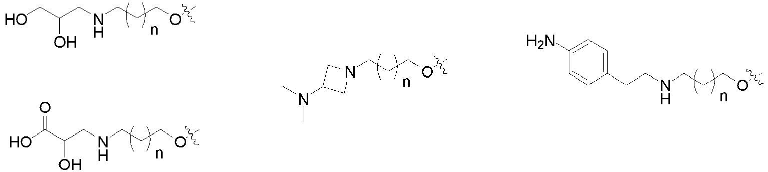 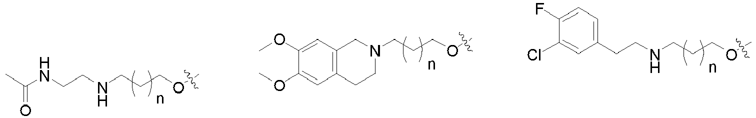 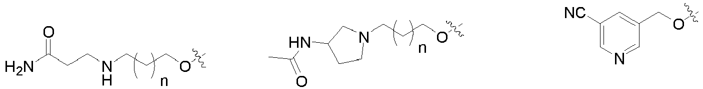 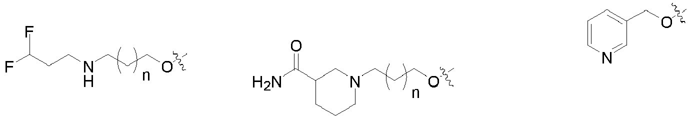 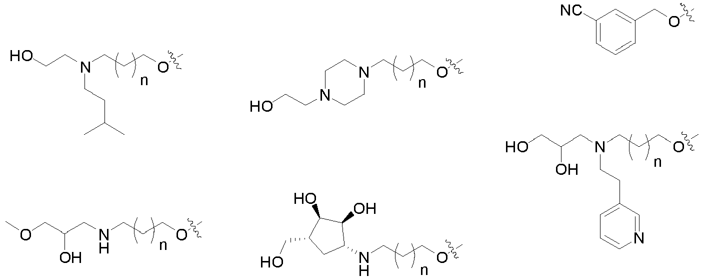 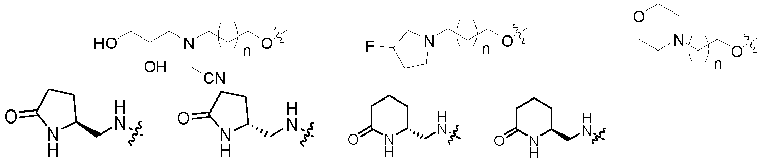 wherein, n=1-2.

In another preferred embodiment, R⁶ is selected from the following group:

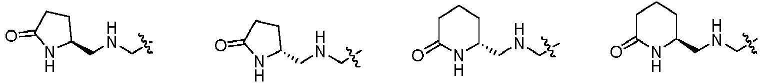

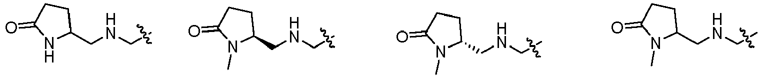

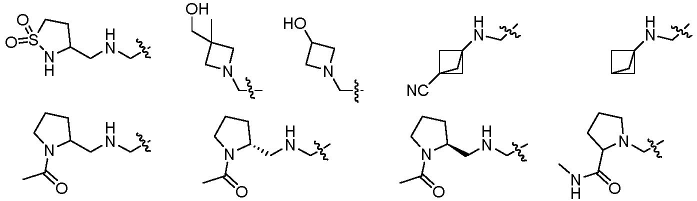

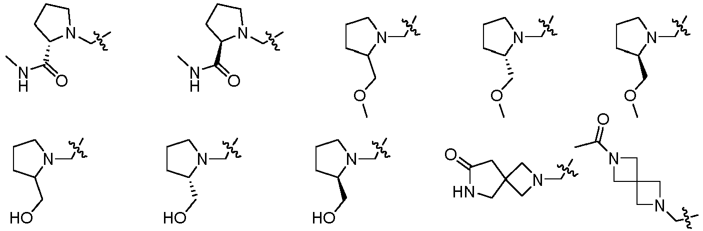

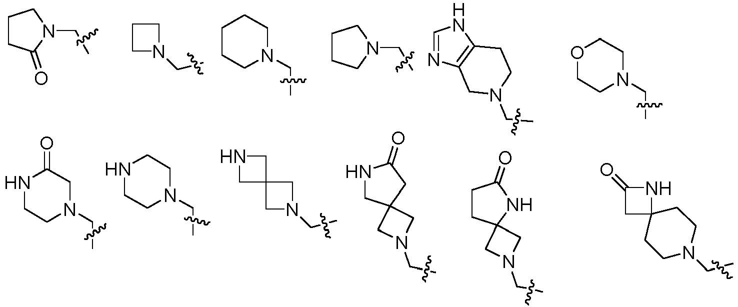

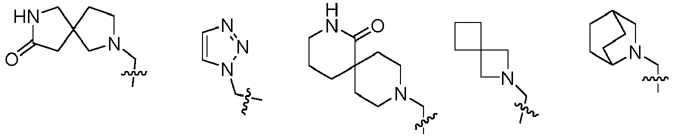

In another preferred embodiment, the compound of Formula I is selected from the following group:

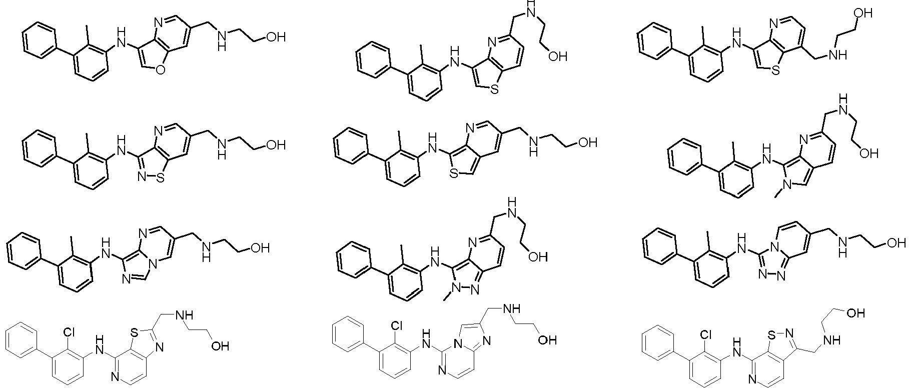

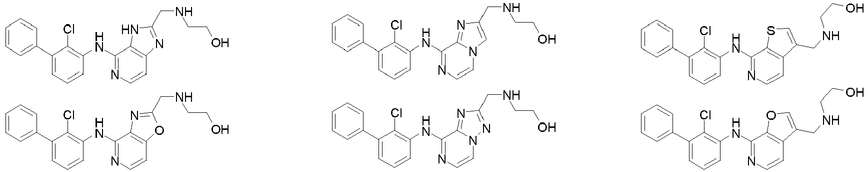

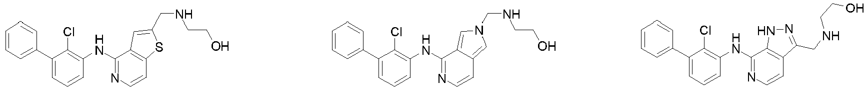

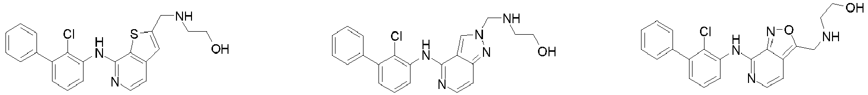

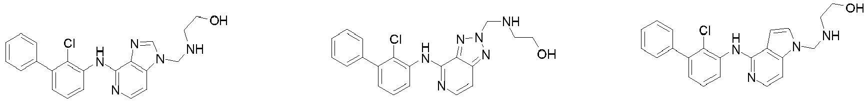

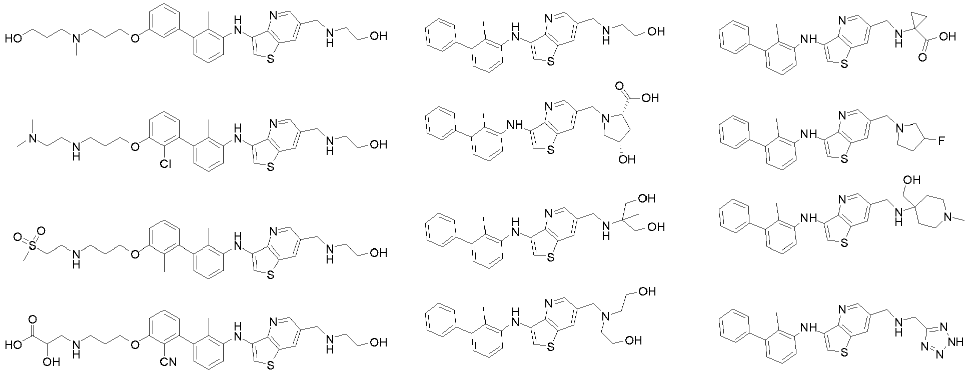

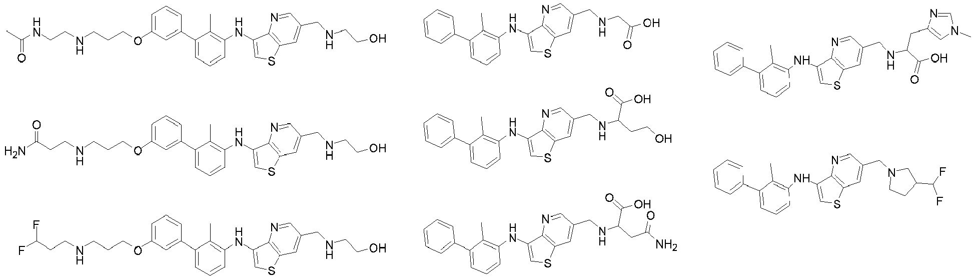

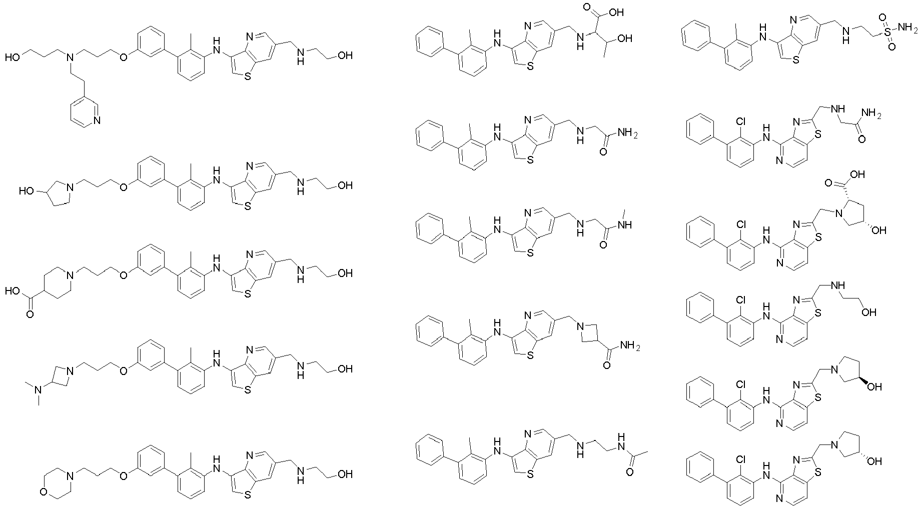

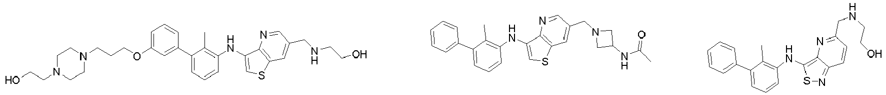

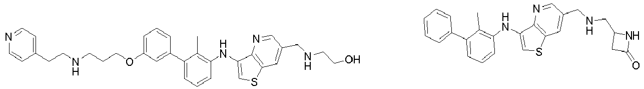

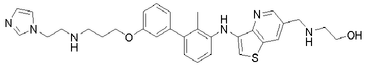

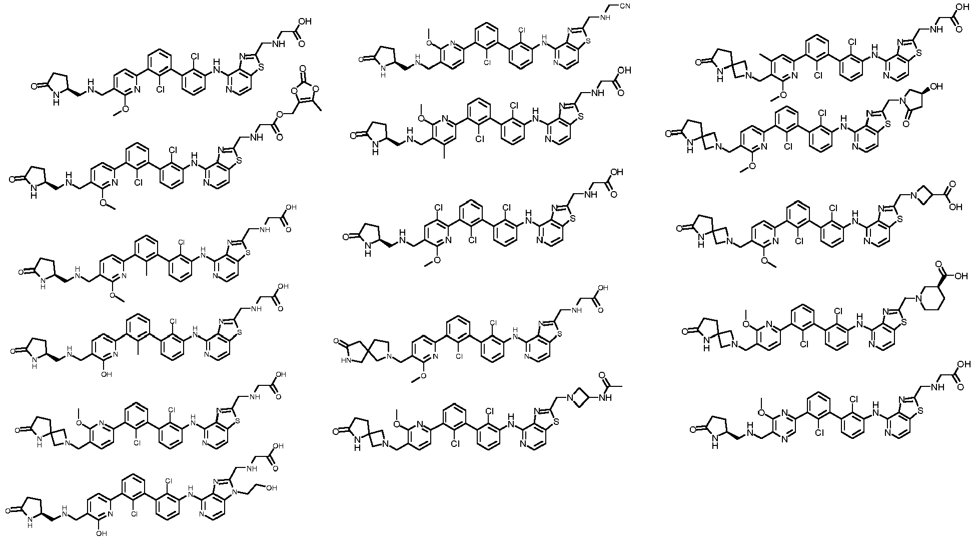

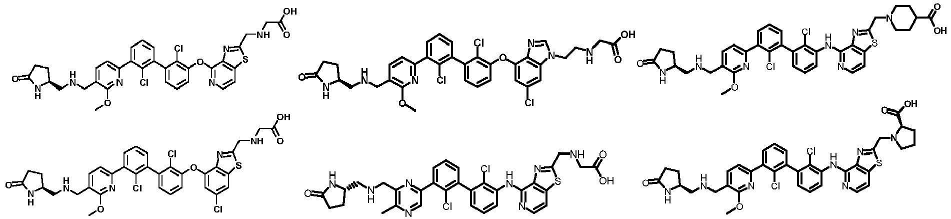

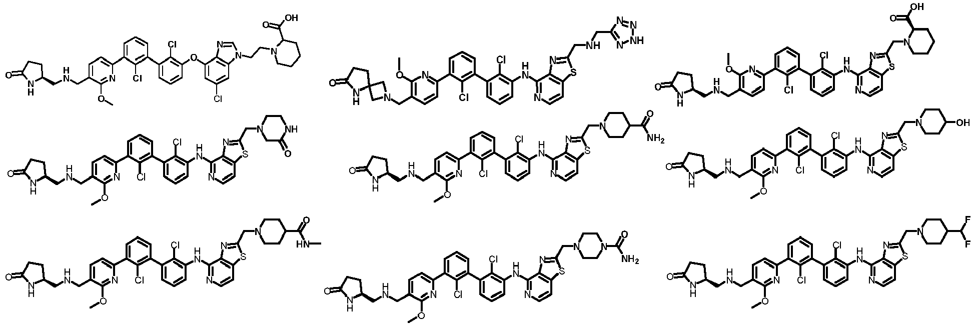

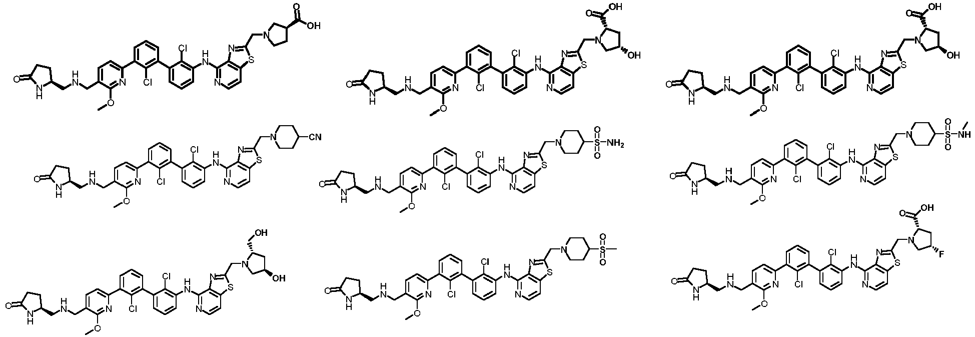

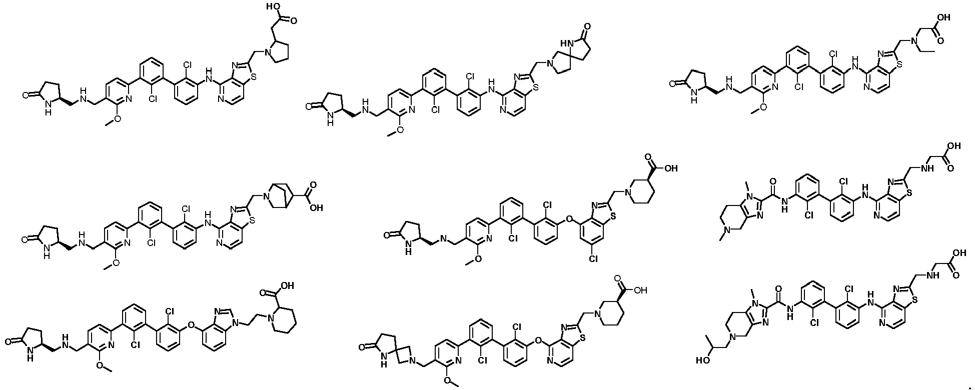

In some embodiments, the compound of Formula I is selected from the following group:

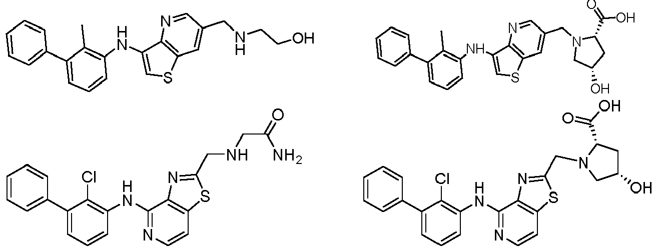

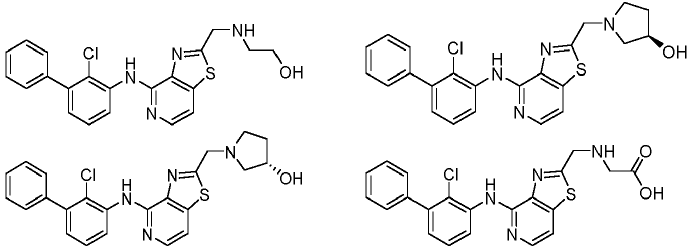

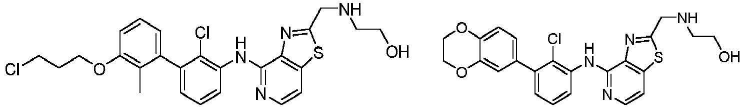

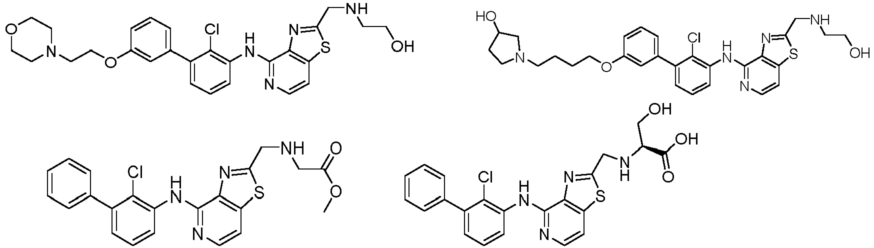

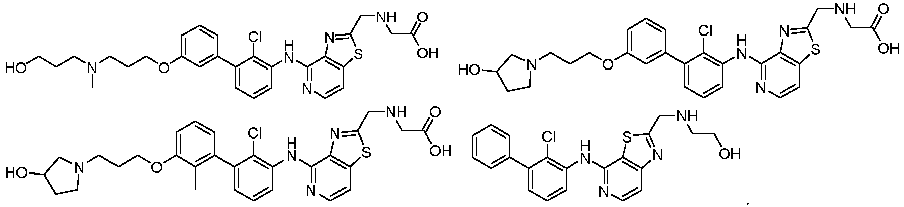

The second aspect of the present invention provides a pharmaceutical composition comprising (1) the compound, or stereoisomers or tautomers thereof, or pharmaceutically acceptable salts, hydrates or solvates thereof according to the first aspect of the present invention; (2) a pharmaceutically acceptable carrier.

The third aspect of the present invention provides the use of the compound, or stereoisomers or tautomers thereof, or pharmaceutically acceptable salts, hydrates or solvates thereof according to the first aspect of the present invention, or the pharmaceutical composition according to the second aspect of the present invention for preparing pharmaceutical compositions for preventing and/or treating diseases related to the activity or expression of PD-1/PD-L1.

In some embodiments, the disease is selected from the group consisting of tumors, pathogen infections, and diseases related to autoimmune response.

In some embodiments, the pharmaceutical composition is used for the treatment of diseases selected from the group consisting of melanoma (e.g. metastatic malignant melanoma), renal cancer (e.g. clear cell carcinoma), prostate cancer (e.g. hormone refractory prostate adenocarcinoma), breast cancer, colon cancer and lung cancer (such as non-small cell lung cancer), bone cancer, pancreatic cancer, skin cancer, head or neck cancer, skin or intraocular melanoma, uterine cancer, ovarian cancer, rectal cancer, anal cancer, gastrointestinal cancer, testicular cancer, uterine cancer, fallopian tube cancer, endometrial cancer, cervical cancer, vaginal cancer, vulva cancer, Hodgkin's disease, non-Hodgkin's lymphoma, esophageal cancer, small bowel cancer, endocrine system cancer, thyroid cancer, parathyroid cancer, adrenal cancer, soft tissue sarcoma, urethral cancer, penile cancer, chronic or acute leukemia (including acute myeloid leukemia, chronic myeloid leukemia, acute lymphoblastic leukemia, chronic lymphocytic leukemia), childhood solid tumors, lymphocytic lymphoma tumors, bladder cancer, kidney or ureter cancer, renal pelvis cancer, central nervous system (CNS) neoplasms/tumors, primary CNS lymphoma, tumor angiogenesis, spinal axis tumors, brainstem glioma, pituitary gland adenoma, Kaposi's sarcoma, epidermoid carcinoma, squamous cell carcinoma, T-cell lymphoma, environmentally induced cancers (including those induced by asbestos), and the combinations thereof. Metastatic cancer, especially metastatic cancer that expresses PD-L1.

In some embodiments, the pharmaceutical composition can be used in combination regimen. Preferably, the combination regimen: combined tumor chemotherapy, other tumor immunotherapeutics (small molecule compounds, antibodies, etc.), radiotherapy, tumor targeted drugs, tumor vaccines (such as human papilloma virus (HPV), hepatitis virus (HBV and HCV) and Kaposi herpes sarcoma virus (KHSV)).

In some embodiments, the pharmaceutical composition is used alone or in combination for the treatment of patients exposed to specific toxins or pathogens, which includes, but is not limited to, the treatment of various viruses, pathogenic bacteria, pathogenic fungi, pathogenic parasites, etc, e.g., infections established by pathogens, such as HIV, hepatitis virus (A, B, C), influenza virus, herpes virus, Giardia, malaria, Leishmania, staphylococcus aureus, pseudomonas aeruginosa.

In some embodiments, the pharmaceutical composition is used to induce therapeutic autoimmune response.

In some embodiments, the pharmaceutical composition is used to treat patients with inappropriate accumulation of other autoantigens, such as amyloid deposits, including Aβ in Alzheimer's disease, cytokines such as TNFα and IgE.

The fourth aspect of the present invention provides the PD-1/PD-L1 inhibitor, comprising the compound as described in the first aspect of the present invention, or stereoisomers or tautomers thereof, or pharmaceutically acceptable salts, hydrates or solvates thereof.

It should be understood that, in the present invention, each of the technical features specifically described above and below (such as those in the Examples) can be combined with each other, thereby constituting new or preferred technical solutions which need not be specified again herein .

### EMBODIMENTS FOR CARRYING OUT THE INVENTION

The inventor designed and synthesized a novel PD-1 small molecule inhibitor after long-term and in-depth research. The inventor completed the present invention on this basis.

### Terms

Unless otherwise defined, all technical and scientific terms used herein have the same meaning as commonly understood by one of ordinary skill in the art to which this invention belongs.

As used herein, when used in reference to a particular recited value, the term "about" means that the value can vary by no more than 1% from the recited value. For example, as used herein, the expression "about 100" includes all the values between 99 and 101 and themselves (eg, 99.1, 99.2, 99.3, 99.4, etc.).

As used herein, the terms "containing" or "including (comprising)" may be opened form, semi-closed form, or closed form. In other words, the terms also include situations such as "essentially consisting of..." or "consisting of..."

### DEFINITIONS

As used herein, the term "alkyl" includes straight or branched alkyl groups. For example, C₁-C₈ alkyl refers to straight or branched alkyls having from 1-8 carbon atoms, such as methyl, ethyl, propyl, isopropyl, butyl, isobutyl, tert-butyl, and the like.

As used herein, the term "alkenyl" includes straight or branched alkenyl groups. For example, C₂-C₆ alkenyl refers to straight or branched alkenyl groups having 2-6 carbon atoms, such as vinyl, allyl, 1-propenyl, isopropenyl, 1-butenyl, 2-butenyl, and the like.

As used herein, the term "alkynyl" includes straight or branched alkynyl groups. For example, "C₂-C₆ alkynyl" refers to straight or branched alkynyls having 2-6 carbon atoms, such as ethynyl, propynyl, butynyl, and the like.

As used herein, the term "C₃-C₈ cycloalkyl" refers to cycloalkyl groups having 3 to 10 carbon atoms. It may be a monocyclic ring, such as cyclopropyl, cyclobutyl, cyclopentyl, cyclohexyl, and the like. It may also be of bicyclic form, such as bridged or spiro ring form. As used herein, the term "C₁-C₈ alkoxyl" refers to straight or branched alkoxy groups having 1-8 carbon atoms; for example, methoxy, ethoxy, propoxy, isopropoxy, butoxy, isobutoxy, tert-butoxy, and the like.

As used herein, the term "3-10 membered heterocycloalkyl with 1-3 heteroatoms selected from the group consisting of N, S and O" refers to a saturated or partially saturated cyclic group having 3-10 atoms, wherein 1-3 atoms are heteroatoms selected from the group consisting of N, S and O. It may be a monocyclic ring or bicyclic form, such as bridged or spiro ring form. Specific examples may be oxetane, azetidine, tetrahydro-2H-pyranyl, piperidinyl, tetrahydrofuranyl, morpholinyl and pyrrolidinyl, and the like.

As used herein, the term "C₆-C₁₀ aryl" refers to aryl groups having 6 to 10 carbon atoms, such as phenyl, naphthyl, and the like.

As used herein, the term "5-10 membered heteroaryl having 1-3 heteroatoms selected from the group consisitng of N, S and O" refers to cyclic aromatic groups having 5-10 atoms, of which 1-3 is selected from the group consisting of N, S and O. It may be a monocyclic ring or fused ring form. Specific examples may be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazyl, furyl, thienyl, isoxazolyl, thiazolyl, oxazolyl, etc.

Unless otherwise specified, the groups of the present invention can be substituted with substituents selected from the group consisting of halogen, nitrile, nitro, hydroxyl, amino, C₁-C₆ alkyl-amino, C₁-C₆ alkyl, C₂-C₆ alkenyl, C₂-C₆ alkynyl, C₁-C₆ alkoxyl, halogenated C₁-C₆ alkyl, halogenated C₂-C₆ alkenyl, halogenated C₂-C₆ alkynyl, halogenated C₁-C₆ alkoxyl, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂ -C₆ alkenyl-carbonyl, C₃-C₆ cycloalkylcarbonyl, C₁-C₆ alkyl-sulfonyl, etc.

As used herein, "halogen" or "halogen atom" refers to F, Cl, Br, and I. More preferably, the halogen or halogen atom is selected from F, Cl and Br. "Halogenated" means substituted by an atom selected from F, Cl, Br, and I.

Unless otherwise specified, the structural Formula described herein are intended to include all isomeric forms (such as enantiomeric, diastereomeric, and geometric isomers (or conformational isomers)): for example, R, S configuration of asymmetrical centers, (Z), (E) isomers of double bonds, etc. Therefore, the single stereochemical isomers or enantiomers, diastereomers or geometric isomers (or conformers) of the compounds of the invention, or mixtures thereof all fall within the scope of the invention.

As used herein, the term "tautomer" means that structural isomers having different energies can exceed the low energy barrier and thereby transform between each other. For example, proton tautomers (proton shift) includes interconversion by proton transfer, such as 1H-carbazole and 2H-carbazole. Valence tautomers include interconversion through some bonding electron recombination.

As used herein, the term "solvate" refers to a complex of specific ratio formed by a compound of the invention coordinating to a solvent molecule.

### Compound of Formula I

Provided herein is a compound according to Formula I, the stereoisomers or tautomers thereof, or pharmaceutically acceptable salts, hydrates or solvates thereof: 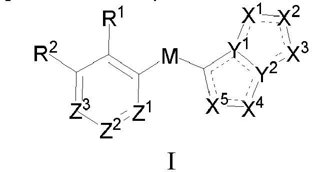 wherein,
M is selected from O or NH;
X² is selected from the group consisting of N, NR³, CR³, O, S, N=CR³, CR³=N or CR³=CR³;
X⁴ is selected from the group consisting of N, CH, O, S, N=CH, CH=N or CH=CH; and one and merely one of X² and X⁴ is group selected from the group consisting of CR³=CR³, N=CR³ or CR³=N (R₃ is H in X⁴);
X¹ and X⁵ are independently selected from the group consisting of N, CH, O or S;
X³ is selected from the group consisting of N, CR³, O or S; and
at least one of X³ and X⁴ is O, S or N;
Y¹ and Y² are independently N or C;
Z¹, Z² and Z³ are independently N or CR⁴;
R¹ is selected from the group consisting of H, halogen, CN, substituted or unsubstituted C₁-C₆ alkyl, substituted or unsubstituted C₁-C₆ alkoxyl;
R² is selected from the group consisting of substituted or unsubstituted C₆-C₁₀ aryl, substituted or unsubstituted 5-10 membered heteroaryl with 1-3 heteroatoms selected from N, S and O, or substituted or unsubstituted 5-10 membered heterocyclyl with 1-3 heteroatoms selected from N, S and O; and one or more H of R² are substituted by R⁵;
R³ is selected from the group consisting of H substituted or unsubstituted C₁-C₆ alkyl, or 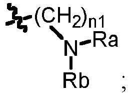 and at least one of R³ is 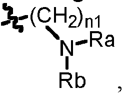 n1 is 1, 2, 3 or 4;
Ra and Rb are independently selected from the group consisting of H, -(C=O)-substituted or unsubstituted C₁-C₈ alkyl, substituted or unsubstituted C₁-C₈ alkyl, substituted or unsubstituted C₂-C₆ alkenyl, substituted or unsubstituted C₂-C₆ alkynyl, substituted or unsubstituted C₁-C₈ alkylamino, substituted or unsubstituted C₁-C₈ alkoxyl, substituted or unsubstituted C₃-C₁₀ cycloalkyl, substituted or unsubstituted 3-10 membered heterocyclyl with 1-3 heteroatoms selected from the group consisting of N, S and O, substituted or unsubstituted C₆-C₁₀ aryl, substituted or unsubstituted 5-10 membered heteroaryl with 1-3 heteroatoms selected from the group consisting of N, S and O, or substituted or unsubstituted 5-10 membered heterocyclyl with 1-3 heteroatoms selected from the group consisting of N, S and O; or
or Ra and Rb and adjacent N atoms together form a substituted or unsubstituted 5-10 membered heterocyclyl with 1-3 heteroatoms selected from the group consisting of N, S and O;
R⁴ is selected from the group consisting of H, halogen, CN, substituted or unsubstituted C₁-C₆ alkyl, substituted or unsubstituted C₁-C₆ alkoxyl;
R⁵ is selected from H, CN, halogen, substituted or unsubstituted (-L₁-L₂-(CH₂)_{q} - (substituted or unsubstituted 5-7 membered heteroaryl)), substituted or unsubstituted (-L₁-L₂-(CH₂)_{q}-N(Ra)(Rb)), -O-substituted or unsubstituted (-(CH₂)_{q}O-(CH₂)_{q}-N(Ra)(Rb)), or substituted or unsubstituted group selected from the group consisting of C₆-C₁₀ aryl group, 5-10 membered heteroaryl with 1-3 heteroatoms selected from the group consisting of N, S and O, -L₁-L₂-6-10 membered aryl-(CH₂)_{q}-N(Ra)(Rb), -L₁-L₂-5-10 membered heteroaryl-(CH₂)_{q}-N(Ra)(Rb); wherein L₁ and L₂ are each independently selected from the group consisting of none, substituted or unsubstituted C₁-C₈ alkylene, -NH-C(=O)-NH-, -C(=O)-NH-, -O-, -S- or -NH-; or two R⁵ and the connected carbon atoms together form a group selected from substituted or unsubstituted 5-7 membered heteroaryl, substituted or unsubstituted 5-7 membered heterocyclyl;
q is selected from the group consisting of 0, 1, 2, 3, 4, 5, 6, 7 or 8;
unless otherwise specified, "substituted" refers to being substituted by one or more (for example, 2, 3, 4, etc.) substituents selected from the group consisting of halogen, C₁-C₆ alkoxyl, halogenated C₁-C₆ alkoxyl, C₃-C₈ cycloalkyl, halogenated C₃-C₈ cycloalkyl, methyl sulfone group, -S(=O)₂NH₂, -S(=O)₂NHCH₃, oxo(=O), -CN hydroxyl, -NH₂, C₁-C₆ amine carboxy, C₁-C₆ amide (-C(=O)-N(Rc)₂ or -NH-C(=O)(Rc), Rc is H or C₁-C₅ alkyl), 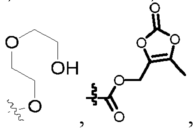 or substituted or unsubstituted groups selected from the group consisting of C₁-C₆ alkyl, C₆-C₁₀ aryl, 5-10 membered heteroaryl with 1-3 heteroatoms selected from N, S, O, and 5-10 membered heterocyclyl (including fused ring, spiro ring) with 1-3 heteroatoms selected from N, S and O, - (CH₂) -C₆-C₁₀ aryl, -(CH₂)- (5-10 membered heteroaryl with 1-3 heteroatoms selected from N, S and O), and the substitution is substituted by substituents selected from the group consisting of halogen, C₁-C₆ alkyl, C₁-C₆ alkoxyl, oxo, -CN, -OH, C₆-C₁₀ aryl, 5-10 membered heteroaryl with 1-3 heteroatoms selected from N, S and O, -O-(CH₂)₄-CN;
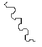 is the attachment site of the group;
each 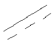 is independently a single bond or a double bond;
attached condition is that the compound of Formula I has a chemically stable structure.

The preferred compound of Formula I is the specific compound shown in the example of the present application.

### PREPARATION OF COMPOUND OF FORMULA I

The invention also provides a method for preparing the compound as described in the first aspect of the invention, which includes the steps: in appropriate solvent, compound 1 was substituted to obtain compound 2. Compound 2 and compound 3 are allowed to react to obtain compound 4 with the presence of palladium catalyst, alkali and phosphine compound. Compound 4 is reduced to obtain compound 5, and compound 5 is subjected to reductive amination reaction to obtain the compound of Formula 6.

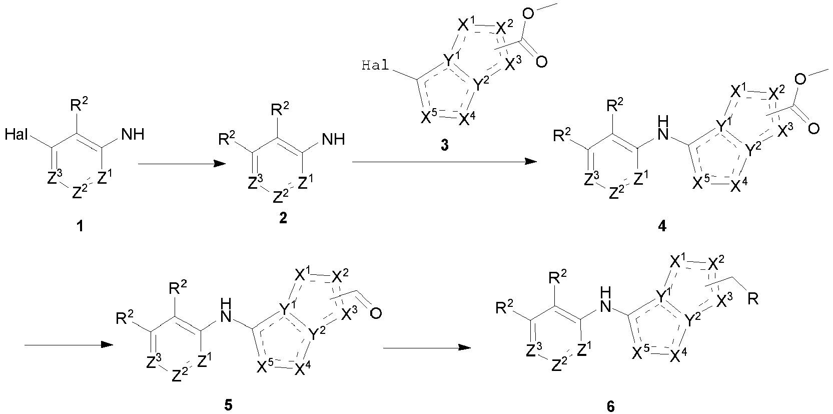

### PHARMACEUTICAL COMPOSITION AND THE ADMINISTRATION THEREOF

Since the compound herein has excellent PD-1 inhibitory activity, the compound of the present invention and various crystal forms thereof, pharmaceutically acceptable inorganic or organic salts, hydrates or solvates thereof, and pharmaceutical composition containing the compound according to the present invention as main active ingredient can be used to prevent and/or treat diseases related to the PD-1/PD-L1 signaling pathway (for example, cancer).

The pharmaceutical composition of the invention comprises the compound of the present invention in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg polymorphs of the invention per dose, preferably, 10-200 mg polymorphs of the invention per dose. Preferably, the "dose" is a capsule or tablet.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral, parenteral (intravenous, intramuscular or subcutaneous).

Solid dosage forms for oral administration include capsules, tablets, pills, powders and granules. In these solid dosage forms, the active compounds are mixed with at least one conventional inert excipient (or carrier), such as sodium citrate or CaHPO4, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Compounds of the present invention can be administrated alone, or in combination with any other pharmaceutically acceptable compounds.

In the case of co-administration, the pharmaceutical composition can also include one or more other pharmaceutically acceptable compounds. One or more other pharmaceutically acceptable compounds may be used simultaneously, separately or sequentially with the compound of the present invention.

When the pharmaceutical compositions are used, a safe and effective amount of compound of the present invention is applied to a mammal (such as human) in need of, wherein the dose of administration is a pharmaceutically effective dose. For a person weighed 60 kg, the daily dose is usually 1-2000 mg, preferably 20-500 mg. Of course, the particular dose should also depend on various factors, such as the route of administration, patient healthy status, which are well within the skills of an experienced physician.

In the preferred embodiments of the present invention, the pharmaceutical compositions can be used:
(1) for the treatment of various tumors, including but not limited to melanoma (e.g. metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer), bone cancer, pancreatic cancer, skin cancer, head or neck cancer, skin or intraocular melanoma, uterine cancer, ovarian cancer, rectal cancer, anal cancer, gastrointestinal cancer, testicular cancer, uterine cancer, fallopian tube cancer, endometrial cancer, cervical cancer, vaginal cancer, vulva cancer, Hodgkin's disease, non-Hodgkin's lymphoma, esophageal cancer, small bowel cancer, endocrine system cancer, thyroid cancer, parathyroid cancer, adrenal cancer, soft tissue sarcoma, urethral cancer, penile cancer, chronic or acute leukemia (including acute myeloid leukemia, chronic myeloid leukemia, acute lymphoblastic leukemia, chronic lymphocytic leukemia), childhood solid tumors, lymphocytic lymphoma tumors, bladder cancer, kidney or ureter cancer, renal pelvis cancer, central nervous system (CNS) neoplasms/tumors, primary CNS lymphoma, tumor angiogenesis, spinal axis tumors, brainstem glioma, pituitary gland adenoma, Kaposi's sarcoma, epidermoid carcinoma, squamous cell carcinoma, T-cell lymphoma, environmentally induced cancers (including those induced by asbestos), and the combination thereof. Metastatic cancer, especially metastatic cancer that expresses PD-L1.
(2) in combination administration regimes, such as combined tumor chemotherapy, other tumor immunotherapeutics (small molecule compounds, antibodies, etc.), radiotherapy, tumor-targeted drugs, tumor vaccines, such as human papilloma virus (HPV), hepatitis virus (HBV and HCV) and Kaposi herpes sarcoma virus (KHSV)). It can be administered before, after, or simultaneously with the agent, or it can be co-administered with other known therapies.
(3) alone or in combination for the treatment of patients exposed to specific toxins or pathogens, which includes, but is not limited to, the treatment of various viruses, pathogenic bacteria, pathogenic fungi, pathogenic parasites, etc., e.g., infections established by pathogens, such as HIV, hepatitis virus (A, B, C), influenza virus, herpes virus, Giardia, malaria, Leishmania, staphylococcus aureus, pseudomonas aeruginosa..
(4) to induce therapeutic autoimmune response to treat patients with inappropriate accumulation of other autoantigens, such as amyloid deposits, including Aβ in Alzheimer's disease, cytokines such as TNFa and IgE .

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

In each embodiment:
Analysis Method I
LCMS: Agilent 6110, UV detector: G1315D
Chromatography column: Xbridge C18 3.0×50 mm, 2.5 uM, column temperature 30 °C
Mobile phase: A: H₂O (0.05% TFA), B: acetonitrile, gradient elution: 0-1 min 10%B, 1-8 min 10-95%B, 9 min 95%B

### Synthesis of Intermediate A:

### 3-aminothieno[3,2-b]ridine-6-carbonitrile

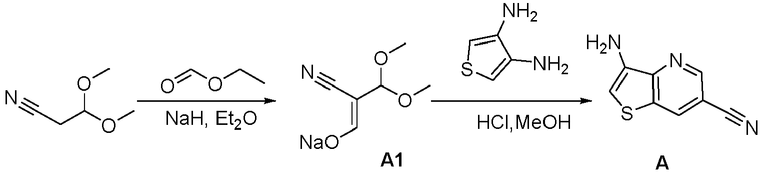

### (E)-2-cyano-3,3-dimethoxyprop-1-en-1-alkoxide sodium

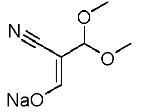

To the mixture of sodium hydride (2.08 g, 52.1 mmol) in ether (100 mL), compound 3,3-dimethoxypropionitrile (5 g, 43.4 mmol) and ethyl formate (6.43 g, 86.8 mmol) was added successively. The reaction mixture was stirred at room temperature for two days. The resulting mixture was filtered, the solid was washed three times with ether, and dried to obtain the target compound (5 g, 70%).

### 3-aminothieno[3,2-b]pyridine-6-carbonitrile

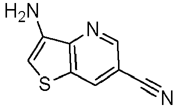

Concentrated hydrochloric acid (0.3 mL, 12N) was added to the solution of compound A1 (0.3 g, 1.8 mmol) in methanol (20 mL). The reaction mixture was stirred at room temperature for 10 minutes followed by adding thiophene-3,4-diamine (0.3 g, 1.8 mmol) in methanol (10 mL). The reaction mixture was stirred at 80°C for 2 hours, then concentrated and the crude product was purified by prep-HPLC to obtain the target compound as white solids (100 mg, 30%).
MS-ESI: m/z 176.0[M+H]⁺.
¹H NMR (400 MHz, CDCl₃): *δ* 8.96 (s, 1H), 8.78 (s, 1H), 7.44 (s, 1H).

### Synthesis of Intermediate B:

### 4-chloro-2-(methoxymethyl)thiazolo[4,5-c]pyridine

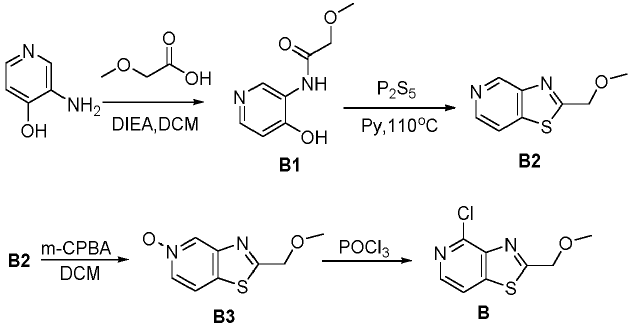

### N-(4-hydroxypyridin-3-yl)-2-methoxyacetamide

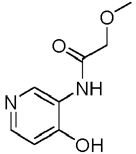

Oxalyl chloride (218.2 mmol, 18.4 mL) was added successively and dropwisely to a solution of 2-methoxyacetic acid (9.8 g, 109.1 mmol) and N,N-dimethylformamide (0.1 mL) in dichloromethane (30 mL) at room temperature. After 2 hours, the reaction mixture was concentrated, and the crude product was dissolved in dichloromethane (10 mL). The solution was added dropwisely to the mixture of 3-aminopyridine-4-phenol (8.0 g, 4.54 mmol) and diisopropylamine (38.5 mL, 218.1 mmol) in dichloromethane (100 ml) at room temperature. The reaction mixture was heated to reflux for 2 hours and then stirred overnight at room temperature. Concentrated and the crude product was purified by silica gel column (dichloromethane/methanol=20/1) to obtain the target compound as a white solid (3.1 g, 23%).
¹H NMR (400 MHz, CDCl₃): *δ* 11.51 (brs, 1H), 9.18 (s, 1H), 8.68 (s, 1H), 7.68 (dd, *J*= 7.2, 0.8 Hz, 1H), 6.25 (d, *J=* 7.2 Hz, 1H), 4.16 (s, 2H), 3.41 (s, 3H).

### 2-(Methoxymethyl)thiazolo[4,5-c]pyridine

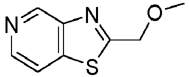

Phosphorus pentasulfide (4.5 g, 20.4 mmol) was added to the solution of compound B1 (3.1 g, 17.0 mmol) in pyridine (30 mL) at room temperature, then the reaction mixture was heated to 110°C for 3 hours and concentrated. The crude product was dissolved in ethyl acetate, washed with brine and dried over anhydrous sodium sulfate. The mixture was filtered and concentrated, and the crude product was purified by silica gel column chromatography (petroleum ether/ethyl acetate= 10/1-3/1) to obtain the target compound as a yellow solid (700 mg, 22%) .
¹H NMR (400 MHz, CDCl₃): *δ* 9.30 (s, 1H), 8.53 (d, J= 5.6 Hz, 1H), 7.87 (dd, *J*= 7.2,0.8 Hz, 1H), 4.89 (s, 2H), 3.57 (s, 3H).

### 5-oxidated 2-(methoxymethyl)thiazolo[4,5-c]pyridine

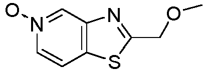

3-chloroperoxybenzoic acid (1.14 g, 4.64 mmol) was added in portions to the solution of compound B2 (760 mg, 4.22 mmol) in dichloromethane (10 mL) at room temperature. The reaction mixture was stirred overnight at room temperature and concentrated. The crude product was purified by silica gel column chromatography (dichloromethane/methanol=20/1 elution) to obtain the target compound (800 mg, 96%) as a yellow solid.
MS (ESI): m/z= 197.1 [M+H]⁺.

### 4-chloro-2-(methoxymethyl)thiazolo[4,5-c]pyridine

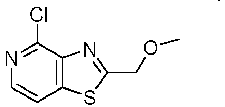

Compound B3 (800 mg, 4.08 mmol) was added in portions to phosphorus oxychloride (10 mL) at room temperature, then the reaction mixture was heated to 100°C and stirred for 1 hour, concentrated and dried.The crude product was dissolved in ethyl acetate, washed with saturated sodium bicarbonate solution and saturated brine, dried over anhydrous sodium sulfate and concentrated, and the crude product was purified by silica gel column chromatography (petroleum ether/ethyl acetate=30/1-10/1) to obtain the target compound (600 mg, 69 %) as a yellow solid.
MS (ESI): m/z= 215.2 [M+H]⁺.
¹HNMR(400 MHz, CDCl₃): *ð* 8.31 (d, *J=* 5.6 Hz, 1 H), 7.80 (d, *J =* 5.6 Hz, 1 H), 4.92 (s, 2 H), 3.59 (s, 3 H).

### Synthesis of Intermediate C:

### 6-(2-Chloro-3-(4,4,5,5-tetramethyl-1,3,2-dioxaborolan-2-yl)phenyl)-2-methoxynicotinic aldehyde

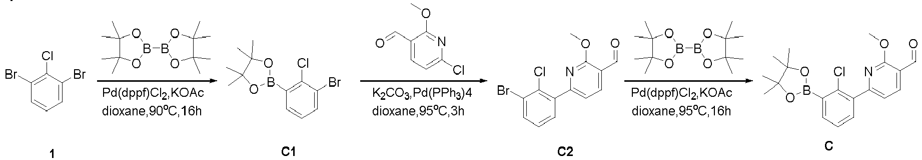

### 2-(3-Bromo-2-chloro-phenyl)-4,4,5,5-tetramethyl-[1,3,2]dioxaborolane

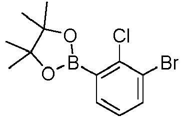

1,1'- bis (diphenylphosphino) ferrocene palladium dichloride (678 mg, 0.93 mmol), pinacol diborate (4.7 g, 18.52 mmol), and potassium acetate (5.44 g, 55.56 mmol) were added to the solution of 1,3-dibromo-2-chlorobenzene (5 g, 18.52 mmol) in 1,4-dioxane (200 mL). The mixture was stirred overnight at 90 degrees under nitrogen. After cooling, the reaction solution was poured into water (200 mL), and extracted with ethyl acetate (200 mL*3). The organic phase was dried over anhydrous sodium sulfate, filtered, and concentrated. The residue was purified by normal phase silica gel column (petroleum ether: ethyl acetate=100:1) to obtain the target compound C1 (2.7 g, 46%) as a white solid.
¹HNMR(400 MHz, CDCl₃): δ 7.67-7.65 (m, 1H), 7.60-7.58 (m, 1H), 7.11-7.07 (m, 1H), 1.36 (s, 12H).

### 6-(3 -bromo-2-chlorophenyl)-2-methoxynicotine aldehyde

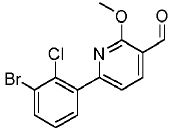

A mixture of C1 (1.71 g, 5.39 mmol), 6-chloro-2-methoxypyridine-3-carbaldehyde (1.02 g, 5.93 mmol), tetrakis-triphenylphosphine palladium (312 mg, 0.27 mmol), 1,4 - dioxane (27 mL), water (2.7 mL), and potassium carbonate (1.49 g, 10.77 mmol) was stirred at 95 °C for 3 hours under nitrogen. After cooled to room temperature, the reaction solution was diluted with dichloromethane (100 mL), and then washed with water (50 mL) and saturated brine (50 mL). The organic phase was dried over anhydrous sodium sulfate, filtered and concentrated. The residue was purified by normal phase silica gel column (petroleum ether: ethyl acetate=100:1) to obtain the target compound C2 (1.24 g, 70%) as white solid.
¹HNMR (400 MHz, CDCl₃): δ 10.41 (s, 1H), 8.20-8.18 (m, 1H), 7.73-7.71 (m, 1H), 7.52-7.50 (m, 1H), 7.30-7.22 (m, 2H), 4.10 (s, 3H).

### 6-(2-Chloro-3-(4,4,5,5-tetramethyl-1,3,2-dioxaborolan-2-yl)phenyl)-2-methoxynicotine aldehyde

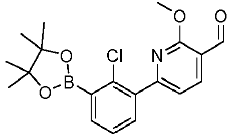

To a solution of C2 (720 mg, 2.2 mmol) in 1,4-dioxane (20 mL), 1,1'-bis(diphenylphosphino) ferrocene palladium(II) dichloride (161 mg , 0.22 mmol), pinacol diborate (616 mg, 2.43 mmol), and potassium acetate (606 mg, 6.17 mmol) were added. The mixture was stirred overnight at 95 °C under nitrogen. The reaction solution was cooled, poured into water (50 mL), and extracted with ethyl acetate (50 mL*3). The organic phase was dried over anhydrous sodium sulfate, filtered and concentrated. The residue was purified by normal phase silica gel column (petroleum ether: ethyl acetate=100:1) to obtain the target compound C (500 mg, 46%) as a white solid.
¹H NMR (400 MHz, CDCl₃): δ 10.41 (s, 1H), 8.16 (d, *J=* 8Hz, 1H) 7.72-7.70 (m, 1H), 7.62-7.60 (m, 1H), 7.37-7.31 (m, 2H), 4.09 (s, 3H), 1.37 (s, 12H).

### Example 1:

### 2-(((3-((2-Methyl-[1,1'-biphenyl]-3-yl)amino)thieno[3,2-b]pyridin-6-yl)methyl)amino)ethane-1-ol

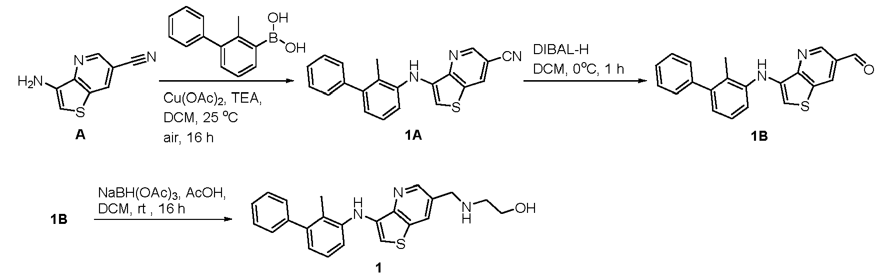

### 3-((2-methyl-[1,l'-biphenyl]-3-yl)amino)thieno[3,2-b]pyridine-6-carbonitrile.

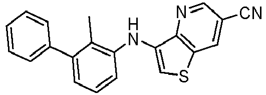

To the solution of intermediate A (65 mg, 0.37 mmol), (2-methyl-[1,1'-biphenyl]-3-yl)boronic acid (157 mg, 0.74 mmol) in dichloromethane (5 mL), copper acetate (67 mg, 0.37 mmol) and triethylamine (75 mg, 0.74 mmol) were added. The reaction solution was stirred at room temperature for 16 hours under air; the reaction solution was concentrated, and the residue was separated and purified by normal phase chromatography (petroleum ether/acetic acid = 1.5/1) to obtain the target compound 1A (28 mg, 22%) as a yellow solid.
MS (ESI): m/z = 342.1 [M+H]⁺.

### 3-((2-methyl-[1,r-biphenyl]-3-yl)amino)thieno[3,2-b]pyridine-6-carbaldehyde

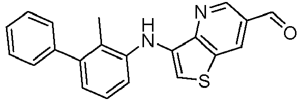

To a solution of compound 1A (28 mg, 0.08 mmol) in anhydrous dichloromethane (4 mL), diisobutylaluminum hydride (0.1 mL, 0.10 mmol, 1 M in toluene) was added dropwisely in an ice bath. The reaction solution was slowly warmed to room temperature and stirred for 1 hour; The reaction solution was quenched by water in an ice bath. The crude product was separated and purified by normal phase chromatography (petroleum ether/ethyl acetate=1/1) to obtain the target compound 1B (20 mg, 74%) as a yellow solid.
MS (ESI): m/z = 345.1 [M+H]⁺.

### 2-(((3-((2-methyl-[1,1'-biphenyl]-3-yl)amino)thieno[3,2-b]pyridin-6-yl)methyl)amino) ethane-1-ol

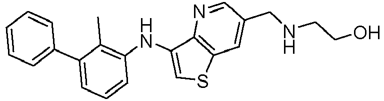

Acetic acid (1 drop) was added to the solution of compound 1B (20 mg, 0.060 mmol), 2-aminoethane-1-ol (11 mg, 0.18 mmol) in dichloromethane (3 mL). After the reaction solution was stirred for 2 hours at room temperature, sodium triacetoxyborohydride (64 mg, 0.30 mmol) was added, and the reaction solution was stirred overnight at room temperature. The reaction solution was concentrated and the crude product was purified byprep-HPLC to obtain the target compound (6.8 mg, 30%) as a white solid.
MS (ESI): m/z = 390.3 [M+H]⁺.
¹H NMR (400 MHz, MeOH-*d*₄)*δ* 8.63 (s, 1H), 8.30 (s, 1H), 7.47-7.28 (m, 6H), 7.22-7.20 (m, 1H), 7.00 (s, 1H), 6.86 (d, *J=* 7.4 Hz, 1H), 3.98 (s, 2H), 3.68 (t, *J=* 5.6 Hz, 2H), 2.77 (t, *J=* 5.6 Hz, 2H), 2.21 (s, 3H).

### Example 2

### (2S,4S)-4-hydroxy-1-((3-((2-methyl-[1,1'-biphenyl]-3-yl)amino)thieno[3,2-b]pyridine-6-yl)methyl)pyrrolidine-2-carboxylic acid

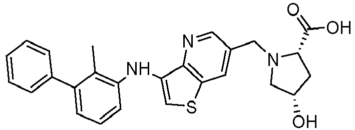

The target compound was prepared from compound 1B and (2S,4S)-4-hydroxypyrrolidine-2-carboxylic acid under conditions similar to Example 5.MS (ESI): m/z = 460.2 [M+H]⁺.
¹H NMR (400 MHz, MeOH-*d*₄) *δ* 8.75 (s, 1H), 8.47 (s, 1H), 8.42 (s, 1H), 7.45-7.37 (m, 3H), 7.35-7.28 (m, 3H), 7.22-7.20 (m, 1H), 7.07 (s, 1H), 6.87 (dd, *J=* 7.7, 1.2 Hz, 1H), 4.51-4.36 (m, 3H), 3.88-3.80 (m, 1H), 3.47-3.39 (m, 1H), 3.23-3.16 (m, 1H), 2.68-2.57(m, 1H), 2.19 (s, 3H), 2.16-2.06 (m, 1H).

### Example 3

### 2-(((4-((2-chloro-[1,1'-biphenyl]-3-yl)amino)thiazolo[4,5-c]pyridin-2-yl)methyl)amino)acetamide

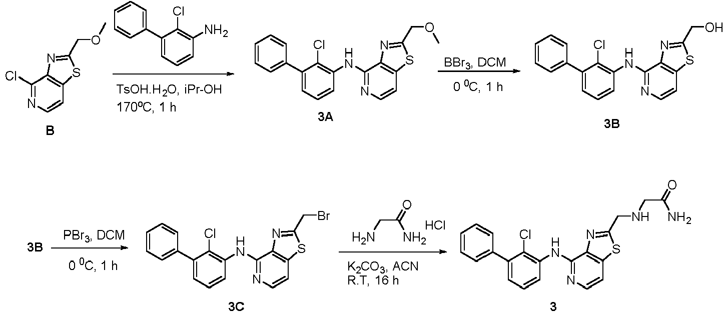

### N-(2-chloro-[1,1'-biphenyl]-3-yl)-2-(methoxymethyl)thiazolo[4,5-c]pyridin-4-amine

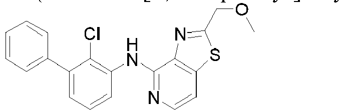

In a microwave reactor, A solution of intermediate B (500 mg, 2.33 mmol), 2-chloro-[1,1'-biphenyl]-3-amine (312 mg, 1.55 mmol) and p-toluenesulfonic acid monohydrate (444 mg, 2.33 mmol) in isopropanol (8 mL) was stirredat 170 °C for one hour under microwave; the reaction solution was diluted with ethyl acetate, washed twice with saturated aqueous sodium bicarbonate solution. The organic phase was concentrated, and the residue was separated and purified by normal phase chromatography (petroleum ether/ethyl acetate = 10/1), the title compound (300 mg, 51%) was obtained as a white solid.
MS (ESI): m/z = 382.1 [M+H]⁺.

### (4-((2-chloro-[1,1'-biphenyl]-3-yl)amino)thiazolo[4,5-c]pyridin-2-yl)methanol

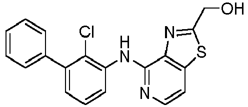

Boron tribromide (0.46 mL, 0.46 mmol) in dichloromethane was slowly added to a solution of compound 3A (87 mg, 0.23 mmol) in dichloromethane (4 mL) under ice bath. The reaction solution was stirred for one hour at 0 °C; was and then diluted with dichloromethane, washed twice with saturated aqueous sodium bicarbonate solution. The organic phase was concentrated, and the residue was purified by normal phase chromatography (petroleum ether/ethyl acetate=4/1) to obtain the title compound (45 mg, 52%) as a white solid.
MS (ESI): m/z = 368.0 [M+H]⁺.

### 2-(Bromomethyl)-N-(2-chloro-[1,r-biphenyl]-3-yl)thiazolo[4,5-c]pyridin-4-amine

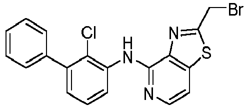

Phosphorus tribromide (65 mg, 0.24 mmol) was slowly added to a solution of compound 3B (45 mg, 0.12 mmol) in dichloromethane (4 mL) under ice bath, and then stirred for one hour at 0 °C. The solution was diluted with dichloromethane, washed twice with saturated aqueous sodium bicarbonate solution. The organic phase was concentrated and the residue was purified by normal phase chromatography (petroleum ether/ethyl acetate=8/1) to obtain the title compound (35 mg, 68%) as a white solid.
MS (ESI): m/z = 430.0 [M+H]⁺.

### 2-(((4-((2-Chloro-[1,1'-biphenyl]-3-yl)amino)thiazolo[4,5-c]pyridin-2-yl)methyl)amino) acetamide

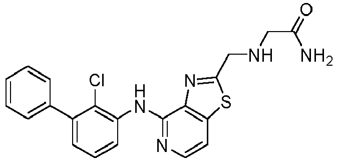

Anhydrous potassium carbonate (19 mg, 0.138 mmol) was added to the mixture of compound 3C (10 mg, 0.023 mmol), 2-aminoacetamide hydrochloric acid (8 mg, 0.069 mmol) in acetonitrile (3 mL). The reaction mixture was stirred overnight, filtered, concentrated, and the residue was purified by prep-HPLC to obtain the target product (4 mg, 41%) as a white solid.
MS (ESI): m/z = 424.1[M+H]⁺.
¹H NMR (400 MHz, CD₃OD) *δ* 8.71 (dd, *J=* 8.3, 1.5 Hz, 1H), 8.04 (d, J= 5.7 Hz, 1H), 7.45-7.39 (m, 5H), 7.39-7.34 (m, 2H), 7.01 (dd, *J=* 7.6, 1.5 Hz, 1H), 4.25 (s, 2H), 3.40 (s, 2H).

### Example 4

### (2S,4S)-1-((4-((2-chloro-[1,1'-biphenyl|-3-yl)amino)thiazolo[4,5-c[pyridin-2-yl) (methyl)-4-hydroxypyrrolidine-2-carboxylic acid

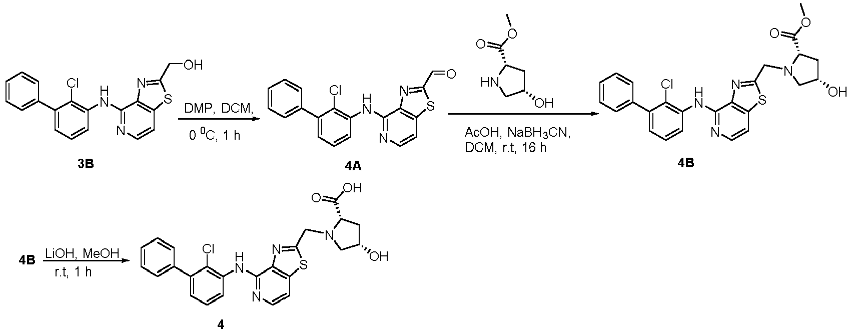

### 4-((2-Chloro-[1,1'-biphenyl]-3-yl)amino)thiazolo[4,5-c]pyridine-2-carbaldehyde

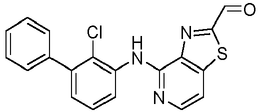

Dess-Martin oxidant (229 mg, 0.54 mmol) was added to a solution of compound 3B (100 mg, 0.27 mmol) in dichloromethane (5 mL) in portions in an ice-water bath. The reaction solution was stirred for one hour, quenched with water, extracted with dicholromethane, dried, and concentrated. The residue was purified by normal phase chromatography (petroleum ether/ethyl acetate=10/1) to obtain the title compound (70 mg, 71%) as yellow solid.
MS (ESI): m/z = 366.1 [M+H]⁺.
Methyl (2S,4S)-1-((4-((2-chloro-[1,1'-biphenyl]-3-yl)amino)thiazolo[4,5-c]pyridine-2-(yl)methyl)-4-hydroxypyrrolidine-2-carboxylate

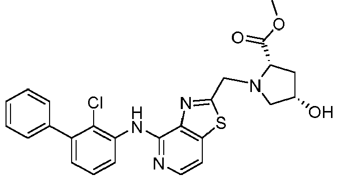

To a solution of compound 4A (40 mg, 0.11 mmol), methyl (2S, 4S)-4-hydroxypyrrolidine-2-carboxylate hydrochloric acid (40 mg, 0.22 mmol) in dichloromethane (5 mL), acetic acid (1 drop) was added. After the reaction solution was stirred at room temperature for three hours, sodium cyanoborohydride (14 mg, 0.22 mmol) was added, and the reaction solution was continued to stir overnight. The reaction solution was quenched with water extracted with dichloromethane, filtered, and concentrated. The residue was separated and purified by normal phase chromatography (petroleum ether/ethyl acetate=2/1) to obtain the title compound (15 mg, 28%) as white solid.
MS (ESI): m/z = 495.1 [M+H]⁺.

### (2S,4S)-1-((4-((2-chloro-[1,1'-biphenyl]-3-yl)amino)thiazolo[4,5-c]pyridin-2-yl) (methyl)-4-hydroxypyrrolidin-2-carboxylic acid

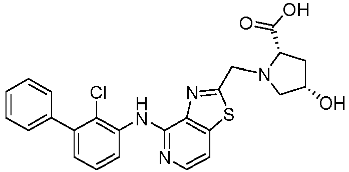

Lithium hydroxide monohydrate (3 mg, 0.060 mmol) was added to a solution of compound 4B (15 mg, 0.030 mmol) in methanol (3 mL) and water (0.5 mL). The reaction solution was stirred at room temperature for one hour, concentrated, and the residue was purified by prep-HPLC to obtain the target product (7 mg, 50%) as white solid.
MS (ESI): m/z = 481.1[M+H]⁺.
¹HNMR(400 MHz, CD₃OD) *δ* 8.68 (dd, *J=* 8.2, 1.5 Hz, 1H), 8.02 (d, *J=* 5.7 Hz, 1H), 7.45-7.31 (m, 7H), 7.00 (dd, *J=* 7.5, 1.6 Hz, 1H), 4.58 (s, 1H), 4.48 (d, *J=* 15.6 Hz, 1H), 4.29 (s, 1H), 4.18 (d, *J=* 15.3 Hz, 1H), 3.54-3.45 (m,1H), 3.21-3.19 (m, 1H), 2.91-2.84 (m, 1H), 2.55-2.46 (m,1H), 1.99-1.90 (m,1H).

### Example 5

### 2-(((4-((2-Chloro-[1,1'-biphenyl]-3-yl)amino)thiazolo[4,5-c]pyridin-2-yl)methyl)amino)ethane-1-ol

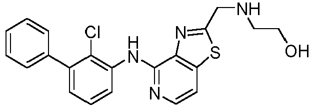

The target compound was prepared by using compound 4A and 2-aminoethane-1-ol under conditions similar to Example 4.
MS (ESI): m/z = 411.1[M+H]⁺.
¹H NMR (400 MHz, MeOD-*d*₄) *δ* 8.72 (dd, *J* = 8.3, 1.5 Hz, 1H), 8.04 (d, *J* = 5.7 Hz, 1H), 7.48-7.32 (m, 7H), 7.01 (dd, *J=* 7.6, 1.5 Hz, 1H), 4.27 (s, 2H), 3.69 (t, *J=* 5.5 Hz, 2H), 2.86 (t, *J* = 5.5 Hz, 2H).

### Example 6

### (R)-1-((4-((2-Chloro-[1,1'-biphenyl]-3-yl)amino)thiazolo[4,5-c[pyridin-2-yl)methyl)pyrrolidin-3-ol

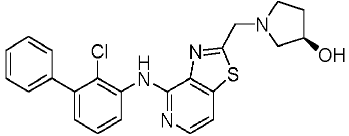

The target compound was prepared by using compound 3C and (R)-pyrrolidin-3-ol under conditions similar to Example 3.
MS (ESI): m/z = 437.1[M+H]+.
¹H NMR (400 MHz, CD₃OD) *δ* 8.72 (dd, *J=* 8.3, 1.6 Hz, 1H), 8.03 (d, *J=* 5.7 Hz, 1H), 7.44-7.32 (m, 7H), 7.00 (dd, *J* = 7.6, 1.6 Hz, 1H), 4.58 (s, 1H), 4.40-4.34 (m, 1H), 4.13 (d, *J* = 3.2 Hz, 2H), 3.01-2.89 (m, 2H), 2.75-2.67 (m, 2H), 2.20-2.10 (m, 1H), 1.80-1.72 (m, 1H).

### Example 7

### (S)-1-((4-((2-Chloro-[1,1'-biphenyl]-3-yl)amino)thiazolo[4,5-c]pyridin-2-yl)methyl)pyrrolidin-3-ol

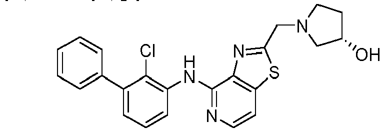

The target compound was prepared by using compound 3C and (S)-pyrrolidin-3-ol under conditions similar to Example 3.
MS (ESI): m/z = 437.1[M+H]+.
¹H NMR (400 MHz, CD₃OD) *δ* 8.72 (dd, *J=* 8.3, 1.6 Hz, 1H), 8.04 (d, J= 5.7 Hz, 1H), 7.44-7.33 (m, 7H), 7.01 (dd, *J=* 7.6, 1.6 Hz, 1H), 4.58 (s, 1H), 4.41-4.35 (m, 1H), 4.14 (d, *J* = 3.2 Hz, 2H), 3.00-2.89 (m, 2H), 2.76-2.67 (m, 2H), 2.22-2.12 (m, 1H), 1.81-1.72 (m, 1H).

### Example 8

### ((4-((2-Chloro-[1,1'-biphenyl]-3-yl)amino)thiazolo[4,5-c]pyridin-2-yl)methyl)glycine

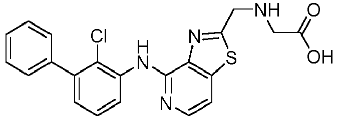

The target compound was prepared from compound 3C and glycine under conditions similar to Example 3.
MS (ESI): m/z = 425.1 [M+H]⁺.
¹H NMR (400 MHz, DMSO-d6) δ 8.77 (d, J = 8.8 Hz, 1H), 8.67 (s, 1H), 8.06 (d, J = 5.4 Hz, 1H), 7.53 (d, J = 5.4 Hz , 1H), 7.48-7.38 (m, 6H), 7.02 (dd, J = 7.7, 1.5 Hz, 1H), 4.17 (s, 2H), 3.09 (s, 2H).

### Example 9

### 2-(((4-((2-chloro-3'-(3-chloropropoxy)-2'-methyl-[1,1'-biphenyl]-3-yl)amino)thiazolo [4,5-c]pyridin-2-yl)methyl)amino)ethane-1-ol

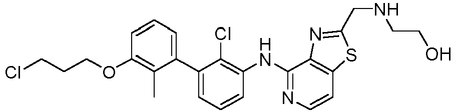

The target compound was obtained according to the method in Example 3by replacing the corresponding starting materials.
MS (ESI): m/z = 517.1 [M+H]⁺.
¹H NMR (400 MHz, CD₃OD)δ 8.75 (d, J = 6.9 Hz, 1H), 8.05 (d, J = 5.7 Hz, 1H), 7.41 (d, J = 5.7 Hz, 1H), 7.38-7.31 (m, 1H), 7.19 (t, J = 7.9 Hz, 1H), 6.95 (d, J = 7.7 Hz, 1H), 6.88 (dd, J = 7.5, 1.5 Hz, 1H), 6.74 (d, J = 7.6 Hz , 1H), 4.25 (s, 2H), 4.17 (m, 2H), 3.79 (t, J = 6.4 Hz, 2H), 3.68 (t, J = 5.5 Hz, 2H), 2.84 (t, J = 5.5 Hz , 2H), 2.30-2.22 (m, 2H), 1.96 (s, 3H).

### Example 10

### 2-(((4-((2-chloro-3-(2,3-dihydrobenzo[b][1,4]dioxin-6-yl)phenyl)amino)thiazolo[4, 5-c)pyridin-2-yl)methyl)amino)ethane-1-ol

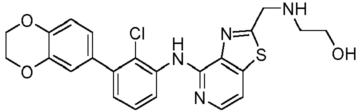

The target compound was obtained according to the method in Example 3 by replacing the corresponding starting materials.
MS (ESI): m/z = 469.1 [M+H]⁺.
¹HNMR(400 MHz, CD₃OD) δ 8.65 (dd, J = 8.3, 1.5 Hz, 1H), 8.04 (d, J = 5.7 Hz, 1H), 7.40 (d, J = 5.7 Hz, 1H), 7.35-7.29 (m, 1H), 6.99 (dd, J = 7.6, 1.6 Hz, 1H), 6.90-6.85 (m, 3H), 4.35 (s, 2H), 4.27 (s, 4H), 3.75-3.69 (m, 2H)), 2.97-2.91 (m, 2H).

### Example 11

### 2-(((4-((2-chloro-3'-(2-morpholinoethoxy)-[1,1'-biphenyl]-3-yl)amino)thiazolo[4,5-c)pyridin-2-yl)methyl)amino)ethane-1-ol

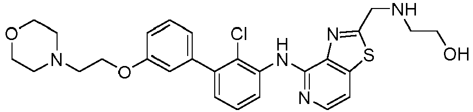

The target compound was obtained according to the method in Example 3 by replacing the corresponding starting materials.
MS (ESI): m/z = 540.1[M+H]⁺.
¹H NMR (400 MHz, CD₃OD) δ 8.70 (dd, J= 8.3, 1.5 Hz, 1H), 8.01 (d, J= 5.7 Hz, 1H), 7.36 (d, *J=* 5.7 Hz, 1H), 7.32 (m, 2H), 7.00-6.93 (m, 4H), 4.23 (s, 2H), 4.15 (t, *J=* 5.5 Hz, 2H), 3.72-3.65 (m, 6H), 2.84 (t, *J=* 5.5 Hz, 2H), 2.79 (t, *J=* 5.5 Hz, 2H), 2.61-2.54 (m, 4H).

### Example 12

### 1-(4-((2'-chloro-3'-((2-(((2-hydroxyethyl)amino)methyl)thiazolo[4,5-c]pyridin-4-yl)amino)-[1,1'-biphenyl]-3-yl)oxo)butyl)pyrrolidin-3-ol

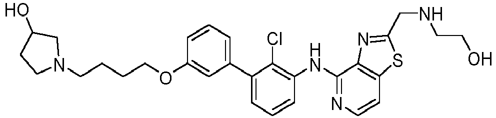

The target compound was obtained according to the method in example 3 by replacing the corresponding starting materials.
MS (ESI): m/z = 568.6[M+H]⁺.
¹H NMR (400 MHz, CD₃OD) δ 8.70 (dd, *J=* 8.3, 1.6 Hz, 1H), 8.03 (d, *J*= 5.7 Hz, 1H), 7.40 (d, *J=* 5.7 Hz, 1H), 7.38-7.29 (m, 2H), 7.03-6.90 (m, 4H), 4.34 (ddd, *J=* 9.5, 6.3, 3.2 Hz, 1H), 4.25 (s, 2H), 4.03 (t, *J=* 6.1 Hz, 2H), 3.68 (t, *J=* 5.4 Hz, 2H), 2.93-2.73 (m, 4H), 2.70-2.50 (m, 4H), 2.17-2.06 (m, 1H), 1.86-1.68 (m, 5H).

### Example 13

### methyl ((4-((2-chloro-[1,1'-biphenyl]-3-yl)amino)thiazolo[4,5-c]pyridin-2-yl)methyl)glycine

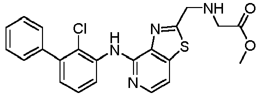

The target compound was obtained according to the method in Example 3 by replacing the corresponding starting materials.
MS (ESI): m/z = 438.9[M+H]⁺.
¹H NMR (400 MHz, CD₃OD) δ 8.72 (dd, *J=* 8.3, 1.6 Hz, 1H), 8.04 (d, J= 5.7 Hz, 1H), 7.46-7.33 (m, 7H), 7.01 (dd, *J=* 7.6, 1.6 Hz, 1H), 4.27 (s, 2H), 3.71 (s, 3H), 3.56 (s, 2H).

### Example 14

### ((4-((2-chloro-3'-(3-((3-hydroxypropyl)(methyl)amino)propoxy)-[1,1'-biphenyl]-3-yl)amino)thiazolo[4,5-c]pyridin-2-yl)methyl)glycine

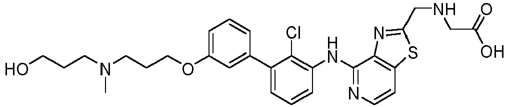

The target compound was obtained according to the method in Example 3 by replacing the corresponding starting materials.
MS (ESI): m/z = 570.5[M+H]⁺.
¹H NMR (400 MHz, CD₃OD) δ 8.70 (d, *J=* 7.0 Hz, 1H), 8.04 (d, *J=* 5.7 Hz, 1H), 7.40 (d, *J=* 5.6 Hz, 1H), 7.34 (m, 2H), 7.04-6.94 (m, 4H), 4.38 (s, 2H), 4.15 (t, *J=* 5.7 Hz, 2H), 3.67 (t, *J=* 5.7 Hz, 2H), 3.44 (s, 2H), 3.29-3.23 (m, 2H), 3.18 (t, *J=* 7.4 Hz, 2H), 2.82 (s, 3H), 2.27-2.15 (m, 2H), 1.95-1.85 (m, 2H).

### Example 15

### ((4-((2-chloro-3'-(3-(3-hydroxypyrrolidine-1-yl)propoxy)-[1,1'-biphenyl]-3-yl)amino)thiazolo[4,5-c]pyridine-2-yl)methyl)glycine

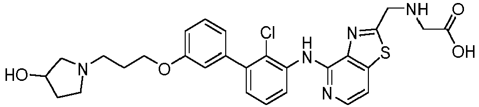

The target compound was obtained according to the method in Example 3 by replacing the corresponding starting materials.
MS (ESI): m/z = 568.5 [M+H]⁺.
¹H NMR (400 MHz, CD₃OD-d4) δ 8.71 (d, J = 7.0 Hz, 1H), 8.06 (d, J = 5.7 Hz, 1H), 7.41 (d, J = 5.7 Hz, 1H), 7.35 (td, J = 8.1, 1.5 Hz, 2H), 7.04 - 6.94 (m, 4H), 4.53 (s, 1H), 4.43 (s, 2H), 4.15 (t, J = 5.7 Hz, 2H), 3.64 - 3.53 (m, 2H), 3.49 (s, 2H), 3.42 - 3.32 (m, 4H), 2.26 - 2.18 (m, 3H), 2.07-1.95 (m, 1H).

### Example 16

### ((4-((2-chloro-3'-(3-(3-hydroxypyrrolidin-1-yl)propoxy)-2'-methyl-[1,1'-biphenyl]-3 -yl)amino)thiazolo[4,5-c]pyridin-2-yl)methyl)glycine

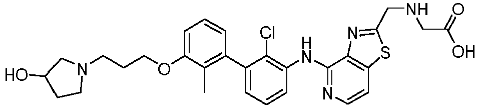

The target compound was obtained according to the method in Example 3 by replacing the corresponding starting materials.
MS (ESI): m/z = 582.5[M+H]⁺.
¹H NMR (400 MHz, CD₃OD)δ 8.74 (dd, *J* = 8.3, 1.5 Hz, 1H), 8.07 (d*, J =* 5.7 Hz, 1H), 7.42 (d, *J=* 5.7 Hz, 1H), 7.35 (m, 1H), 7.19 (m, 1H), 6.95 (d, *J=* 8.3 Hz, 1H), 6.87 (dd, *J=* 7.5, 1.5 Hz, 1H), 6.76 (d, *J=* 7.0 Hz, 1H), 4.54-4.47 (m, 1H), 4.37 (s, 2H), 4.18-4.11 (m, 2H), 3.57-3.44 (m, 2H), 3.42 (s, 2H), 3.40-3.27 (m, 4H), 2.27-2.17 (m, 3H), 2.14-1.94 (m, 4H).

### Example 17

### 2-(((4-((2-chloro-[1,1'-biphenyl]-3-yl)amino)thiazolo[5,4-c]pyridin-2-yl)methyl)amino)ethane-1-ol

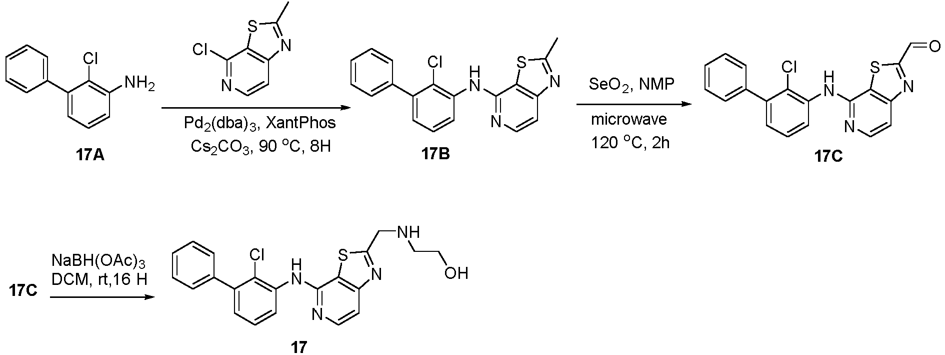

### N-(2-Chloro-[1,1' -biphenyl] -3 -yl)-2-methylthioazolo[5,4-c]pyridin-4-amine

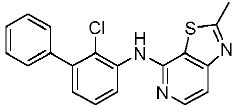

Tris(dibenzylideneacetone)dipalladium (36 mg, 0.04 mmol), 4,5-bisdiphenylphosphine-9,9-dimethyloxa anthracene (46 mg, 0.08 mmol) and cesium carbonate (267 mg, 0.82 mmol) were added to a mixture of 4-chloro-2-methylthio azolo[5,4-c]pyridine (75 mg, 0.41 mmol) and 2-chloro-[1,1'-biphenyl]-3-amine (116 mg, 0.57 mmol) in toluene (4 mL). The reaction mixture was heated to 90 °C and stirred for 8 hours under nitrogen. After cooling, the reaction solution was filtered with kieselgur and eluted with dichloromethane. The filtrate was concentrated, and the residue was separated and purified by normal phase chromatography (petroleum ether/ethyl acetate=9/1) to obtain the target compound 17B (120 mg, 83%) as pale yellow solid.
MS (ESI): m/z = 351.9 [M+H]⁺.

### 4-((2-Chloro-[1,l'-biphenyl]-3-yl)amino)thiazolo[5,4-c]pyridine-2-carbaldehyde

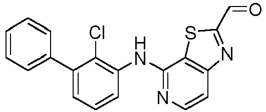

Tin dioxide (61 mg, 0.55 mmol) was added to a solution of compound 17B (40 mg, 0.11 mmol) in 1-methyl-2-pyrrolidone (4 mL) at room temperature, and the mixture was heated to 120 °C under microwave and stirred for two hours. After cooling, the crude product was purified by prep-HPLC to obtain the title compound 17C (20 mg crude product, 35%) as a yellow solid.
MS (ESI): m/z = 383.9 [M+18+H]⁺.

### 2-(((4-((2-Chloro-[1,1'-biphenyl]-3-yl)amino)thiazolo[5,4-c]pyridin-2-yl)methyl)amino) ethane-1-ol

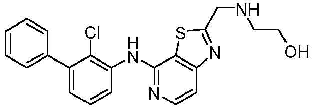

A catalytic amount of acetic acid (1 drop) was added to the solution of Example 17C (20 mg, 0.035 mmol), 2-aminoethane-1-ol (8 mg, 0.14 mmol) in dichloromethane. After the reaction solution was stirred at room temperature for 16 hours, sodium triacetoxyborohydride (63 mg, 0.30 mmol) was added. The reaction solution was stirred at room temperature for 4 hours, then quenched with water (10 mL), extracted with dichloromethane for three times (15 mL*3). The combined organic phase was dried and concentrated. The residue was purified by prep-HPLC to obtain the target compound (4 mg, 25%) as a yellow solid.
MS (ESI): m/z = 410.9 [M+H]⁺.
¹H NMR (400 MHz, MeOD-*d*₄) δ 8.08 (d, *J=* 5.8 Hz, 1H), 7.65 (dd, *J* = 8.1, 1.6 Hz, 1H), 7.45-7.33 (m, 7H), 7.21 (dd, *J=* 7.7, 1.6 Hz, 1H), 4.33 (s, 2H), 3.71 (t, J = 5.4 Hz, 2H), 2.92 (t, J = 5.4 Hz, 2H).

### Example 18:

### (S)-((4-((2,2'-dichloro-3'-(6-methoxy-5-((((5-carbonylpyrrolidin-2-yl)methyl)amino)methyl)pyridin-2-yl)-[1,1'-biphenyl]-3-yl)amino)thiazolo[4,5-c]pyridin-2-yl)methyl)glycine

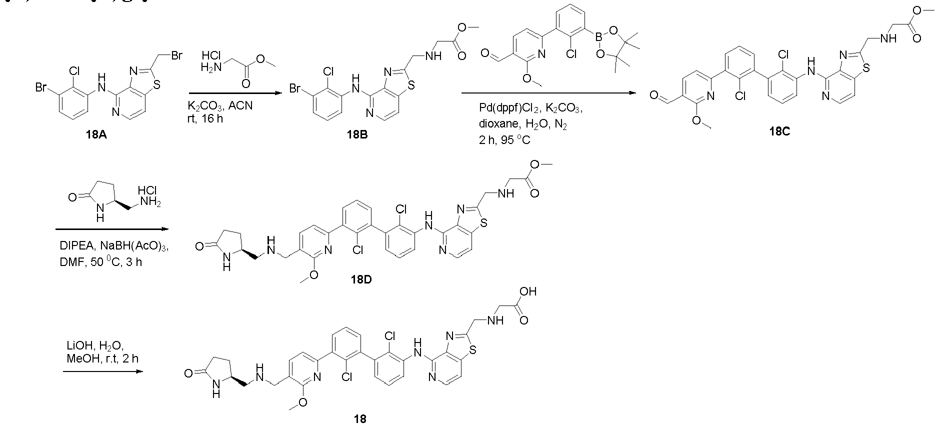

### Methyl((4-((3-bromo-2-chlorophenyl)amino)thiazolo[4,5-c]pyridin-2-yl)methyl)glycinate

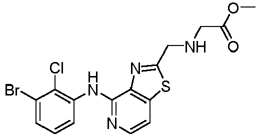

Anhydrous potassium carbonate (191 mg, 1.38 mmol) was added to the solution (3 mL) of compound 18A (100 mg, 0.23 mmol), glycine methyl ester hydrochloride (58 mg, 0.46 mmol) in acetonitrile. The reaction solution was stirred overnight at room temperature. After the reaction completed, the reaction mixture was extracted and, concentrated, and the crude product was separated and purified by flash normal phase chromatography (ethyl acetate: petroleum ether=7: 1) to obtain the target product 18B (80 mg, 79%) as a pale yellow solid.
MS (ESI): m/z = 440.7[M+H]⁺.

### methyl ((4-((2,2'-dichloro-3'-(5-formyl-6-methoxypyridin-2-yl)-[1,1'-biphenyl]-3-yl)amino)thiazolo[4,5-c]pyridin-2-yl)methyl)glycinate

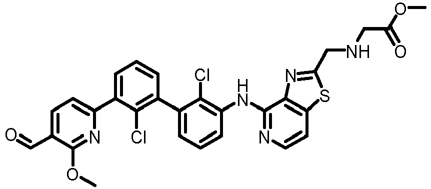

[1,1'-Bis(diphenylphosphino)ferrocene]dichloropalladium(II) (15 mg, 0.018 mmol) was added to a mixture of 18B (80 mg, 0.18 mmol), 6-(2-chloro-3-(4,4,5,5-tetramethyl-1,3,2-dioxaborolan-2-yl)phenyl)-2-methoxynicotinaldehyde (101 mg, 0.27 mmol), potassium carbonate (75 mg, 0.54 mmol)in dioxane (3 mL) and water (0.5 mL). The mixture was heated to 95 °C for 2 hours under nitrogen atmosphere, filtered and concentrated. The residue was purified by a normal phase silica gel column (ethyl acetate: petroleum ether=8:1) to obtain the target compound 18C (100 mg, 89%) as light yellow solid.
MS (ESI): m/z = 607.9[M+H]⁺.Methyl((4-((2,2'-dichloro-3'-(5-formyl-6-methoxypyridin-2-yl)-[1,1'-biphenyl]-3- (yl)amino)thiazolo[4,5-c]pyridin-2-yl)methyl)glycinate

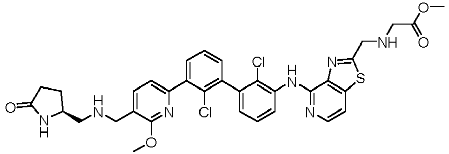

N,N-diisopropylethylamine (104 mg, 0.80 mmol) was added to compound 18C (100 mg, 0.16 mmol), (S)-5-(aminomethyl)pyrrolidin-2-one hydrochloric acid (121 mg, 0.80 mmol) in N,N-dimethylformamide (3 mL) . The reaction solution was heated to 50°C and stirred for two hours. Sodium triacetoxyborohydride (170 mg, 0.80 mmol) was added in portions, and continued to stir for one hour. The reaction mixture was quenched with methanol (0.5 mL), concentrated and the residue was purified by flash reverse phase chromatography (80% acetonitrile-10 nmol/mL aqueous ammonium bicarbonate solution) to obtain the target product 18D (70 mg, 63%) as a light yellow solid.
MS (ESI): m/z = 706.0[M+H]⁺.

### (S)-((4-((2,2'-Dichloro-3'-(6-methoxy-5-((((5-carbonylpyrrolidin-2-yl)methyl)amino)methyl)pyridin-2-yl)-[1,1'-biphenyl]-3-yl)amino)thiazolo[4,5-c]pyridin-2-yl)methyl)glycine

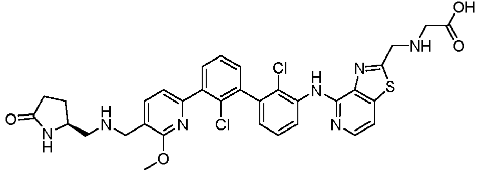

To a mixture of compound 18D (70 mg, 0.10 mmol) in methanol (3 mL) and water (0.5 mL), lithium hydroxide monohydrate (17 mg, 0.40 mmol) was added. The reaction solution was stirred at room temperature for two hours. After the reaction was completed, the reaction solution was separated and purified by flash reverse phase chromatography (35% acetonitrile-0.1% formic acid aqueous solution elution) to obtain the target product example 18 (30 mg, 43%) as a pale yellow solid.
MS (ESI): m/z = 692.0[M+H]⁺.
NMR: ¹H NMR (400 MHz, DMSO-*d*₆) δ 8.84 (d, J = 8.7 Hz, 1H), 8.63 (s, 1H), 8.08 (d, J = 5.6 Hz, 1H), 7.79 (d, J = 7.4 Hz, 1H), 7.68 (s, 1H), 7.64 (dd, J = 7.8, 1.4 Hz, 1H), 7.55 (d, J = 5.6 Hz, 1H), 7.51 (m, 1H), 7.44 (m, 1H), 7.39 (dd, J = 7.7, 1.7 Hz, 1H), 7.25 (d, J = 7.7 Hz, 1H), 7.02 (d, J = 7.8 Hz, 1H), 4.18 (s, 2H), 3.89 (s, 3H), 3.68 (s, 2H), 3.60 (m, 1H), 3.22 (s, 2H), 2.52 (d, J = 5.8 Hz, 2H), 2.10-2.00 (m, 3H), 1.72-1.60 (m, 1H).

### Example 19:

### (5-methyl-2-carbonyl-1,3-dioxazole-4-yl) methyl (S) - ((4 - ((2,2 '- dichloro-3' - (6-methoxy-5 - ((5-carbonylpyrrolidin-2-yl) methyl) pyridine-2-yl) - [1,1 '- biphenyl] - 3-yl) amino) thiazolo [4,5-c] pyridine-2-yl) methyl) glycine

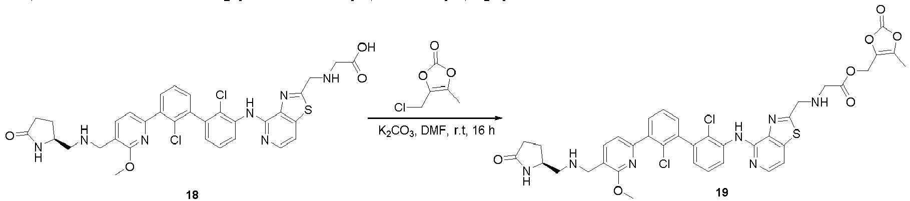

### (5-methyl-2-carbonyl-1,3-dioxazole-4-yl) methyl (S) - ((4 - ((2,2'- dichloro-3' - (6-methoxy-5 - ((5-carbonylpyrrolidin-2-yl) methyl) pyridine-2-yl) - [1,1'- biphenyl] - 3-yl) amino) thiazolo [4,5-c] pyridine-2-yl) methyl) glycine

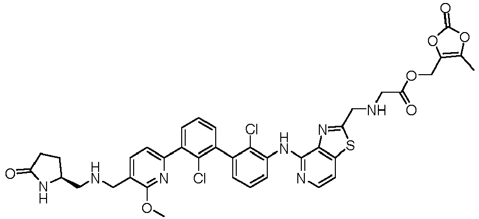

To the solution of Example 18 (80 mg, 0.12 mmol) and anhydrous potassium carbonate (50 mg, 0.36 mmol) in N,N-dimethylformamide (3 mL), 4-(chloromethyl)- 5-methyl-1,3-dioxazol-2-one (36 mg, 0.24 mmol)was added, and the reaction solution was stirred overnight at room temperature. After the reaction was completed, the reaction solution was separated and purified by flash reverse phase chromatography (eluted with 65% acetonitrile-0.1% formic acid aqueous solution) to obtain the target product Example 19 (18 mg, 18%) as a white solid.
MS (ESI): m/z = 804.2[M+H]⁺.
¹H NMR (400 MHz, DMSO-*d*₆) δ 8.84 (dd, J = 8.3, 1.4 Hz, 1H), 8.62 (s, 1H), 8.09 (d, J = 5.6 Hz, 1H), 7.79 (d, J = 7.6 Hz, 1H), 7.67 (s, 1H), 7.64 (dd, J = 7.7, 1.7 Hz, 1H), 7.56 (d, J = 5.6 Hz, 1H), 7.51 (m, 1H), 7.45 (m, 1H), 7.39 (dd, J = 7.5, 1.7 Hz, 1H), 7.25 (d, J = 7.6 Hz, 1H), 7.02 (dd, J = 7.5, 1.4 Hz, 1H), 4.97 (s, 2H), 4.21 (d, J = 5.6 Hz, 2H), 3.89 (s, 3H), 3.68 (s, 2H), 3.65-3.58 (m, 1H), 3.55 (d, J = 6.6 Hz, 2H), 3.51-3.41 (m, 1H), 2.52 (d, J = 5.9 Hz, 2H), 2.11 (s, 3H), 2.10-2.01 (m, 3H), 1.72-1.60 (m, 1H).

The following compounds were obtained using methods similar to those in Examples 18-19 by replacing the corresponding starting materials.

| Number | Compound structure | LCMS, HNMR |
|---|---|---|
| 20 | 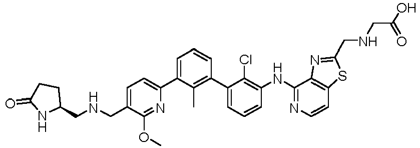 | MS (ESI): m/z = 672.0[M+H]⁺.¹H NMR (400 MHz, DMSO-*d*₆) δ 8.81 (d, J = 7.0 Hz, 1H), 8.65 (s, 1H), 8.07 (d, J = 5.6 Hz, 1H), 7.78 (d, J = 7.5 Hz, 1H), 7.68 (s, 1H), 7.55 (d, J = 5.6 Hz, 1H), 7.46 -7.39 (m, 2H), 7.34 (m, 1H), 7.19 (d, J = 6.6 Hz, 1H), 7.11 (d, J = 7.6 Hz, 1H), 6.97 (dd, J = 7.5, 1.4 Hz, 1H), 4.18 (s, 2H), 3.86 (s, 3H), 3.67 (s, 2H), 3.62-3.58 (m, 1H), 3.24 (s, 2H), 2.52 (d, J = 6.0 Hz, 2H), 2.15-1.99 (m, 6H), 1.70-1.63 (m, 1H). |
| 21 | 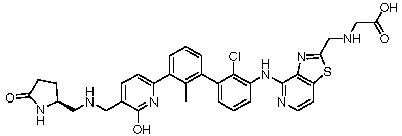 | MS (ESI): m/z = 658.0[M+H]⁺.¹H NMR (400 MHz, DMSO-*d*₆) δ 8.79 (d, J = 7.4 Hz, 1H), 8.65 (s, 1H), 8.07 (d, J = 5.7 Hz, 1H), 7.69 (s, 1H), 7.54 (d, J = 5.9 Hz, 1H), 7.49 - 7.39 (m, 2H), 7.37 - 7.28 (m, 2H), 7.23 (dd, J = 7.2, 1.8 Hz, 1H), 6.92 (dd, J = 7.5, 1.5 Hz, 1H), 6.16 (d, J = 6.9 Hz, 1H), 4.17 (s, 2H), 3.61 - 3.57 (m, 1H), 3.50 (s, 2H), 3.15 (s, 2H), 2.50 (d, J = 6.0 Hz, 2H), 2.14 - 2.02 (m, 3H), 1.97 (s, 3H), 1.72 - 1.61 (m, 1H). |
| 22 | 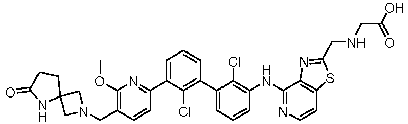 | MS (ESI): m/z = 704.0[M+H]⁺.¹H NMR (400 MHz, DMSO-*d*₆) δ 8.84 (dd, J = 8.3, 1.4 Hz, 1H), 8.62 (s, 1H), 8.12 (s, 1H), 8.08 (d, J = 5.6 Hz, 1H), 7.68 (d, J = 7.6 Hz, 1H), 7.63 (dd, J = 7.7, 1.7 Hz, 1H), 7.56 (d, J = 5.6 Hz, 1H), 7.51 (t, J = 7.6 Hz, 1H), 7.44 (t, J = 7.9 Hz, 1H), 7.39 (dd, J = 7.5, 1.7 Hz, 1H), 7.24 (d, J = 7.5 Hz, 1H), 7.02 (dd, J = 7.5, 1.5 Hz, 1H), 4.19 (s, 2H), 3.88 (s, 3H), 3.54 (s, 2H), 3.41 (d, J = 7.7 Hz, 2H), 3.27 (s, 2H), 3.09 (d, J = 7.5 Hz, 2H), 2.27 -2.20 (m, 2H), 2.16 - 2.08 (m, 2H). |
| 23 | 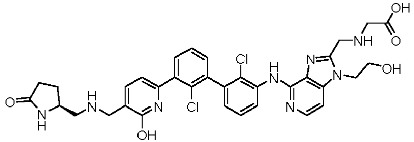 | MS (ESI): m/z = 705.1[M+H]⁺.¹H NMR (400 MHz, CD₃OD-*d*₄) δ 8.64 (d, J = 9.9 Hz, 1H), 7.97 (d, J = 6.0 Hz, 1H), 7.73 (d, J = 7.5 Hz, 1H), 7.59 - 7.45 (m, 3H), 7.45 - 7.34 (m, 1H), 7.12 (d, J = 5.3 Hz, 1H), 6.98 (d, J = 8.1 Hz, 1H), 6.48 (d, J = 7.0 Hz, 1H), 4.68 - 4.54 (m, 2H), 4.43 (s, 2H), 4.04 - 3.90 (m, 3H), 3.87 (s, 2H), 3.65 (s, 2H), 3.05 - 2.91 (m, 2H), 2.43 - 2.27 (m, 3H), 1.90- 1.81 (m, 1H). |
| 24 | 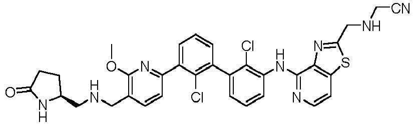 | MS (ESI): m/z = 673.1 [M+H]⁺.¹H NMR (400 MHz, DMSO-*d*₆) δ 8.81 (d, J = 8.3 Hz, 1H), 8.66 (s, 1H), 8.09 (d, J = 5.6 Hz, 1H), 7.79 (d, J = 7.3 Hz, 1H), 7.68 (s, 1H), 7.64 (dd, J = 7.6, 1.5 Hz, 1H), 7.57 (d, J = 5.6 Hz, 1H), 7.51 (t, J = 7.6 Hz, 1H), 7.45 (t, J = 7.9 Hz, 1H), 7.39 (dd, J = 7.4, 1.3 Hz, 1H), 7.25 (d, J = 7.5 Hz, 1H), 7.03 (d, J = 7.5 Hz, 1H), 4.22 (d, J = 5.5 Hz, 2H), 3.87 (d, J = 10.5 Hz, 3H), 3.78 (d, J = 6.4 Hz, 2H), 3.70 (s, 2H), 3.65 - 3.55 (m, 1H), 2.51 (d, J = 5.9 Hz, 2H), 2.11 -2.01 (m, 3H), 1.73 - 1.60 (m, 1H). |
| 25 | 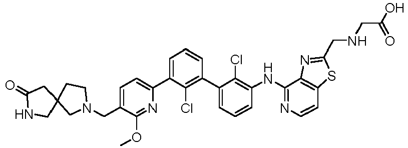 | MS (ESI): m/z = 718.1[M+H]⁺.¹H NMR (400 MHz, DMSO-*d*₆) δ 8.84 (dd, J = 8.2, 1.4 Hz, 1H), 8.62 (s, 1H), 8.08 (d, J = 5.7 Hz, 1H), 7.75 (d, J = 7.6 Hz, 1H), 7.65 (dd, J = 7.7, 1.7 Hz, 1H), 7.58 - 7.47 (m, 3H), 7.44 (t, J = 8.0 Hz, 1H), 7.39 (dd, J = 7.6, 1.6 Hz, 1H), 7.26 (d, J = 7.5 Hz, 1H), 7.02 (dd, J = 7.6, 1.5 Hz, 1H), 4.18 (s, 2H), 3.88 (s, 3H), 3.58 (s, 2H), 3.28 (s, 2H), 3.14 (q, J = 24.6, 9.5 Hz, 3H), 2.70 - 2.61 (m, 1H), 2.61 - 2.51 (m, 2H), 2.25 - 2.09 (m, 2H), 1.85 - 1.73 (m, 2H). |
| 26 | 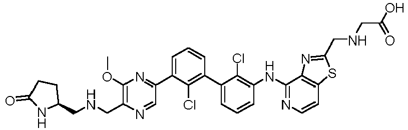 | MS (ESI): m/z = 693.1[M+H]⁺.¹H NMR (400 MHz, DMSO-*d*₆) δ 8.84 (d, J = 7.8 Hz, 1H), 8.65 (s, 1H), 8.35 (s, 1H), 8.08 (d, J = 5.6 Hz, 1H), 7.60 - 7.54 (m, 3H), 7.52 - 7.42 (m, 2H), 7.05 (d, J = 7.9 Hz, 1H), 4.20 (s, 2H), 3.87 (s, 3H), 3.68 - 3.57 (m, 2H), 3.46 - 3.41 (m, 1H), 3.37 (s, 2H), 2.43 - 2.27 (m, 2H), 2.06 - 1.88 (m, 3H), 1.60 - 1.48 (m, 1H). |
| 27 | 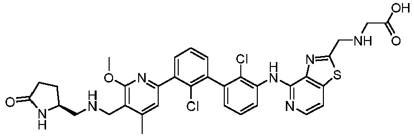 | MS (ESI): m/z = 706.1[M+H]⁺.¹H NMR (400 MHz, DMSO-*d*₆) δ 8.84 (dd, J = 8.3, 1.2 Hz, 1H), 8.62 (s, 1H), 8.24 (s, 1H), 8.08 (d, J = 5.7 Hz, 1H), 7.66 (s, 1H), 7.60 (dd, J = 7.7, 1.7 Hz, 1H), 7.55 (d, J = 5.6 Hz, 1H), 7.50 (t, J = 7.6 Hz, 1H), 7.44 (t, J = 7.9 Hz, 1H), 7.38 (dd, J = 7.5, 1.7 Hz, 1H), 7.09 (s, 1H), 7.01 (dd, J = 7.6, 1.4 Hz, 1H), 4.19 (s, 2H), 3.86 (s, 3H), 3.70 (s, 2H), 3.62 - 3.53 (m, 1H), 3.30 (s, 2H), 2.52 (d, J = 5.0 Hz, 2H), 2.36 (s, 3H), 2.13 - 1.98 (m, 3H), 1.71 - 1.57 (m, 1H). |
| 28 | 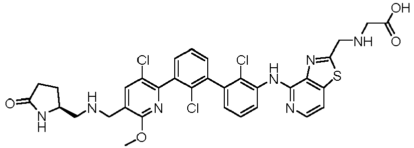 | MS (ESI): m/z = 728.0[M+H]⁺.¹H NMR (400 MHz, DMSO-*d*₆) δ 8.84 (d, J = 8.2 Hz, 1H), 8.63 (s, 1H), 8.24 (s, 1H), 8.07 (d, J = 5.2 Hz, 1H), 7.90 (s, 1H), 7.71 (s, 1H), 7.58 - 7.49 (m, 2H), 7.49 - 7.40 (m, 3H), 7.02 (d, J = 6.9 Hz, 1H), 4.19 (s, 2H), 3.83 (s, 3H), 3.69 (d, J = 10.0 Hz, 2H), 3.65 - 3.58 (m, 1H), 3.29 (s, 2H), 2.53 (d, J = 6.0 Hz, 2H), 2.15 - 1.99 (m, 3H), 1.74 - 1.58 (m, 1H). |
| 29 | 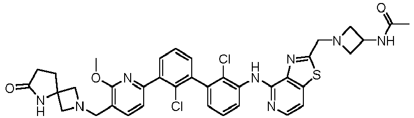 | MS (ESI): m/z = 743.8 [M+H]⁺.¹H NMR (400 MHz, DMSO-*d*₆) δ 8.83 (d, J = 8.1 Hz, 1H), 8.63 (s, 1H), 8.33 (d, J = 7.5 Hz, 1H), 8.10 (s, 1H), 8.09 (s, 1H), 7.68 (d, J = 7.8 Hz, 1H), 7.64 (dd, J = 7.7, 1.6 Hz, 1H), 7.57 (d, J = 5.7 Hz, 1H), 7.51 (t, J = 7.6 Hz, 1H), 7.45 (t, J = 7.9 Hz, 1H), 7.39 (dd, J = 7.6, 1.6 Hz, 1H), 7.24 (d, J = 7.4 Hz, 1H), 7.03 (dd, J = 7.5, 1.4 Hz, 1H), 4.32 (q, J = 14.0, 7.2 Hz, 1H), 4.08 (s, 2H), 3.88 (s, 3H), 3.67 (t, J = 7.4 Hz, 2H), 3.54 (s, 2H), 3.41 (d, J = 7.4 Hz, 2H), 3.14 - 3.04 (m, 4H), 2.27 - 2.19 (m, 2H), 2.16 - 2.09 (m, 2H), 1.76 (s, 3H). |
| 30 | 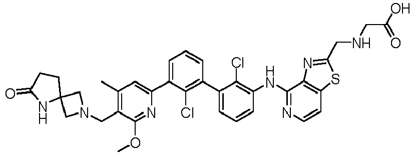 | MS (ESI): m/z = 718.1 [M+H]⁺.¹H NMR (400 MHz, DMSO-*d*₆) δ 8.84 (dd, J = 8.4, 1.2 Hz, 1H), 8.63 (s, 1H), 8.08 (d, J = 5.5 Hz, 2H), 7.61 (dd, J = 7.6, 1.7 Hz, 1H), 7.56 (d, J = 5.6 Hz, 1H), 7.50 (t, J = 7.6 Hz, 1H), 7.44 (t, J = 7.9 Hz, 1H), 7.38 (dd, J = 7.4, 1.6 Hz, 1H), 7.09 (s, 1H), 7.01 (dd, J = 7.6, 1.4 Hz, 1H), 4.19 (s, 2H), 3.85 (s, 3H), 3.58 (s, 2H), 3.35 (s, 2H), 3.26 (d, J = 7.6 Hz, 2H), 3.15 (d, J = 7.4 Hz, 2H), 2.35 (s, 3H), 2.18 - 2.12 (m, 2H), 2.11 - 2.05 (m, 2H). |
| 31 | 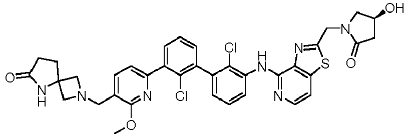 | MS (ESI): m/z = 730.6 [M+H]⁺.¹H NMR (400 MHz, DMSO-*d*₆) δ 8.80 (dd, J = 8.4, 1.4 Hz, 1H), 8.68 (s, 1H), 8.12 (s, 1H), 8.10 (s, 1H), 7.68 (d, J = 7.4 Hz, 1H), 7.64 (dd, J = 7.7, 1.7 Hz, 1H), 7.59 (d, J = 5.7 Hz, 1H), 7.51 (t, J = 7.6 Hz, 1H), 7.45 (t, J = 7.9 Hz, 1H), 7.39 (dd, J = 7.5, 1.6 Hz, 1H), 7.24 (d, J = 7.4 Hz, 1H), 7.04 (dd, J = 7.5, 1.5 Hz, 1H), 4.92 - 4.80 (m, 2H), 4.36 - 4.29 (m, 1H), 3.88 (s, 3H), 3.71 - 3.67 (m, 1H), 3.54 (s, 2H), 3.41 (d, J = 7.7 Hz, 2H), 3.28 (d, J = 10.2 Hz, 1H), 3.09 (d, J = 7.7 Hz, 2H), 2.67 - 2.54 (m, 2H), 2.31 - 2.20 (m, 2H), 2.17 - 2.09 (m, 2H). |
| 32 | 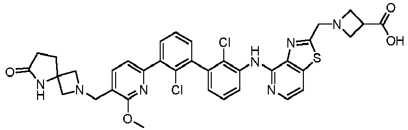 | MS (ESI): m/z = 730.1 [M+H]⁺.¹H NMR (400 MHz, DMSO-*d*₆) δ 8.82 (dd, J = 8.4, 1.5 Hz, 1H), 8.64 (s, 1H), 8.11 (s, 1H), 8.09 (d, J = 5.7 Hz, 1H), 7.68 (d, J = 7.5 Hz, 1H), 7.64 (dd, J = 7.7, 1.7 Hz, 1H), 7.56 (d, J = 5.6 Hz, 1H), 7.51 (t, J = 7.6 Hz, 1H), 7.45 (t, J = 8.0 Hz, 1H), 7.39 (dd, J = 7.5, 1.7 Hz, 1H), 7.24 (d, J = 7.5 Hz, 1H), 7.03 (dd, J = 7.5, 1.5 Hz, 1H), 4.07 (s, 2H), 3.88 (s, 3H), 3.58 (t, J = 7.8 Hz, 2H), 3.54 (s, 2H), 3.43 -3.38 (m, 4H), 3.29 - 3.27 (m, 1H), 3.09 (d, J = 7.7 Hz, 2H), 2.29 - 2.19 (m, 2H), 2.16 - 2.08 (m, 2H). |
| 33 | 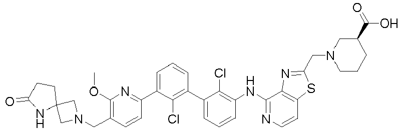 | MS (ESI): m/z = 738.2 [M+H]⁺.¹H NMR (400 MHz, DMSO-*d*₆) δ 8.83 (dd, J = 8.3, 1.5 Hz, 1H), 8.63 (s, 1H), 8.11 - 8.08 (m, 2H), 7.68 (d, J = 7.5 Hz, 1H), 7.64 (dd, J = 7.7, 1.7 Hz, 1H), 7.56 (d, J = 5.6 Hz, 1H), 7.51 (t, J = 7.7 Hz, 1H), 7.45 (t, J = 8.0 Hz, 1H), 7.39 (dd, J = 7.5, 1.7 Hz, 1H), 7.24 (d, J = 7.4 Hz, 1H), 7.02 (dd, J = 7.5, 1.5 Hz, 1H), 3.99 (s, 2H), 3.88 (s, 3H), 3.54 (s, 2H), 3.41 (d, J = 7.8 Hz, 3H), 3.10 (d, J = 7.7 Hz, 2H), 3.03 - 2.97 (m, 1H), 2.83 - 2.75 (m, 1H), 2.50 - 2.48 (m, 1H), 2.45 - 2.40 (m, 1H), 2.40 - 2.32 (m, 1H), 2.27 - 2.20 (m, 3H), 2.16 - 2.09 (m, 2H), 1.88 - 1.77 (m, 1H), 1.72 - 1.62 (m, 1H), 1.56 - 1.45 (m, 1H), 1.40 - 1.30 (m, 1H). |
| 34 | 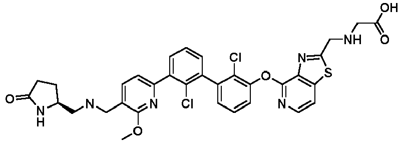 | MS (ESI): m/z = 693.1 [M+H]⁺.¹H NMR (400 MHz, DMSO-*d*₆) δ 10.05 (s, 1H), 8.79 (d, J = 8.3, 1H), 8.17 (s, 1H), 8.10 (d, J = 5.6, 1H), 7.88 (d, J = 7.7 Hz, 1H), 7.65 (dd, J = 7.7, 1.6 Hz, 1H), 7.60 (d, J = 5.6 Hz, 1H), 7.55 (m, 1H), 7.50-7.42 (m, 2H), 7.38 (d, J = 7.6 Hz, 1H), 7.18 (m, 1H), 7.05 (d, J = 7.3 Hz, 1H), 4.48-4.16 (m, 2H), 3.96 (s, 3H), 2.66 - 2.62 (m, 1H), 2.34 - 2.25 (m, 2H), 2.23 - 2.13 (m, 2H). |
| 35 | 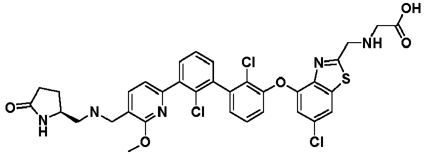 | MS (ESI): m/z = 726.6 [M+H]⁺.¹H NMR (400 MHz, DMSO-*d*₆) δ 7.98 (s, 1H), 7.80-7.78 (m, 1H), 7.65-7.63 (m, 1H), 7.54-7.42 (m, 2H), 7.25-7.23 (m, 2H), 7.19-7.17 (m, 3H), 6.80 (d, J = 2.0 Hz, 1H), 4.11 (s, 2H), 3.88 (s, 3H), 3.68 (s, 2H), 3.66 - 3.62 (m, 1 H), 3.28 (s, 2H), 2.53 - 2.51 (m, 2H), 2.05 - 2.08 (m, 3H), 1.63 - 1.61 (m, 1H). |
| 36 | 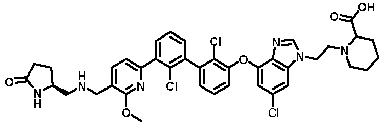 | MS (ESI): m/z = 777.2 [M+H]⁺.¹H NMR (400 MHz, DMSO-*d*₆) δ 8.21 (s, 1H), 7.79 (d, J = 7.6 Hz, 1H), 7.65-7.60 (m, 3H), 7.52-7.32 (m, 3H), 7.25-7.18 (m, 2H), 7.03 (d, J = 7.6 Hz, 1H), 6.65 (d, J = 2.0 Hz, 1H), 4.28 (m, 2H), 3.88 (s, 3H), 3.68-3.60 (m, 1H), 3.08-3.06 (m, 1H), 2.89 - 2.87 (m, 2H), 2.76 - 2.67 (m, 1H), 2.53 - 2.51 (m, 2H), 2.33 - 2.29 (m, 1H), 2.07 - 2.06 (m, 3H), 1.69 - 1.48 (m, 3H), 1.41 - 1.29 (m, 4H). |
| 37 | 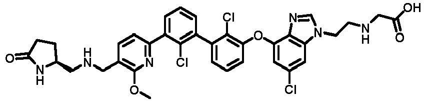 | MS (ESI): m/z = 725.1 [M+H]⁺.¹H NMR (400 MHz, DMSO-*d*₆) δ 8.24 (s, 1H), 7.76 (d, J = 7.2 Hz, 1H), 7.63 (dd, J = 8.0, 2.0 Hz, 1H), 7.51-7.38 (m, 4H), 7.27-7.20 (m, 3H), 6.51 (d, J = 1.2 Hz, 1H), 4.62-4.58 (m, 2H), 4.02 (s, 3H), 3.98-3.97 (m, 2H), 3.90-3.87 (m, 1H), 3.47-3.45 (m, 4H), 2.87 - 2.84 (m, 2H), 2.36 - 2.29 (m, 3H), 1.84 - 1.82 (m, 1H). |
| 38 | 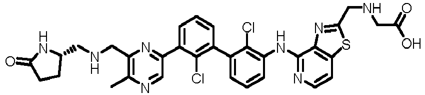 | MS (ESI): m/z = 677.1 [M+H]⁺.¹H NMR (400 MHz, DMSO-*d*₆) δ 8.84 (d, J = 8.4 Hz, 1H), 8.64-8.63 (m, 2H), 8.22 (s, 1H), 8.08 (d, J = 5.5 Hz, 1H), 7.68-7.53 (m, 4H), 7.03 (d, J = 7.7 Hz, 1H), 4.19 (s, 2H), 3.92 (s, 2H), 3.62-3.59 (m, 1H), 3.31 (s, 2H), 2.67 - 2.51 (m, 5H), 2.08 - 2.05 (m, 3H), 1.68 - 1.66 (m, 1H). |
| 39 | 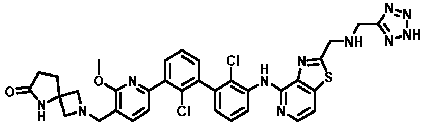 | MS (ESI): m/z = 728.4 [M+H]⁺.¹H NMR (400 MHz, DMSO-*d*₆) δ 10.05 (brs, 1H), 8.78 (d, J = 2.8 Hz, 1H), 8.69-8.67 (m, 1H), 8.11 (s, 1H), 8.09 (d, J = 5.5 Hz, 1H), 7.65-7.63 (m, 1H),7.60-7.37 (m, 6H), 7.05 (m, 2H), 4.39-4.21 (m, 10H), 3.96 (s, 3H), 2.19- 2.17 (m, 2H), 2.15 - 2.13 (m, 2H). |
| 40 | 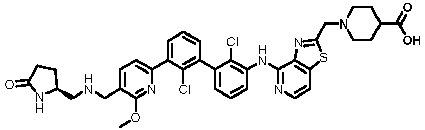 | MS (ESI): m/z = 746.2 [M+H]⁺.¹H NMR (400 MHz, DMSO-*d*₆) δ 8.82 (d, J = 8.4 Hz, 1H), 8.63 (s, 1H), 8.27 (s, 1H), 8.09 (d, J = 5.6 Hz, 1H), 7.79 (d, J = 7.7 Hz, 1H), 7.66 (s, 1H), 7.64 (dd, J = 7.8, 1.7 Hz, 1H), 7.56 (d, J = 5.6 Hz, 1H), 7.51-7.39 (m, 3H), 7.25 (d, J = 7.4 Hz, 1H), 7.03 (dd, J = 7.6, 1.5 Hz, 1H), 3.95 (s, 2H), 3.89 (s, 3H), 3.68 (s, 2H), 3.63- 3.58 (m, 1H), 2.92 - 2.84 (m, 2H), 2.52 - 2.50 (m, 2H), 2.29 - 2.19 (m, 3H), 2.13 - 2.00 (m, 3H), 1.85 - 1.75 (m, 2H), 1.70 - 1.54 (m, 3H). |
| 41 | 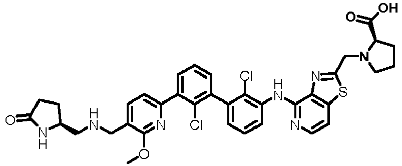 | MS (ESI): m/z = 732.2 [M+H]⁺.¹H NMR (400 MHz, DMSO-*d*₆) δ 8.84 (d, J = 8.4 Hz, 1H), 8.62 (s, 1H), 8.30 (s, 1H), 8.08 (d, J = 5.7 Hz, 1H), 7.79 (d, J = 7.7 Hz, 1H), 7.65 (s, 1H), 7.63-7.38 (m, 5H), 7.25 (d, J = 7.5 Hz, 1H), 7.02 (dd, J = 7.5, 1.5 Hz, 1H), 4.42 (m, 1H), 4.11 (m, 1H), 3.89 (s, 3H), 3.68 (s, 2H), 3.64 - 3.55 (m, 2H), 3.46 - 3.41 (m, 1H), 3.10 - 3.02 (m, 1H), 2.52 (d, J = 6.0 Hz, 2H), 2.44 - 2.28 (m, 1H), 2.13 - 2.01 (m, 3H), 1.94 - 1.81 (m, 1H), 1.81 - 1.59 (m, 3H) |
| 42 | 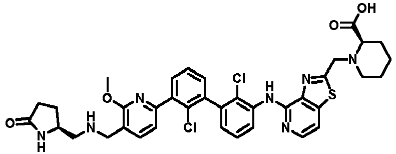 | MS (ESI): m/z = 746.2 [M+H]⁺.¹H NMR (400 MHz, DMSO-*d*₆) δ 8.84 (dd, J = 8.3, 1.3 Hz, 1H), 8.62 (s, 1H), 8.23 (s, 1H), 8.08 (d, J = 5.7 Hz, 1H), 7.79 (d, J = 7.5 Hz, 1H), 7.67 - 7.64 (m, 1H), 7.63 (m, 1H), 7.55-7.38 (m, 4H), 7.25 (d, J = 7.4 Hz, 1H), 7.02 (dd, J = 7.6, 1.5 Hz, 1H), 4.22-4.06 (m, 2H), 3.89 (s, 3H), 3.70 (s, 2H), 3.65 - 3.55 (m, 2H), 3.49 - 3.42 (m, 1H), 3.02 - 2.91 (m, 1H), 2.52 (d, J = 6.0 Hz, 2H), 2.14 - 2.02 (m, 3H), 1.90 - 1.81 (m, 1H), 1.81-1.60 (m, 2H), 1.56 -1.30 (m, 4H). |
| 43 | 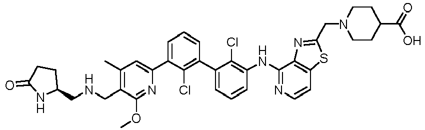 | MS (ESI): m/z = 760.7 [M+H]⁺.¹H NMR (400 MHz, DMSO-*d*₆) δ 8.82 (dd, J = 8.4, 1.5 Hz, 1H), 8.62 (s, 1H), 8.09 (d, J = 5.6 Hz, 1H), 7.62 - 7.58 (m, 2H), 7.56 (d, J = 5.6 Hz, 1H), 7.52 (m, 1H), 7.45 (m, 1H), 7.41 (dd, J = 7.6, 1.6 Hz, 1H), 7.15 (s, 1H), 7.02 (dd, J = 7.6, 1.4 Hz, 1H), 3.95 (s, 2H), 3.90 (s, 3H), 3.75 - 3.66 (m, 1H), 2.88 (m, 2H), 2.68 - 2.53 (m, 1H), 2.47 (s, 2H), 2.40 (s, 3H), 2.34 - 2.17 (m, 4H), 2.17 - 2.03 (m, 3H), 1.80 (m, 2H), 1.74 - 1.65 (m, 1H), 1.64 - 1.53 (m, 2H). |
| 44 | 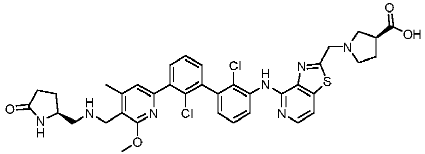 | MS (ESI): m/z = 746.2 [M+H]⁺.¹H NMR (400 MHz, DMSO-*d*₆) δ 8.81 (d, J = 8.2 Hz, 1H), 8.63 (s, 1H), 8.25 (s, 1H), 8.09 (d, J = 5.6 Hz, 1H), 7.63 (s, 1H), 7.60 (dd, J = 7.7, 1.5 Hz, 1H), 7.56 (d, J = 5.6 Hz, 1H), 7.50 (m, 1H), 7.44 (m, 1H), 7.38 (dd, J = 7.4, 1.7 Hz, 1H), 7.09 (s, 1H), 7.02 (dd, J = 7.5, 1.4 Hz, 1H), 4.10 (dd, J = 8.8, 2.0 Hz, 2H), 3.86 (s, 3H), 3.69 (s, 2H), 3.59 - 3.55 (m, 1H), 2.98 - 2.94 (m, 1H), 2.91 - 2.84 (m, 2H), 2.71 (m, 2H), 2.52 (m, 2H), 2.36 (s, 3H), 2.11 - 1.94 (m, 5H), 1.67 - 1.60 (m, 1H). |
| 45 | 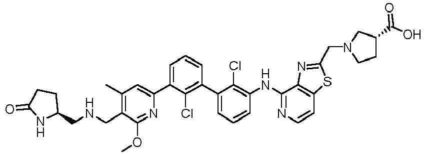 | MS (ESI): m/z = 746.2 [M+H]⁺.¹H NMR (400 MHz, DMSO-*d*₆) δ 8.82 (dd, J = 8.4, 1.5 Hz, 1H), 8.63 (s, 1H), 8.26 (s, 1H), 8.09 (d, J = 5.6 Hz, 1H), 7.63 (s, 1H), 7.60 (dd, J = 7.7, 1.7 Hz, 1H), 7.56 (d, J = 5.6 Hz, 1H), 7.50 (m, 1H), 7.44 (m, 1H), 7.39 (dd, J = 7.5, 1.7 Hz, 1H), 7.09 (s, 1H), 7.02 (dd, J = 7.6, 1.5 Hz, 1H), 4.16 - 4.04 (m, 2H), 3.86 (s, 3H), 3.70 (s, 2H), 3.60 - 3.56 (m, 1H), 2.95 (m, 1H), 2.90 - 2.84 (m, 2H), 2.71 (m, 2H), 2.54 - 2.50 (m, 2H), 2.37 (s, 3H), 2.12 - 1.95 (m, 5H), 1.64 (m, 1H). |
| 46 | 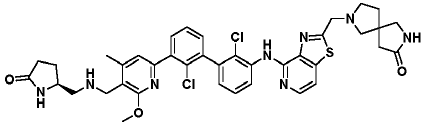 | MS (ESI): m/z = 771.2 [M+H]⁺.¹H NMR (400 MHz, DMSO-*d*₆) δ 8.82 (dd, J = 8.8, 1.6 Hz, 1H), 8.63 (s, 1H), 8.09 (d, J = 5.6 Hz, 1H), 7.63-7.38 (m, 7H), 7.09 (s, 1H), 7.02 (dd, J = 7.2, 1.6 Hz, 1H), 4.12 (s, 2H), 3.86 (s, 3H), 3.69 (s, 2H), 3.27-3.13 (m, 4H), 2.72 - 2.61 (m, 5H), 2.52 - 2.50 (m, 2H), 2.36 (s, 3H), 2.20 - 2.18 (m, 1H), 2.06 - 2.04 (m, 3H), 1.85 - 1.84 (m, 2H), 1.67 - 1.65 (m, 1H). |
| 47 | 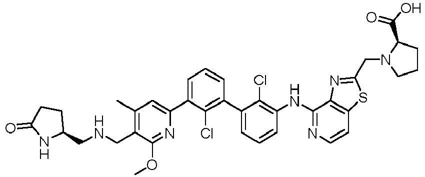 | MS (ESI): m/z = 746.2 [M+H]⁺.¹H NMR (400 MHz, DMSO-*d*₆) δ 8.84 (d, J = 7.5 Hz, 1H), 8.63 (s, 1H), 8.09 (d, J = 5.7 Hz, 1H), 7.63 (s, 1H), 7.60 (dd, J = 7.7, 1.7 Hz, 1H), 7.56 (d, J = 5.6 Hz, 1H), 7.50 (m, 1H), 7.44 (m, 1H), 7.39 (dd, J = 7.6, 1.7 Hz, 1H), 7.09 (s, 1H), 7.02 (dd, J = 7.6, 1.4 Hz, 1H), 4.38 (m, 1 H), 4.13 (m, 1H), 3.86 (s, 3H), 3.70 (s, 2H), 3.62 - 3.54 (m, 1H), 3.52 - 3.47 (m, 1H), 3.11 - 3.02 (m, 1H), 2.68 - 2.59 (m, 1H), 2.56 - 2.50 (m, 2H), 2.37 (s, 3H), 2.14 - 1.99 (m, 4H), 1.91 - 1.82 (m, 1H), 1.82 - 1.72 (m, 2H), 1.68 - 1.60 (m, 1H). |
| 48 | 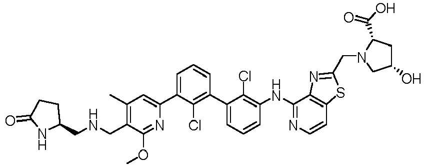 | MS (ESI): m/z = 762.2 [M+H]⁺.¹H NMR (400 MHz, CD₃OD-*d*₄) δ 8.84 (d, J = 8.5 Hz, 1H), 8.63 (s, 1H), 8.19 (s, 1H), 8.09 (d, J = 5.5 Hz, 1H), 7.63 (s, 1H), 7.60 (dd, J = 7.6, 1.6 Hz, 1H), 7.56 (d, J = 5.6 Hz, 1H), 7.50 (m, 1H), 7.44 (m, 1H), 7.39 (dd, J = 7.3, 1.7 Hz, 1H), 7.09 (s, 1H), 7.02 (dd, J = 7.4, 1.6 Hz, 1H), 4.38 (m, 1H), 4.24 - 4.16 (m, 1 H), 4.11 (m, 1H), 3.86 (s, 3H), 3.70 (s, 2H), 3.60 - 3.55 (m, 1H), 3.49 - 3.44 (m, 1H), 2.95 - 2.89 (m, 1H), 2.84 - 2.77 (m, 1H), 2.52 (m, 2H), 2.37 (s, 3H), 2.35 - 2.31 (m, 1 H), 2.11 - 2.00 (m, 4H), 1.81 - 1.72 (m, 1H), 1.67 - 1.61 (m, 1H). |
| 49 | 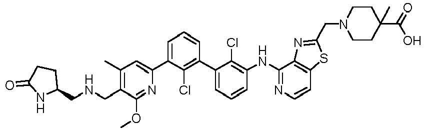 | MS (ESI): m/z = 774.2 [M+H]⁺.¹H NMR (400 MHz, DMSO-*d*₆) δ 8.81 (dd, J = 8.3, 1.2 Hz, 1H), 8.62 (s, 1H), 8.27 (s, 1H), 8.08 (d, J = 5.6 Hz, 1H), 7.63 (s, 1H), 7.60 (dd, J = 7.6, 1.6 Hz, 1H), 7.55 (d, J = 5.6 Hz, 1H), 7.50 (m, 1H), 7.44 (m, 1H), 7.38 (dd, J = 7.5, 1.7 Hz, 1H), 7.08 (s, 1H), 7.01 (dd, J = 7.5, 1.4 Hz, 1H), 3.93 (s, 2H), 3.86 (s, 3H), 3.69 (s, 2H), 3.59 - 3.56 (m, 1H), 2.76 - 2.66 (m, 2H), 2.54 - 2.50 (m, 2H), 2.41 - 2.27 (m, 5H), 2.12 - 1.92 (m, 5H), 1.68 - 1.59 (m, 1H), 1.46 - 1.34 (m, 2H), 1.10 (s, 3H). |
| 50 | 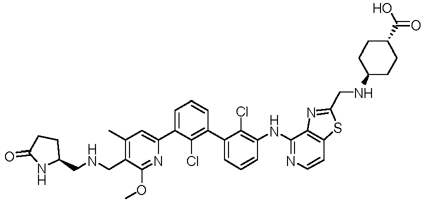 | MS (ESI): m/z = 774.2 [M+H]⁺.¹H NMR (400 MHz, DMSO-*d*₆) δ 8.86 (dd, J = 8.3, 1.4 Hz, 1H), 8.60 (s, 1H), 8.12 (s, 1H), 8.07 (d, J = 5.6 Hz, 1H), 7.63 (s, 1H), 7.60 (dd, J = 7.7, 1.7 Hz, 1H), 7.54 (d, J = 5.6 Hz, 1H), 7.50 (m, 1H), 7.44 (m, 1H), 7.39 (dd, J = 7.5, 1.7 Hz, 1H), 7.09 (s, 1H), 7.01 (dd, J = 7.5, 1.5 Hz, 1H), 4.15 (s, 2H), 3.86 (s, 3H), 3.72 (s, 2H), 3.62 - 3.57 (m, 1H), 2.55 (m, 2H), 2.44 - 2.39 (m, 1H), 2.37 (s, 3H), 2.14 - 2.00 (m, 4H), 1.95 - 1.80 (m, 4H), 1.71 - 1.59 (m, 1H), 1.33 - 1.19 (m, 2H), 1.12 - 1.00 (m, 2H). |
| 51 | 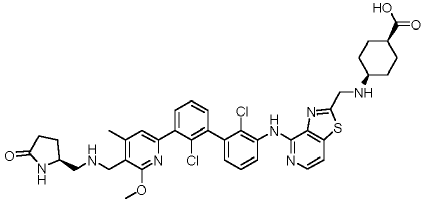 | MS (ESI): m/z = 774.2 [M+H]⁺.¹H NMR (400 MHz, DMSO-*d*₆) δ 8.85 (dd, J = 8.4, 1.2 Hz, 1H), 8.60 (s, 1H), 8.33 (s, 1H), 8.07 (d, J = 5.6 Hz, 1H), 7.64 (s, 1H), 7.60 (dd, J = 7.7, 1.7 Hz, 1H), 7.55 (d, J = 5.6 Hz, 1H), 7.50 (m, 1H), 7.44 (m, 1H), 7.38 (dd, J = 7.5, 1.6 Hz, 1H), 7.09 (s, 1H), 7.01 (dd, J = 7.6, 1.4 Hz, 1H), 4.12 (s, 2H), 3.86 (s, 3H), 3.69 (s, 2H), 3.58 - 3.56 (m, 1H), 2.65 - 2.58 (m, 1H), 2.55 - 2.49 (m, 2H), 2.37 (s, 3H), 2.34 - 2.22 (m, 2H), 2.12 - 1.99 (m, 3H), 1.93 - 1.81 (m, 2H), 1.71 - 1.52 (m, 3H), 1.48 - 1.40 (m, 3H). |
| 52 | 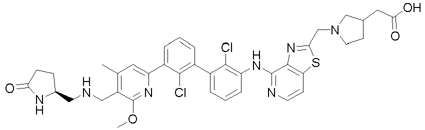 | MS (ESI): m/z = 760.2 [M+H]⁺.¹H NMR (400 MHz, DMSO-*d*₆) δ 8.82 (dd, J = 8.4, 1.3 Hz, 1H), 8.63 (s, 1H), 8.16 (s, 1H), 8.09 (d, J = 5.6 Hz, 1H), 7.64 (s, 1H), 7.60 (dd, J = 7.7, 1.7 Hz, 1H), 7.56 (d, J = 5.6 Hz, 1H), 7.50 (m, 1H), 7.45 (m, 1H), 7.39 (dd, J = 7.6, 1.7 Hz, 1H), 7.09 (s, 1H), 7.02 (dd, J = 7.6, 1.5 Hz, 1H), 4.09 (m, 3H), 3.86 (s, 3H), 3.70 (s, 2H), 3.59 - 3.56 (m, 1H), 2.89 - 2.80 (m, 2H), 2.74 - 2.66 (m, 2H), 2.53 (m, 2H), 2.41 - 2.34 (m, 4H), 2.31 (m, 2H), 2.12 - 1.96 (m, 4H), 1.70 - 1.61 (m, 1H), 1.46 - 1.36 (m, 1H). |
| 53 | 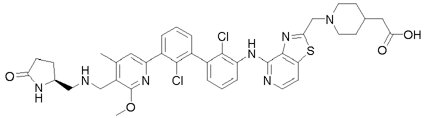 | MS (ESI): m/z = 774.2 [M+H]⁺.¹H NMR (400 MHz, DMSO-*d*₆) δ 8.82 (dd, J = 8.3, 1.2 Hz, 1H), 8.62 (s, 1H), 8.16 (s, 1H), 8.09 (d, J = 5.6 Hz, 1H), 7.64 (s, 1H), 7.60 (dd, J = 7.7, 1.7 Hz, 1H), 7.55 (d, J = 5.6 Hz, 1H), 7.50 (m, 1H), 7.44 (m, 1H), 7.38 (dd, J = 7.5, 1.7 Hz, 1H), 7.09 (s, 1H), 7.02 (dd, J = 7.6, 1.5 Hz, 1H), 3.94 (s, 2H), 3.86 (s, 3H), 3.71 (s, 2H), 3.61 - 3.58 (m, 1H), 2.90 (m, 2H), 2.53 (m, 2H), 2.37 (s, 3H), 2.25 - 2.11 (m, 4H), 2.11 - 1.99 (m, 3H), 1.72 - 1.58 (m, 4H), 1.30 - 1.17 (m, 2H). |
| 54 | 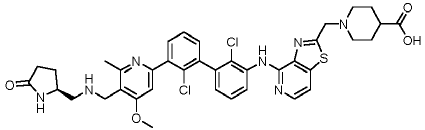 | MS (ESI): m/z = 760.2 [M+H]⁺.¹H NMR (400 MHz, DMSO-*d*₆) δ 8.82 (d, J = 8.4 Hz, 1H), 8.63 (s, 1H), 8.25 (s, 1H), 8.09 (d, J = 5.6 Hz, 1H), 7.63 (s, 1H), 7.58 - 7.36 (m, 5H), 7.07 (s, 1H), 7.01 (d, J = 7.6 Hz, 1H), 3.95 (s, 2H), 3.83 (s, 3H), 3.70 (s, 2H), 3.60 - 3.56 (m, 2H), 3.14 - 3.11 (m, 1H), 2.92 - 2.83 (m, 2H), 2.51 (s, 3H), 2.32 - 2.14 (m, 4H), 2.05 (s, 3H), 1.85 - 1.75 (m, 2H), 1.69 - 1.52 (m, 3H). |
| 55 | 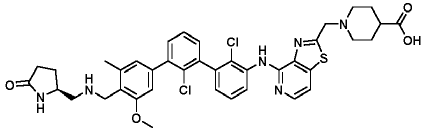 | MS (ESI): m/z = 759.2 [M+H]⁺.¹H NMR (400 MHz, DMSO-*d*₆) δ 8.81 (dd, J = 8.4,1.2 Hz, 1H), 8.63 (s, 1H), 8.08 (d, J = 5.6 Hz, 1H), 7.63 (s, 1H), 7.57 - 7.33 (m, 5H), 7.02-6.99 (dd, J = 7.6, 1.6 Hz, 1H), 6.85 (m, 2H), 3.95 (s, 2H), 3.77 (s, 3H), 3.71 (s, 2H), 2.89 - 2.86 (m, 2H), 2.55 - 2.53 (m, 2H), 2.35 (s, 3H), 2.28 - 2.22 (m, 3H), 2.07-2.05 (m, 3H), 1.82 - 1.78 (m, 2H), 1.64 - 1.57 (m, 3H). |
| 56 | 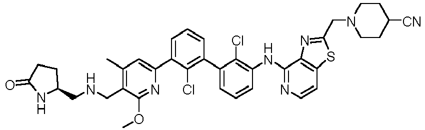 | MS (ESI): m/z = 741.2 [M+H]⁺.¹H NMR (400 MHz, DMSO-*d*₆) δ 8.81 (dd, J = 8.3, 1.3 Hz, 1H), 8.63 (s, 1H), 8.09 (d, J = 5.6 Hz, 1H), 7.63 (s, 1H), 7.60 (dd, J = 7.7, 1.7 Hz, 1H), 7.56 (d, J = 5.6 Hz, 1H), 7.50 (m, 1H), 7.44 (m, 1H), 7.38 (dd, J = 7.5, 1.7 Hz, 1H), 7.09 (s, 1H), 7.02 (dd, J = 7.6, 1.5 Hz, 1H), 3.99 (s, 2H), 3.86 (s, 3H), 3.71 (s, 2H), 3.62 - 3.56 (m, 1H), 2.98 - 2.85 (m, 2H), 2.77 - 2.67 (m, 3H), 2.58 - 2.52 (m, 2H), 2.37 (s, 3H), 2.12 - 2.01 (m, 3H), 1.98 - 1.83 (m, 4H), 1.78 - 1.70 (m, 2H), 1.68-1.61 (m, 1H). |
| 57 | 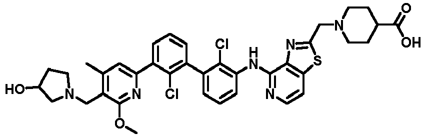 | MS (ESI): m/z = 733.2 [M+H]⁺.¹H NMR (400 MHz, DMSO-*d*₆) δ 8.83 (dd, J = 8.3, 1.4 Hz, 1H), 8.63 (s, 1H), 8.09 (d, J = 5.6 Hz, 1H), 7.63 (m, 1H), 7.60 (m, 1H), 7.56 - 7.46 (m, 2H), 7.39-7.37 (m, 1H), 7.09 (s, 1H), 7.02 (dd, J = 7.6, 1.5 Hz, 1H), 4.20 (m, 1H), 3.95 (s, 2H), 3.85 (s, 3H), 3.57 (m, 2H), 3.47 (m, 1H), 2.89 - 2.85 (m, 2H), 2.67 - 2.47 (m, 2H), 2.45 - 2.26 (m, 4H), 1.98 - 1.73 (m, 3H), 1.75 - 1.40 (m, 3H). |
| 58 | 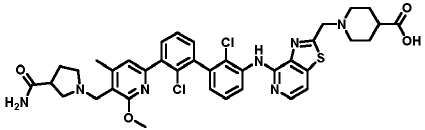 | MS (ESI): m/z = 760.2 [M+H]⁺.¹H NMR (400 MHz, DMSO-*d*₆) δ 8.83 (dd, J = 8.0, 1.2 Hz, 1H), 8.63 (s, 1H), 8.09 (d, J = 5.6 Hz, 1H), 7.63 (m, 1H), 7.61 (m, 1H), 7.57 - 7.43 (m, 2H), 7.39-7.37 (m, 1H), 7.18 (s, 1H), 7.09 (s, 1H), 7.02 (dd, J = 7.6, 1.5 Hz, 1H), 6.72 (s, 1H), 3.96 (s, 2H), 3.85 (s, 3H), 3.58 (m, 2H), 3.37 (m, 1H), 2.89 - 2.86 (m, 2H), 2.75 - 2.72 (m, 2H), 2.61 - 2.59 (m, 1H), 2.44 - 2.41 (m, 1H), 2.28 - 2.22 (m, 3H), 1.84 - 1.78 (m, 4H), 1.60 - 1.57 (m, 2H). |

### Test Example 1: Detect the inhibitory effect of the compound to the binding of PD-1/PD-L1 protein

The PD-1/PD-L1 homogeneous time-resolved fluorescence method is used to detect the binding ability of the compound with PD-L1.

PD-1/PD-L1 binding assay kit (Cisbio, Cat#63ADK000CPDEC) was chosen, which contains two proteins Tag 1-PD-L1 and Tag 2-PD-1, and two antibodies Anti-Tag1-Eu³⁺ and Anti-Tag2- XL 665. The principle of detection was: Anti-tag1-Eu³⁺ was used as the donor of HTRF, and Anti-Tag2-XL 665 was used as the acceptor of HTRF. When Tag 1-PD-L1 interacts with Tag 2-PD-1, the added HTRF donor and the acceptor are close to each other. After the donor has received the excitation energy, part of the energy was transferred to the acceptor, thus generating 665 nm emission light. When the addition of the compound blocked the PD-1/PD-L1 interaction, only 620 nm emission light was generated. The inhibitory effect of the compound can be determined by comparing the ratio of 665 nm/620 nm. Tag 1-PD-L1 was diluted with Diluent buffer (cat# 62DLBDDF) to a working concentration of 10 nM, Tag 2-PD-1 was diluted with Diluent buffer to a working concentration of 500 nM, Anti-Tagl-Eu³⁺ was diluted with detection buffer (cat# 62DB1FDG) at a ratio of 1:100, Anti-Tag2-XL 665 was diluted with detection buffer at a ratio of 1:20, and the compound to be detected was diluted with diluent buffer to a final concentration of 2X. Compound solution (2 µL) was added to each well of a 384-well plate, then Tag 1-PD-L1 (4 µL) and Tag 2-PD-1 (4 µL) were added successively. The mixture was incubated at room temperature for 15 minutes. Then Anti-Tag1-Eu³⁺ (5 µL) and Anti-Tag2-XL 665 (5 µL) were added, and the mixture was incubated overnight at room temperature. BioTek Synergy™ Neo2 multifunctional microplate reader was used to detect so as to provide the 665 nm/620 nm ratio. PrismGraphd 5.02 was used to fit the IC₅₀ curve.

**Table 1 IC₅₀ values of some compounds of the present invention**

| Compound number | PD-L1 IC₅₀ |
|---|---|
| 1 | A |
| 2 | A |
| 3 | A |
| 4 | A |
| 5 | A |
| 6 | B |
| 7 | B |
| 8 | A |
| 9 | N/T |
| 10 | A |
| 11 | N/T |
| 12 | A |
| 13 | N/T |
| 14 | A |
| 15 | A |
| 16 | A |
| 17 | B |
| 18 | A |
| 19 | A |
| 20 | A |
| 21 | A |
| 22 | A |
| 23 | A |
| 24 | A |
| 25 | A |
| 26 | B |
| 27 | A |
| 28 | A |
| 29 | A |
| 30 | A |
| 31 | A |
| 32 | A |
| 33 | A |
| 34 | A |
| 35 | N/T |
| 36 | N/T |
| 37 | N/T |
| 38 | N/T |
| 39 | A |
| 40 | A |
| 41 | A |
| 42 | A |
| 43 | A |
| 44 | A |
| 45 | A |
| 46 | A |
| 47 | A |
| 48 | A |
| 49 | A |
| 50 | A |
| 51 | A |
| 52 | A |
| 53 | A |
| 54 | A |
| 55 | A |
| 56 | A |
| 57 | NT |
| 58 | NT |

| | |
|---|---|
| A represents IC₅₀ of less than 100 nM; B represents IC₅₀ of 100 nM to 1 uM; C represents IC₅₀ of greater than 1 uM; | |

The results show that the compound of the present invention can effectively inhibit the binding of PD-1/PD-L1 at different concentrations. Therefore, it can be used in the treatment of diseases related to the PD-1/PD-L1 interaction.

### Test example 2: Cellular NFAT reporter gene assay

Two types of cells (PD-1 effector cells and PD-L1 aAPC/CHO-K1 cells) were required in the cellular assay of PD-1/PD-L1. PD-1 effector cells express human PD-1 protein and luciferase report gene driven by NFAT, while PD-L1 aAPC/CHO-K1 cells express PD-L1 protein and anti-CD3 antibody. When these two kinds of cells were co-cultured, the interaction of PD-1/PD-L1 would inhibit the signal transmission from TCR to NFAT-RE and interrupt the fluorescence signal mediated by NFAT-RE. When the inhibitor of PD-1 or PD-L1 was added, the interaction of PD-1/PD-L1 was blocked, thus alleviating the inhibitory signal of the TCR to NFAT-RE pathway and enhancing fluorescence signal. The blocking effect of inhibitors was determined by the strength of fluorescence signals.

On the first day of the experiment, the recovered PD-L1 aAPC/CHO-K1 cells were trypsinized. After centrifugation, the concentration was adjusted to 2.5^{∗}10⁵/mL with the culture medium (90% Ham's F-12/10% FBS). Cells were plated in a 384-well plate at an amount of 40 µL, 1^{∗}10⁴ cells per well and placed in an incubator for overnight culture. The next day, the compound to be detected was diluted to 2 times of the required detection concentration with the detection buffer (99% RPMI1640/1% FBS) in gradient. PD-1 cells were centrifugated and adjusted to 6.25^{∗}10⁵/ mL using the detection buffer. The medium in the overnight cultured 384-well plate was discarded, and 20 µL of the diluted compound was added to each well, and 20 µL of PD-1 cells were added. After the plate was incubated in the cell culture incubator for 6 hours, 20 µL of Bio-Glo reagent was added to each well (Promega, cat#G7940). The plate was read with a multi-function microplate reader after 10 minutes. A negative control (only add cells, no compound), and a blank control (only add detection buffer) need to be set for each plate. Prism5 was used to analyze the inhibitory activity of the compound according to the fluorescence value.

The results show that the compound of the present invention can effectively block the interaction of PD-1/PD-L1, and the half-active inhibitory concentration is equivalent to or better than that of the PD-1 inhibitors in the clinical stage.

### Test example 3: Human PBMC functional assays

Two kinds of cells (human PBMC cells and PD-L1 aAPC/CHO-K1 cells) were required in human PBMC functional assay (CHO-K1 cells express full-length human PD-L1 protein and anti-hCD3 antibody). When the two kinds of cells were co-cultured, the anti-hCD3 antibody expressed by aAPC cells binds to CD3 on the surface of PBMC to stimulate PBMC activation and proliferation, but the interaction of PD-1/PD-L1 would inhibit TCR signal transmission. When PD-1 or PD-L1 inhibitors was added, the interaction of PD-1/PD-L1 was blocked, thus releasing the signal to the TCR pathway. The blocking effect of inhibitors was evaluated by detecting the changes of cell surface markers CD25, PD-1 and intracellular cytokine IFNγ after PBMC activation.

On the first day of the experiment, the recovered PD-L1 aAPC/CHO-K1 cells and PD-L1 Negative cells were trypsinized, and the cell concentration was adjusted to 2^{∗}10⁵/mL with medium (90% F12+10% FBS) after centrifugation. Cells were plated in a 96-well plate at 100 µL, 2^{∗}10⁴ cells per well and placed in an incubator for overnight culture. The next day, the cells were treated with a medium containing 10 µg/mL Mitomycin C. The plate was placed under 37 °C for 4 hours and washed three times with PBS. The compound to be tested was prepared and diluted in gradient to 2 times of the required detection concentration. PBMC cells were recovered and adjusted to a concentration of 1^{∗}10⁶/mL according to the operating requirements. Compound (50 µL) was added to the plate, and then 50 µL PBMC cells were added to the plate. After three days of incubation in a cell incubator, the cell supernatant was collected to detect the change of cytokine IFNγ by ELISA. Cells are collected for flow cytometry to detect changes in CD25 and PD-1.

The results show that the compounds of the present invention can effectively block the interaction of PD-1/PD-L1, and the half-active inhibitory concentration is equivalent to or better than that of the PD-1 inhibitors in the clinical phase.

### Test Example 4: In vivo drug efficacy study of the small molecule inhibitors of the present invention in treating tumors

A mouse model of subcutaneously inoculated tumors was established to examine the *in vivo* inhibitory effects of these compounds on tumor growth. The method was presented as follows: after the cultured specific tumor cells was trypsinized, the cells were collected by centrifugation, washed twice with sterile saline and counted. The cells were adjusted to the required concentration with saline. 0.2 ml of cell suspension was subcutaneously inoculated into C57BL/6 or Balb/c immunocompetent mice. After inoculation, the tumor growth was observed until becoming a specific volume. Animals were randomly grouped (6-7 animals in each group) and administered after weighing. The tested compound was administered once a day. The grouping includes: vehicle group, control anti-PD-Ll antibody group, testing compound groups. Mice were tested for tumor growth every week for about 6 weeks. After the tumor volume reached the tumor endpoint, the mice were weighed and euthanized. The tumor tissue, spleen tissue and blood samples were taken. Then the tumor inhibition rate was calculated, the immune cell composition in the tumor, spleen and blood samples was detected, and the immunomodulatory activity of the test compound was calculated.

The results show that the compounds of the present invention can effectively inhibit tumor growth in tumor-bearing mice, and inhibitory effect thereof is equivalent to or better than that of PD-1 inhibitors in the clinical phase.

### Test Example 5: In vivo drug efficacy study of the small molecule inhibitors of the present invention combined with chemotherapeutics in treating tumors

A mouse model of subcutaneously inoculated tumors was established to test the in vivo inhibitory effects of these compounds on tumor growth. The method was presented as follows: after the cultured specific tumor cells was trypsinized, the cells were collected by centrifugation, washed twice with sterile saline and counted. The cells were adjusted to the required concentration with saline. 0.2 mL of cell suspension was subcutaneously inoculated into C57BL/6 or Balb/c immunocompetent mice. After inoculation, the tumor growth was observed until becoming a specific volume. Animals were randomly grouped (6-7 animals in each group) and administered after weighing. The tested compound and the combination drug bevacizumab/Carboplatin/Paclitaxel/Pemetrexed were administered in accordance with the combination regimen. The grouping includes: vehicle group, tested compound combined with chemotherapeutics group, chemotherapeutics group, and tested compound group. Mice were tested for tumor growth every week for about 6 weeks. After the tumor volume reached the tumor endpoint, the mice were weighed and euthanized. The tumor tissue, spleen tissue and blood samples were taken. Then the tumor inhibition rate was calculated, the immune cell composition in the tumor, spleen and blood samples was detected, and the immunomodulatory activity of the test compound and the combination drug was calculated.

The results show that the compounds of the present invention combined with chemotherapeutics can inhibit tumor growth in tumor-bearing mice more effectively, and inhibitory effect thereof is better than that of chemotherapeutics alone.

### Test Example 6: In vivo drug efficacy study of the small molecule inhibitor of the present invention combined with immunomodulator in treating tumors

A mouse model of subcutaneously inoculated tumors was established to test the in vivo inhibitory effects of these compounds on tumor growth. The method was presented as follows: after the cultured specific tumor cells was trypsinized, the cells were collected by centrifugation, washed twice with sterile saline and counted. The cells were adjusted to the required concentration with saline. 0.2 mL of cell suspension was subcutaneously inoculated into C57BL/6 or Balb/c immunocompetent mice. After inoculation, the tumor growth was observed until becoming a specific volume. Animals were randomly grouped (6-7 animals in each group) and administered after weighing.The test compound and the combination drug Nivolumab/Ipilimumab were administered in accordance with the combination regimen. The grouping includes: vehicle group, testing compound combined with immunomodulator group, immunomodulator group, and testing compound group. Mice were tested for tumor growth every week for about 6 weeks. After the tumor volume reached the tumor endpoint, the mice were weighed and euthanized. The tumor tissue, spleen tissue and blood samples were taken. Then the tumor inhibition rate was calculated, the immune cell composition in the tumor, spleen and blood samples was detected, and the immunomodulatory activity of the test compound and the combination drug was calculated.

The results show that the compounds of the present invention combined with immunomodulatory drugs can inhibit tumor growth in tumor-bearing mice more effectively, and inhibitory effect thereof is better than that of immunomodulatory drugs alone.

### Test Example 7: The pharmacokinetic study of the small molecule inhibitors of the present invention in mice

The tested compound was administered to ICR mice intravenously (IV) and orally (PO) separately. Blood samples were collected at different time points. The concentration of test compound in the mouse plasma was determined by LC-MS/MS and related parameters were calculated. The details were as follows: the required amount of the test article was dissolved in 5% DMSO+10% Solutol+85% water for injection to prepare a solution of the required concentration for intravenous or oral administration. The animals were about 6-8 weeks old at the start of the dosing experiment. Blood collection time points for intravenous: 0.083 h, 0.25 h, 0.5 h, 1 h, 2 h, 4 h, 8 h and 24 h after administration. Blood collection time points for oral: 0.25 h, 0.5 h, 1 h, 2 h, 4 h, 6 h, 8 h and 24 h after administration. Biological sample analysis methods and sample detection methods were established. The drug blood concentration at different time points were used to calculate the pharmacokinetic parameters with Phoenix WinNonlin 7.0 software, such as AUC₍₀₋ₜ₎, AUC_{(0-∞)}, T_{1/2}, Cₘₐₓ, Tₘₐₓ and MRT, etc.

The results show that the compounds of the present invention show excellent pharmacokinetic properties.

### Test Example 8: The pharmacokinetic study of the small molecule inhibitors of the present invention in rat

The test compound was administered to SD rat intravenously (IV) and orally (PO) separately. Blood samples were collected at different time points. The concentration of test compound in the rat plasma was determined by LC-MS/MS and related parameters were calculated. The details were as follows: the required amount of the test article was dissolved in 5% DMSO+10% Solutol+85% water for injection to prepare a solution of the required concentration for intravenous or oral administration. The animals were about 6-8 weeks old at the start of the dosing experiment. Blood collection time points for intravenous: 0.083 h, 0.25 h, 0.5 h, 1 h, 2 h, 4 h, 8 h and 24 h after administration. Blood collection time points for oral: 0.25 h, 0.5 h, 1 h, 2 h, 4 h, 6 h, 8 h and 24 h after administration. Biological sample analysis methods and sample detection methods were established. The drug blood concentration at different time points were used to calculate the pharmacokinetic parameters with Phoenix WinNonlin 7.0 software, such as AUC₍₀₋ₜ₎, AUC_{(0-∞)}, T_{1/2}, Cₘₐₓ, Tₘₐₓ and MRT, etc.

The results show that the compounds of the present invention show excellent pharmacokinetic properties.

All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A compound according to Formula I, the stereoisomers or tautomers thereof, or pharmaceutically acceptable salts, hydrates or solvates thereof: 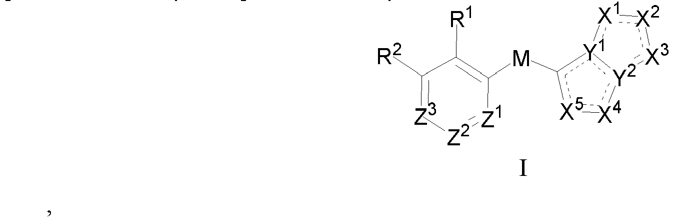 wherein,
M is selected from O and NH;
X² is selected from N, NR³, CR³, O, S, N=CR³, CR³=N and CR³=CR³;
X⁴ is selected from N, CH, O, S, N=CH, CH=N and CH=CH; and
only one of X² and X⁴ is selected CR³=CR³, N=CR³ or CR³=N (R₃ is H in X⁴);
X¹ and X⁵ are independently selected from N, CH, O or S;
X³ is selected of N, CR³, O or S; and
at least one of X³ and X⁴ is O, S or N;
Y¹ and Y² are independently N or C;
Z¹, Z² and Z³ are independently N or CR⁴;
R¹ is selected from of H, halogen, CN, substituted or unsubstituted C₁-C₆ alkyl, substituted and unsubstituted C₁-C₆ alkoxyl;
R² is selected from substituted or unsubstituted C₆-C₁₀ aryl, substituted or unsubstituted 5-10 membered heteroaryl with 1-3 heteroatoms selected from N, S and O, substituted or unsubstituted 5-10 membered heterocyclyl with 1-3 heteroatoms selected from N, S and O; wherein one or more H of R² are substituted by R⁵;
R³ is selected from the group consisting of H, substituted or unsubstituted C₁-C₆ alkyl, or 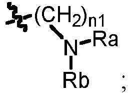 and at least one of R³ is 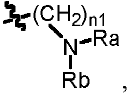 n1 is 1, 2, 3 or 4;
Ra and Rb are independently selected from of H, -(C=O)-substituted or unsubstituted C₁-Cs alkyl, substituted or unsubstituted C₁-C₈ alkyl, substituted or unsubstituted C₂-C₆ alkenyl, substituted or unsubstituted C₂-C₆ alkynyl, substituted or unsubstituted C₁-C₈ alkylamino, substituted or unsubstituted C₁-C₈ alkoxyl, substituted or unsubstituted C₃-C₁₀ cycloalkyl, substituted or unsubstituted 3-10 membered heterocyclyl with 1-3 heteroatoms selected from the group consisting of N, S and O, substituted or unsubstituted C₆-C₁₀ aryl, substituted or unsubstituted 5-10 membered heteroaryl with 1-3 heteroatoms selected from the group consisting of N, S and O, substituted or unsubstituted 5-10 membered heterocyclyl with 1-3 heteroatoms selected from the group consisting of N, S and O; or
Ra and Rb and adjacent N atoms together form a substituted or unsubstituted 5-10 membered heterocyclyl with 1-3 heteroatoms selected from the group consisting of N, S and O;
R⁴ is selected from H, halogen, CN, substituted or unsubstituted C₁-C₆ alkyl, substituted or unsubstituted C₁-C₆ alkoxyl;
R⁵ is selected from H, CN, halogen, substituted or unsubstituted (-L₁-L₂-(CH₂)_{q} - (substituted or unsubstituted 5-7 membered heteroaryl)), substituted or unsubstituted (-L₁-L₂-(CH₂)_{q}-N(Ra)(Rb)), -O-substituted or unsubstituted (-(CH₂)_{q}O-(CH₂)_{q}-N(Ra)(Rb)), or substituted or unsubstituted group selected from the group: C₆-C₁₀ aryl group, 5-10 membered heteroaryl with 1-3 heteroatoms selected from N, S and O, -L₁-L₂-6-10 membered aryl-(CH₂)_{q}-N(Ra)(Rb), -L₁-L₂-5-10 membered heteroaryl-(CH₂)_{q}-N(Ra)(Rb); wherein L₁ and L₂ are independently selected from the group consisting of lnone, substituted or unsubstituted C₁-C₈ alkylene, -NH-C(=O)-NH-, -C(=O)-NH-, -O-, -S- or -NH-; or two RS and the connected carbon atoms together form a group selected from substituted or unsubstituted 5-7 membered heteroaryl, substituted or unsubstituted 5-7 membered heterocyclyl;
q is selected from the group consisting of 0, 1, 2, 3, 4, 5, 6, 7 or 8;
unless otherwise specified, "substituted" refers to being substituted by one or more (for example, 2, 3, 4, etc.) substituents selected from the group consisting of halogen, C₁-C₆ alkoxyl, halogenated C₁-C₆ alkoxyl, C₃-C₈ cycloalkyl, halogenated C₃-C₈ cycloalkyl, methyl sulfone, - S(=O)₂NH₂, -S(=O)₂NHCH₃, oxo(=O), -CN hydroxyl, -NH₂, C₁-C₆ amine carboxy, C₁-C₆ amide (-C(=O)-N(Rc)₂ or -NH-C(=O)(Rc), Rc is H or C₁-C₅ alkyl), 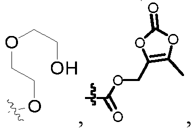 or substituted or unsubstituted groups selected from the group consisting of C₁-C₆ alkyl, C₆-C₁₀ aryl, 5-10 membered heteroaryl with 1-3 heteroatoms selected from N, S, O and 5-10 membered heterocyclyl (including fused ring, spiro ring) with 1-3 heteroatoms selected from N, S and O, -(CH₂) -C₆-C₁₀ aryl, -(CH₂)- (5-10 membered heteroaryl with 1-3 heteroatoms selected from N, S and O), and the substitution means replaced by substituents selected from the group consisting of halogen, C₁-C₆ alkyl, C₁-C₆ alkoxyl, oxo, -CN, -OH, C₆-C₁₀ aryl, 5-10 membered heteroaryl with 1-3 heteroatoms selected from N, S and O, -O-(CH₂)_{q}-CN;
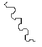 is the attachment site of the group;
each 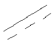 is independently a single bond or a double bond;
with the proviso that the compound of Formula I has a chemically stable structure.

2. The compound of claim 1, stereoisomers or tautomers thereof, or pharmaceutically acceptable salts, hydrates or solvates thereof, wherein the compound has a structure according to Formula II:

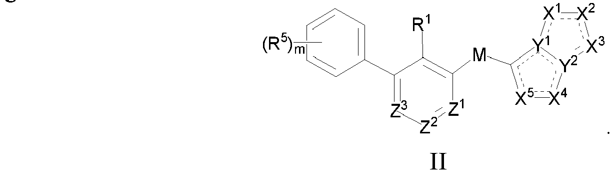

3. The compound of claim 1, stereoisomers or tautomers thereof, or pharmaceutically acceptable salts, hydrates or solvates thereof, wherein the compound has a structure according to Formula III: 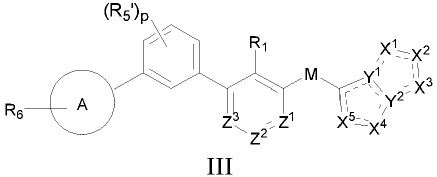 Wherein R₅' is selected from the group consisting of H, CN, halogen, substituted or unsubstituted C₁-C₆ alkyl, substituted or unsubstituted C₁-C₆ alkoxyl;
R₆ is (C₁-C₈ alkylene)-N(Ra)(Rb);
ring A is selected from the group consisting of substituted or unsubstituted C₆-C₁₀ aryl, substituted or unsubstituted 5-10 membered heteroaryl with 1-3 heteroatoms selected from N, S and O, substituted or unsubstituted 5-10 membered heterocyclyl with 1-3 heteroatoms selected from N, S and O;
p is 1, 2, 3, or 4.

4. The compound of claim 1, wherein 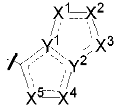 is a structure formed by a heterocyclic ring selected from the group consisting of

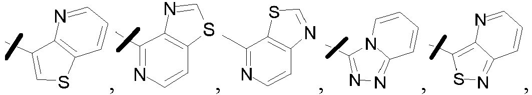

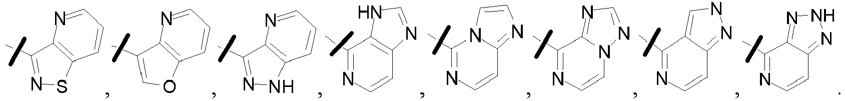

5. The compound of claim 1, wherein the compound has a structure according to Formula IV: 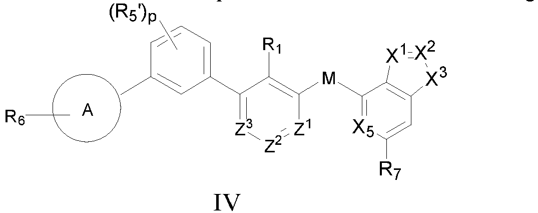 wherin,
X¹ is selected from the group consisting of N or CH;
X² is selected from the group consisting of N or CR₃;
X³ is selected from the group consisting of NR³, O or S;
R₇ is selected from the group consisting of H and halogen.

6. The compound of claim 1, wherein the compound of Formula I is selected from the following group:

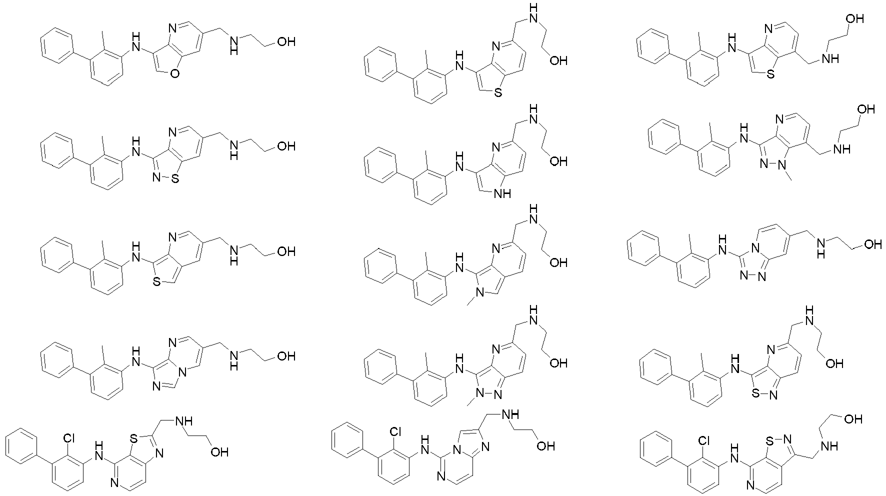

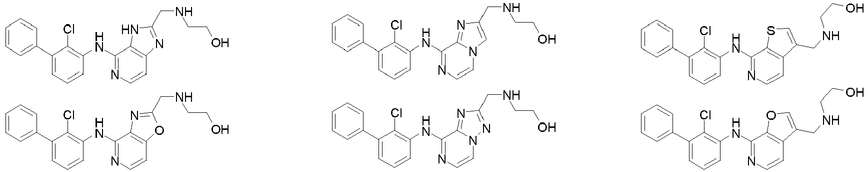

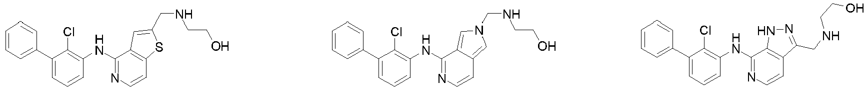

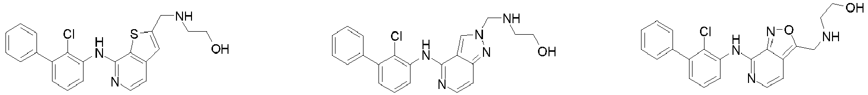

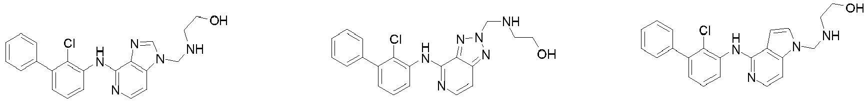

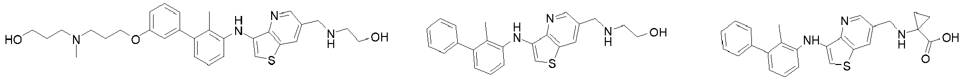

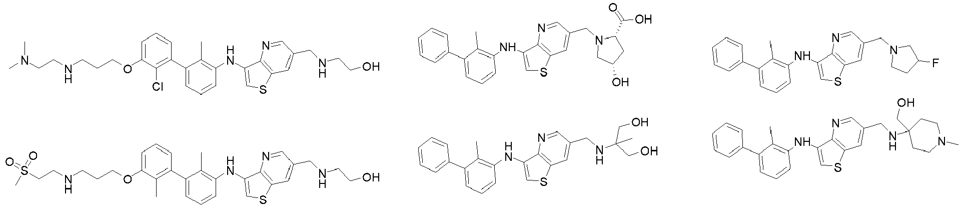

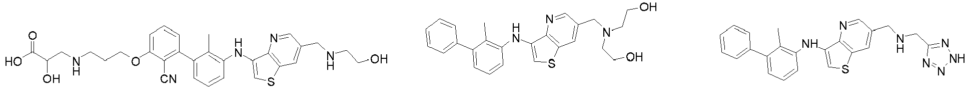

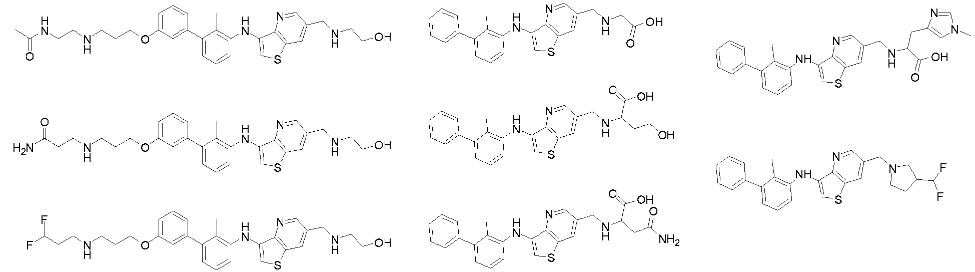

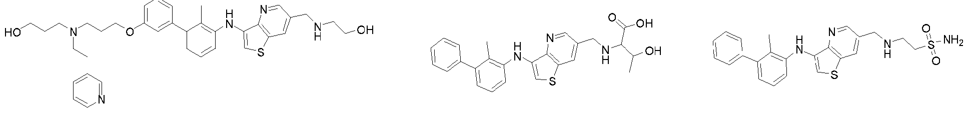

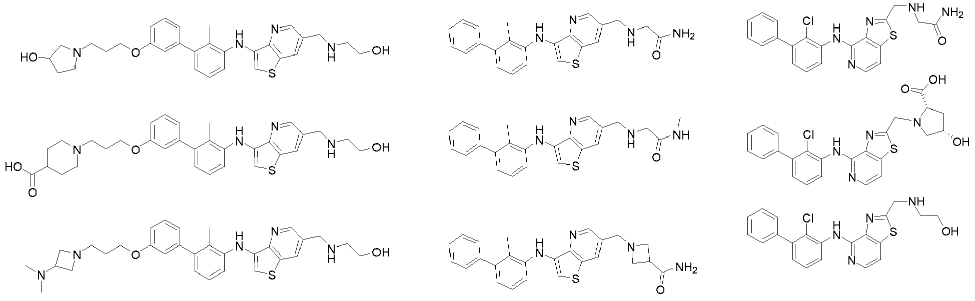

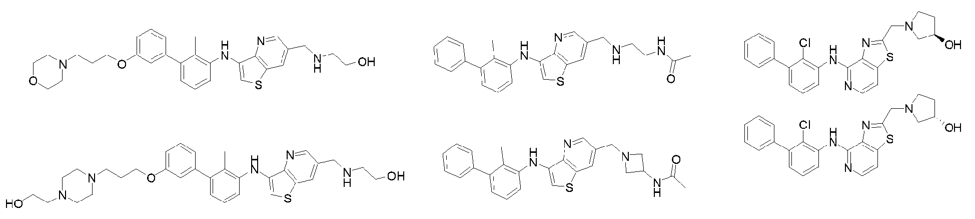

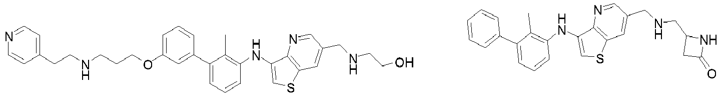

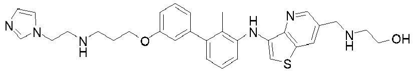

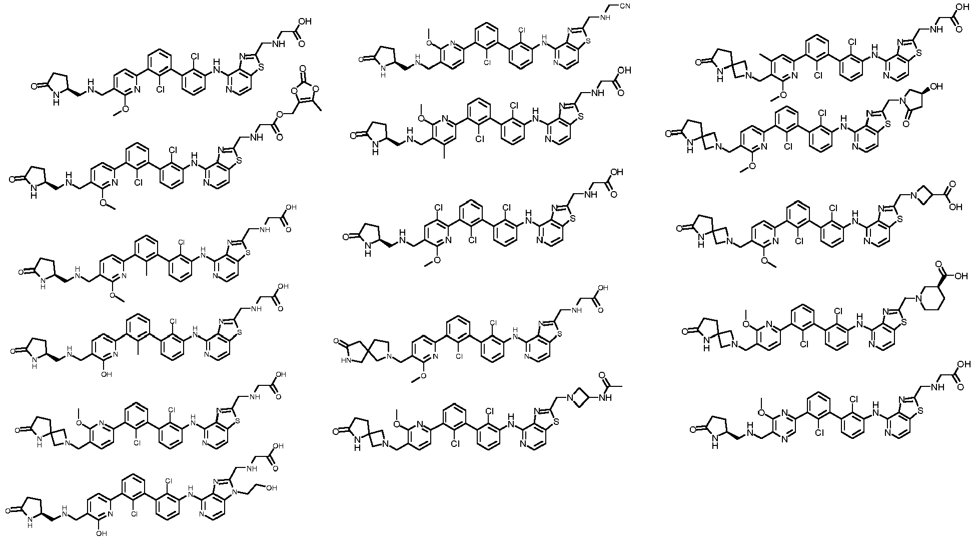

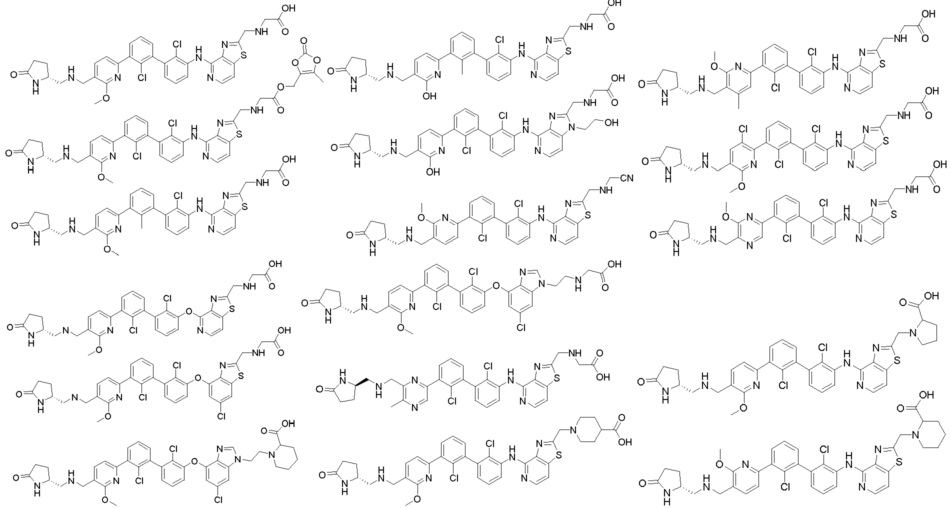

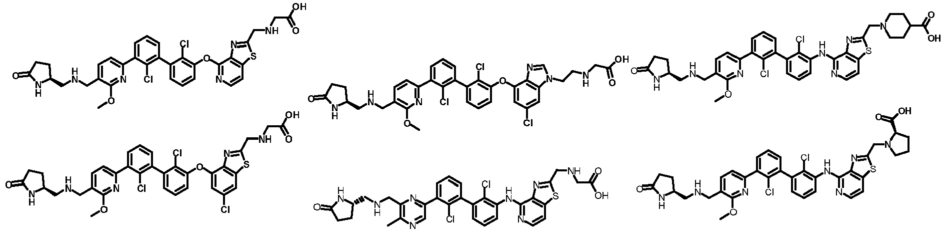

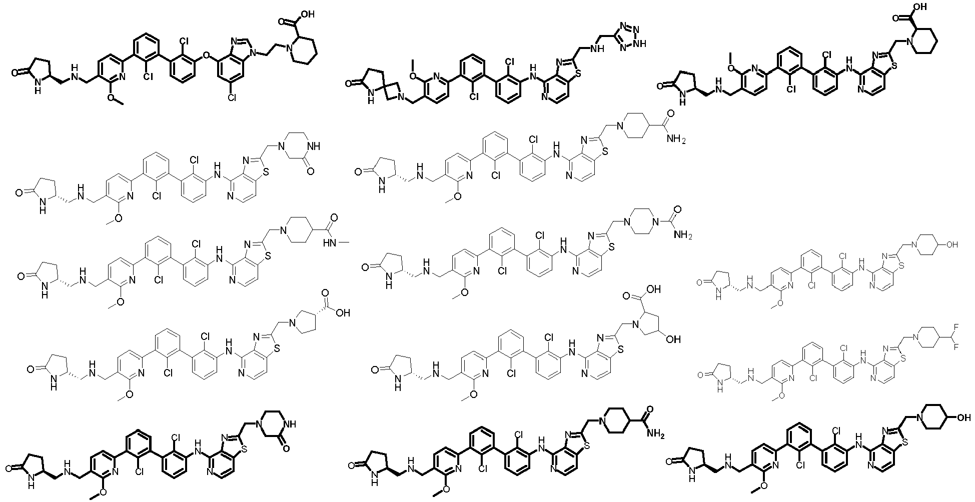

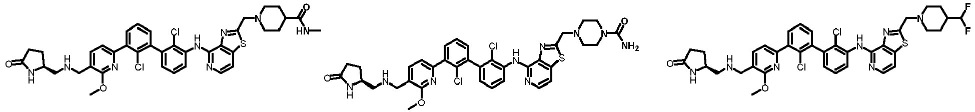

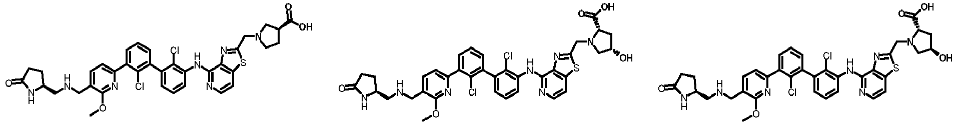

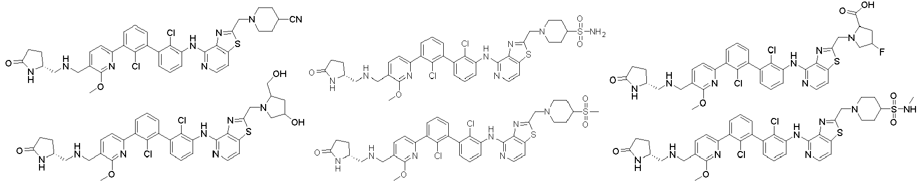

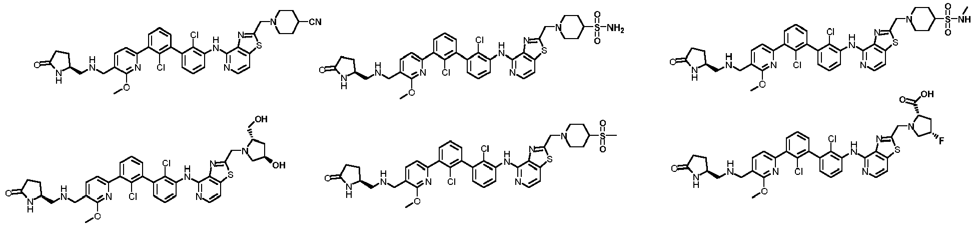

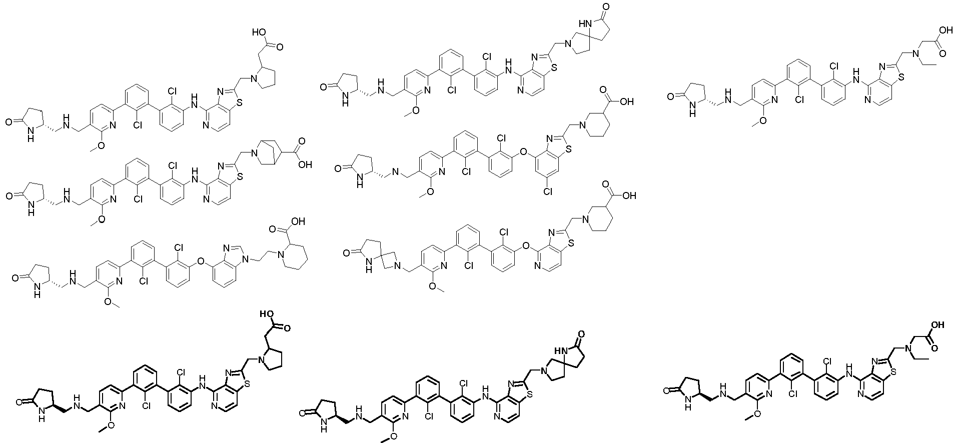

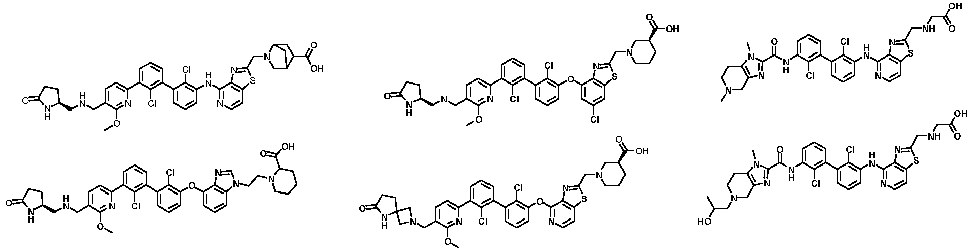

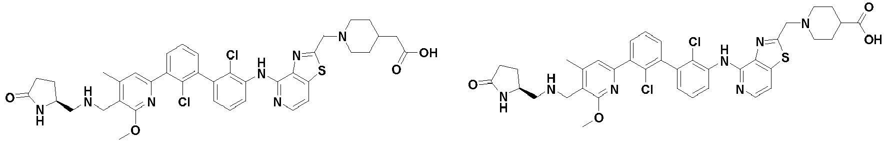

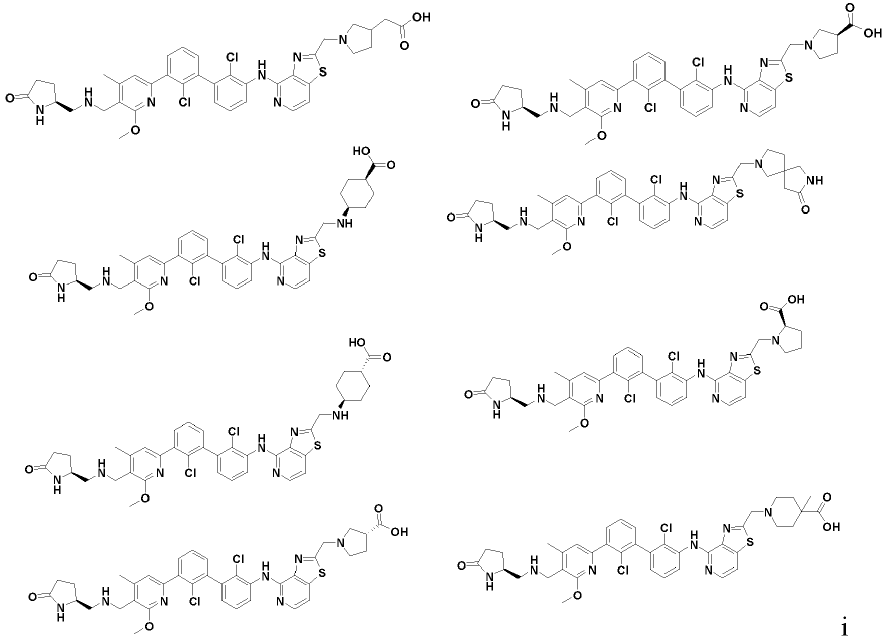

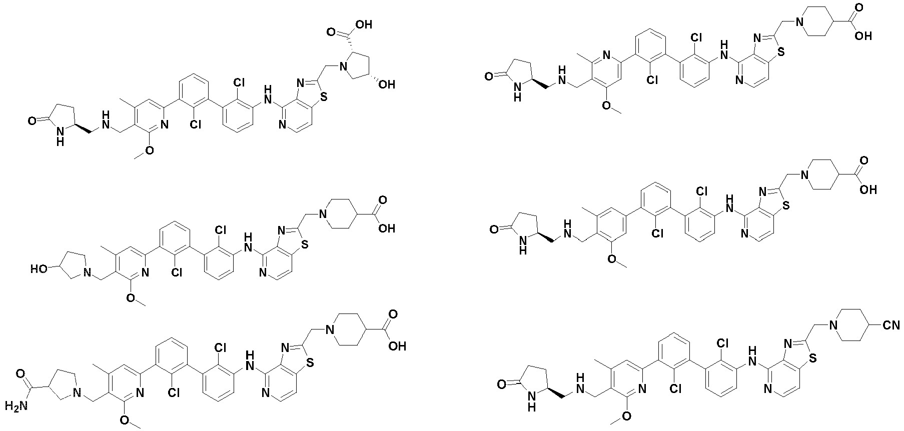

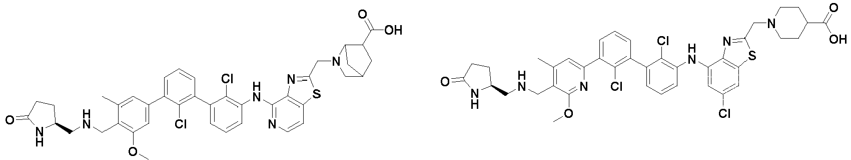

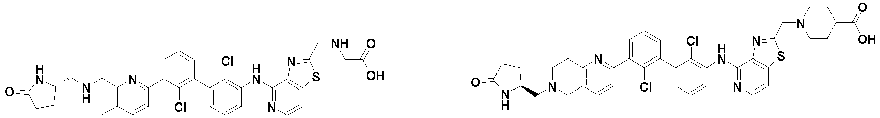

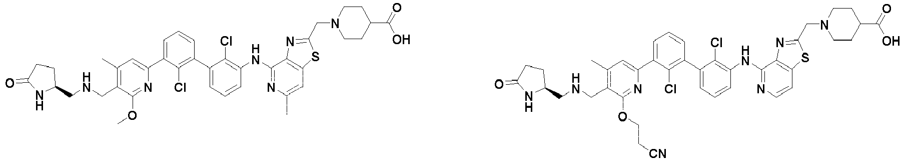

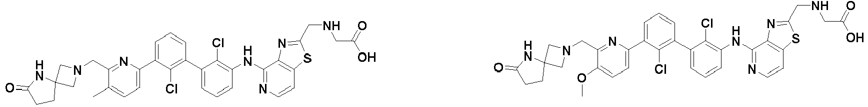

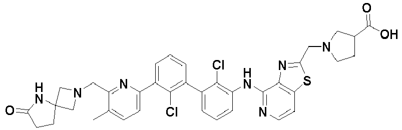

7. A pharmaceutical composition, comprising (1) the compound as decribed in claim 1 or stereoisomers or tautomers thereof, or pharmaceutically acceptable salts, hydrates or solvates thereof; (2) a pharmaceutically acceptable carrier.

8. The use of compound of claim 1 or stereoisomers or tautomers thereof, or pharmaceutically acceptable salts, hydrates or solvates thereof, or the pharmaceutical composition of claim 7 , wherein it preparation of pharmaceutical compositions for preventing and/or treating diseases related to the activity or expression of PD-1/PD-L1.

9. The use of claim 8, wherein the disease is selected from the group consisting of tumors, pathogen infections, and diseases related to autoimmune responses.

10. A PD-1/PD-L1 inhibitor, wherein the inhibitor comprises the compound of claim 1, or stereoisomers or tautomers thereof, or pharmaceutically acceptable salts, hydrates or solvates thereof.
